(19) Europäisches Patentamt
European Patent Office
Office européen des brevets

(11) Numéro de publication : **0 377 528 B1**

(12) # FASCICULE DE BREVET EUROPEEN

(45) Date de publication du fascicule du brevet : **08.12.93 Bulletin 93/49**

(51) Int. Cl.$^5$ : **C07D 211/32**, C07D 405/04, C07D 413/04, A61K 31/445

(21) Numéro de dépôt : **90400006.4**

(22) Date de dépôt : **03.01.90**

(54) **Pipéridines, procédés de préparation et médicaments les contenant.**

(30) Priorité : **05.01.89 FR 8900071**

(43) Date de publication de la demande : **11.07.90 Bulletin 90/28**

(45) Mention de la délivrance du brevet : **08.12.93 Bulletin 93/49**

(84) Etats contractants désignés : **AT BE CH DE DK ES FR GB GR IT LI LU NL SE**

(56) Documents cités : **EP-A- 0 196 132**

(73) Titulaire : **LIPHA, LYONNAISE INDUSTRIELLE PHARMACEUTIQUE**
**34, rue Saint Romain Boîte Postale 8481**
**F-69359 Lyon Cedex 08 (FR)**

(72) Inventeur : **Ferrand, Gérard**
**62, rue des Aqueducs**
**F-69005 Lyon (FR)**
Inventeur : **Dumas, Hervé**
**105, avenue du Vellein**
**F-38090 Villefontaine (FR)**
Inventeur : **Depin, Jean-Claude**
**117, cours Gambetta**
**F-69003 Lyon (FR)**
Inventeur : **Chavernac, Gilles**
**2 D, Chemin de la Bastéro**
**F-69350 La Mulatière (FR)**

(74) Mandataire : **Polus, Camille et al**
**c/o Cabinet Lavoix 2, Place d'Estienne d'Orves**
**F-75441 Paris Cedex 09 (FR)**

EP 0 377 528 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des pipéridines, des procédés permettant de les préparer et leur application dans le domaine thérapeutique.

Le rôle possible des récepteurs sérotoninergiques en association avec les récepteurs adrénergiques dans le traitement de certains troubles cardiovasculaires et en particulier de l'hypertension, est illustré par la 3-[2-[4-(4-fluorobenzoyl)-1-pipéridinyl]éthyl] quinazolin-2,4(1H,3H)-dione connue sous la désignation kétansérine. On connaît aussi l'action sur les récepteurs sérotoninergiques de la 3-[2-[4-(6-fluoro-1,2-benzisoxazol-3-yl)-1-pipéridinyl]éthyl]-2-méthyl-6,7,8,9-tétrahydro-4H-pyrido[1,2-a]pyrimidin-4-one décrite par L.E.J. Kennis et J. Vandenberk dans le brevet européen 196.132 et connue sous la désignation rispéridone. D'autre part, J.P. Cornu et al. ont décrit, dans le brevet WO 83/04.022. les actions antihypertensives, vasodilatatrices et sédatives de pipéridines substituées sur l'atome d'azote par un groupe phényloxoalcoyle, comme par exemple la N-cyano-N'-[[1-[4-(4-fluorophényl)-4-oxobutyl]-4-pipéridinyl]méthyl]-N"-méthylguanidine.

Les composés objets de l'invention sont représentés par la formule générale I

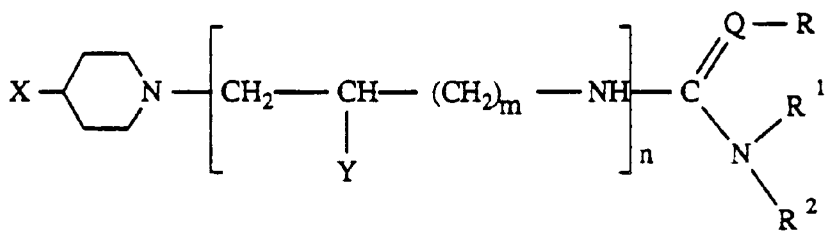

I

dans laquelle X est le groupe 4-fluorobenzoyle, 2-(4-fluorophényl)-1,3-dioxolan-2-yle ou 6-fluoro-1,2-benzisoxazol-3-yle, Y est un atome d'hydrogène ou le groupe hydroxy, m est un nombre entier compris entre 0 et 4 inclus, n est 0 ou 1, Q est un atome d'azote ou le groupe méthine ; lorsque Q est un atome d'azote, R est le groupe cyano ou le groupe carbamoyle ; lorsque Q est le groupe méthine, R est le groupe nitro ; $R^1$ et $R^2$ peuvent être identiques ou différents et sont l'hydrogène, un radical alcoyle inférieur, le radical phényle, le groupe 2,2,2-trifluoroéthyle ou 2-[4-(4-fluorobenzoyl)-1-pipéridinyl] éthyle ou le motif structural $NR^1R^2$ est le radical pipéridino ou le groupe 4-(4-fluorobenzoyl)-1-pipéridinyle.

Le terme inférieur appliqué à un radical alcoyle signifie que le radical peut être linéaire ou ramifié et qu'il peut comprendre de 1 à 6 atomes de carbone.

Les formes tautomères éventuelles des composés de l'invention font partie intégrante de l'invention.

Les composés dans la formule desquels X est le groupe 4-fluorobenzoyle ou 6-fluoro-1,2-benzisoxazol-3-yle, Y est un atome d'hydrogène. m est égal à 0,1 ou 2 et n est égal à 1, constituent une classe particulièrement intéressante.

Les sels pharmaceutiquement acceptables font également partie intégrante de l'invention. Ce peuvent être des sels préparés soit à partir d'acides minéraux comme l'acide chlorhydrique, l'acide bromhydrique, l'acide sulfurique, l'acide phosphorique, soit à partir d'acides organiques comme l'acide tartrique, l'acide citrique, l'acide acétique, l'acide maléique, l'acide fumarique, l'acide oxalique, l'acide méthanesulfonique.

Les composés de l'invention peuvent se préparer selon l'une au moins des méthodes suivantes :

a) Une amine de formule II

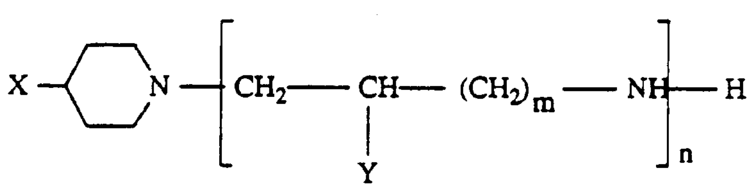

II

est condensée sur un dérivé de formule III

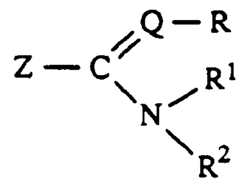

III

Dans les formules II et III, X, Y, Q, R, $R^1$, $R^2$, m et n ont les significations données précédemment. Dans la formule III, Z représente un groupe facilement déplaçable par une amine et est de préférence un radical méthylthio ou un radical phénoxy. La réaction s'effectue dans un solvant inerte. Les solvants préférés sont les alcanols de bas poids moléculaire en particulier l'éthanol et le 2-méthoxyéthanol. La température peut varier entre la température ambiante et la température d'ébullition du solvant utilisé. Le temps de réaction est généralement compris entre 3 et 40 heures.

b) Un dérivé de formule IV

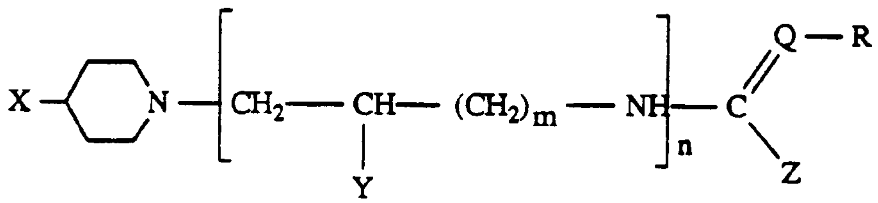

IV

est condensé sur une amine de formule V

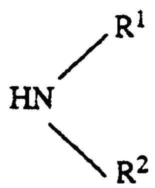

V

Dans les formules IV et V, X, Y, Q, R, $R^1$, $R^2$, m et n ont les significations données précédemment. Dans la formule IV, Z représente un groupe facilement déplaçable par une amine et est de préférence un radical méthylthio ou un radical phénoxy. Cette condensation se fait soit sans solvant en présence d'un excès d'amine de formule V, soit dans un solvant inerte. Les solvants préférés sont les alcanols de bas poids moléculaire, en particulier l'éthanol. La température peut varier entre la température ambiante et la température d'ébullition de l'amine de formule V ou du solvant utilisé. Le temps de réaction est compris entre 1 et 22 heures.

c) Dans le cas particulier où X est le groupe 4-fluorobenzoyle, Q un atome d'azote et R le groupe carbamoyle, les composés de l'invention I correspondants peuvent être obtenus par hydrolyse des composés I pour lesquels X est le groupe 2-(4-fluorophényl)-1,3-dioxolan-2-yle, Q est un atome d'azote et R est le groupe cyano.

d) Dans le cas particulier où X est le groupe 6-fluoro-1,2-benzisoxazol-3-yle, Q un atome d'azote et R le groupe carbamoyle, les composés de l'invention I correspondants sont obtenus par hydrolyse des composés I pour lesquels X est le groupe 6-fluoro-1,2-benzisoxazol-3-yle, Q est un atome d'azote et R est le groupe cyano.

Les amines intermédiaires de formule générale II sont pour certaines d'entre elles des composés connus. Celles qui sont nouvelles ont été préparées selon des techniques usuelles :

α) Lorsque X est le groupe 4-fluorobenzoyle ou 2-(4-fluorophényl)-1,3-dioxolan-2-yle, Y un atome d'hydrogène et que m est égal à 1 et n est égal à 1, les amines correspondantes VIII et IX sont obtenues selon le schéma réactionnel ci-dessous :

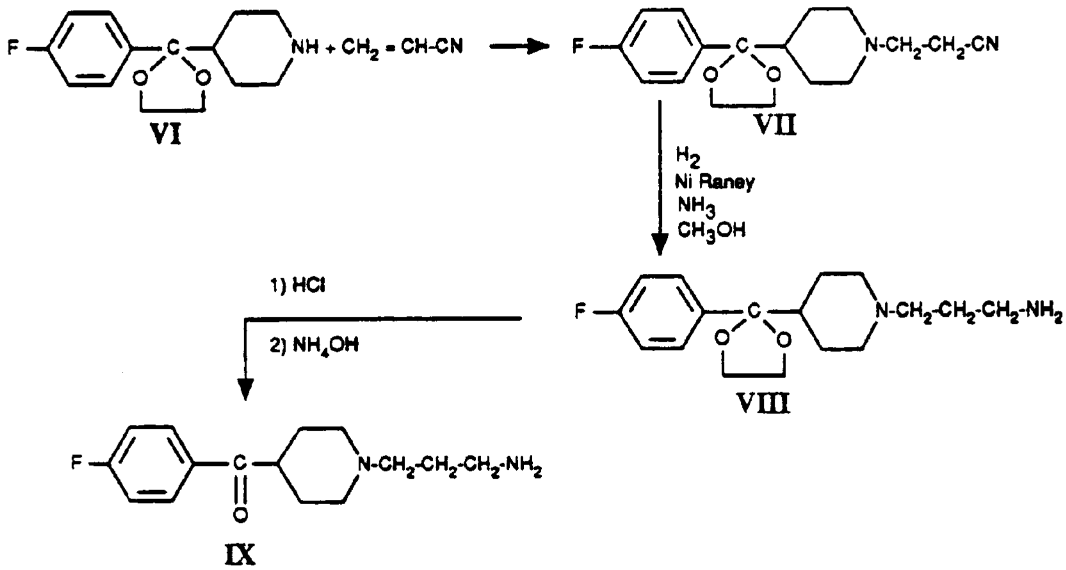


La pipéridine VI réagit avec l'acrylonitrile pour donner un 3-pipéridinopropionitrile de formule VII qui est ensuite réduit catalytiquement en une 1-(3-aminopropyl)pipéridine de formule VIII. La fonction acétal du composé VIII peut être hydrolysée en milieu acide pour donner la 1-(3-aminopropyl)-4-(4-fluorobenzoyl)-pipéridine de formule IX.

β) Lorsque X est le groupe 4-fluorobenzoyle ou 2-(4-fluorophényl)-1,3-dioxolan-2-yle, Y un atome d'hydrogène et que m est égal à 4 et n est égal à 1, les amines correspondantes XI et XII sont obtenues selon le schéma réactionnel ci-dessous :

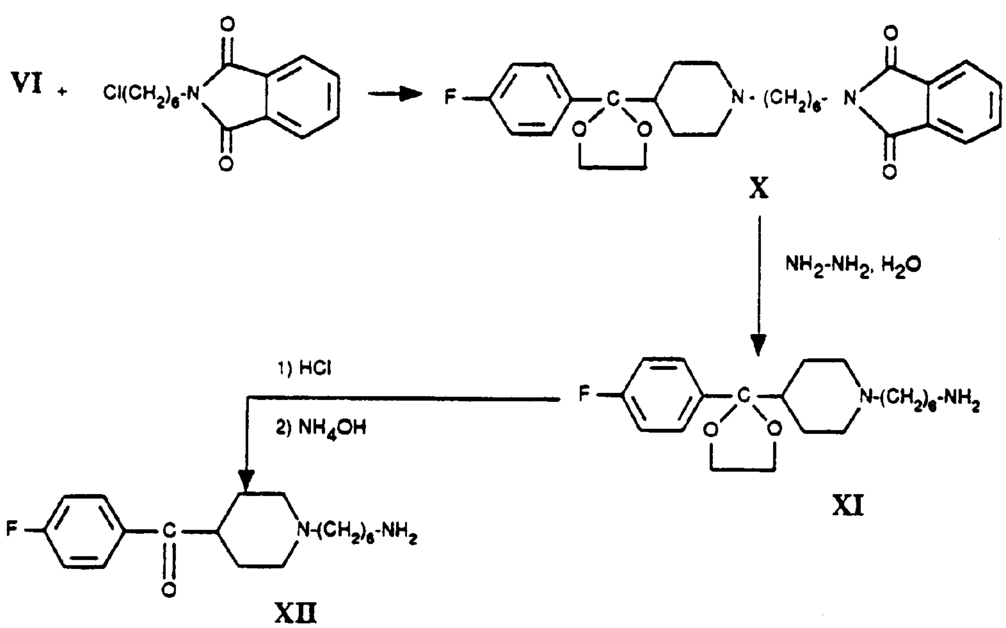


La pipéridine VI est condensée avec le N-(6-chlorohexyl)phtalimide en présence d'une base telle qu'un carbonate alcalin, préférentiellement le carbonate de potassium, pour donner un composé de formule X. Par traitement avec de l'hydrate d'hydrazine, le N-alcoylphtalimide X donne une 1-(6-aminohexyl)pipéridine de formule XI. La fonction acétal du composé XI peut être hydrolysée en milieu acide pour fournir la 1-(6-aminohexyl)-4-(4-fluorobenzoyl)pipéridine de formule XII.

γ) Lorsque X est le groupe 4-fluorobenzoyle ou 2-(4-fluorophényl)-1,3-dioxolan-2-yle, Y le groupe hydroxy et que m est égal à 1 et n est égal à 1, les amines correspondantes XIV et XV sont obtenues selon le schéma réactionnel ci-dessous :

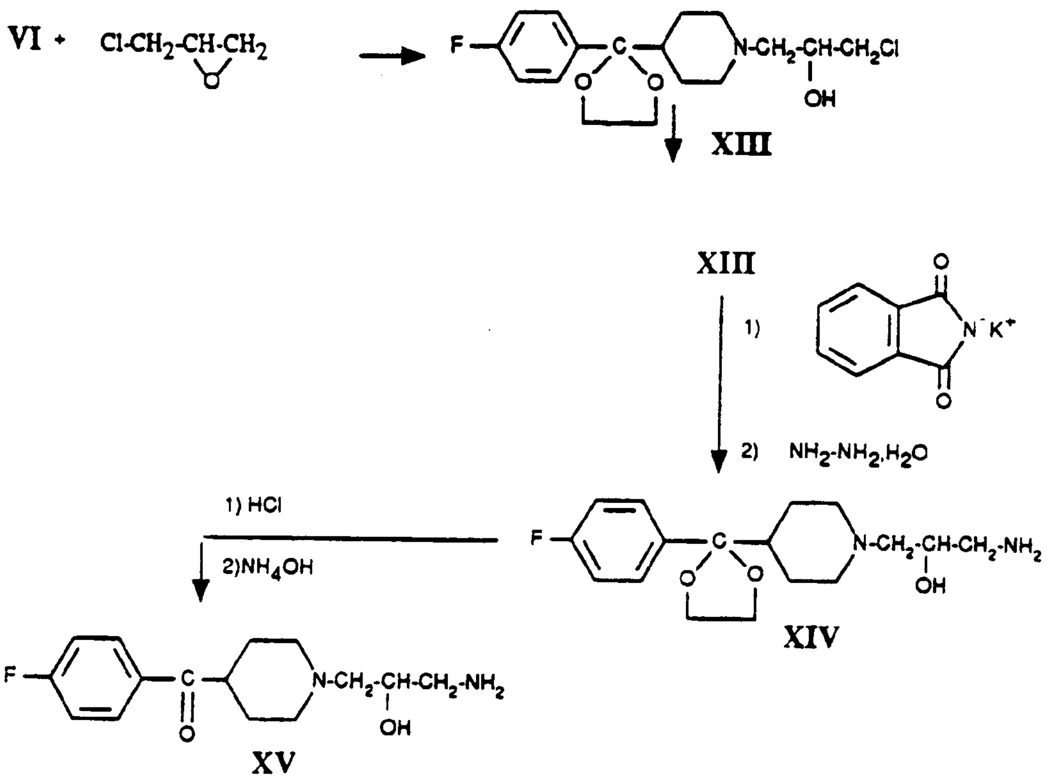


La pipéridine VI est traitée par l'épichlorhydrine pour donner le composé chloré de formule XIII. Par synthèse de Gabriel, la 1-(3-chloro-2-hydroxypropyl)pipéridine XIII donne une 1-(3-amino-2-hydroxypropyl)-pipéridine de formule XIV. La fonction acétal du composé XIV peut être hydrolysée en milieu acide pour fournir le 1-amino-3-[4-(4-fluorobenzoyl)-1-pipéridinyl]propan-2-ol de formule XV.

δ) Lorsque X est le groupe 6-fluoro-1,2-benzisoxazol-3-yle, Y un atome d'hydrogène et que n est égal à 1, les amines corespondantes XIX sont obtenues selon le schéma réactionnel ci-dessous :

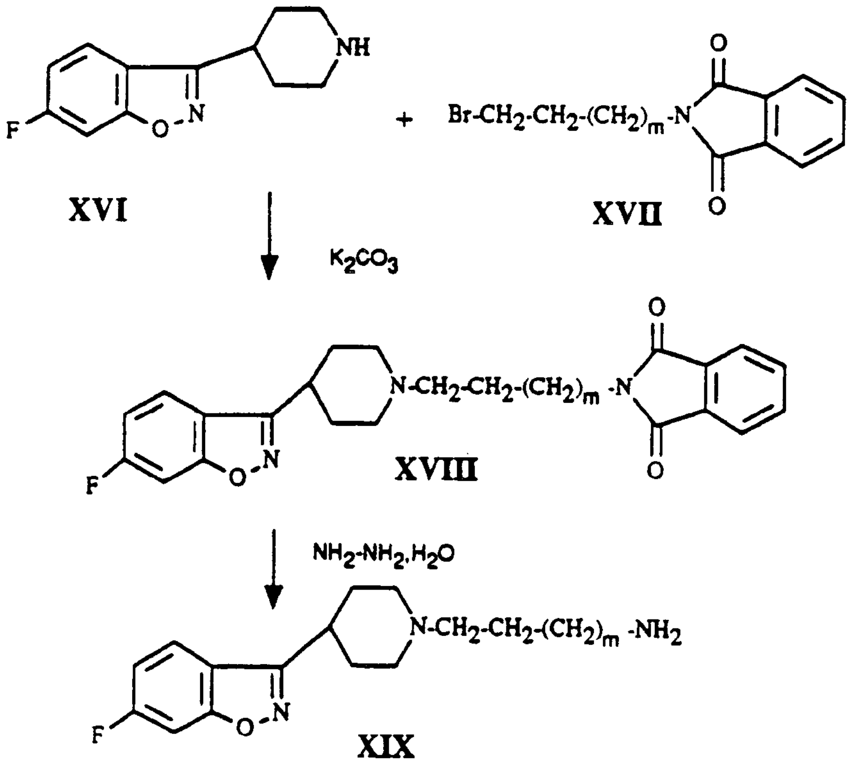


XVI XVII XVIII XIX

La pipéridine XVI réagit avec un N-(ω-bromoalcoyl)phtalimide XVII en présence d'une base telle que le carbonate de potassium, pour donner un phtalimide de formule XVIII, qui est ensuite transformé par hydrazinolyse en un 3-[1-(ω-aminoalcoyl)-4-pipéridinyl]-6-fluoro-1,2-benzisoxazole de formule XIX.

Dans le cas particulier où X est le groupe 6-fluoro-1,2-benzisoxazol-3-yle, Y un atome d'hydrogène et où m est égal à 1 et n est égal à 1, l'amine correspondante XXII peut également être obtenue selon le schéma réactionnel ci-dessous :

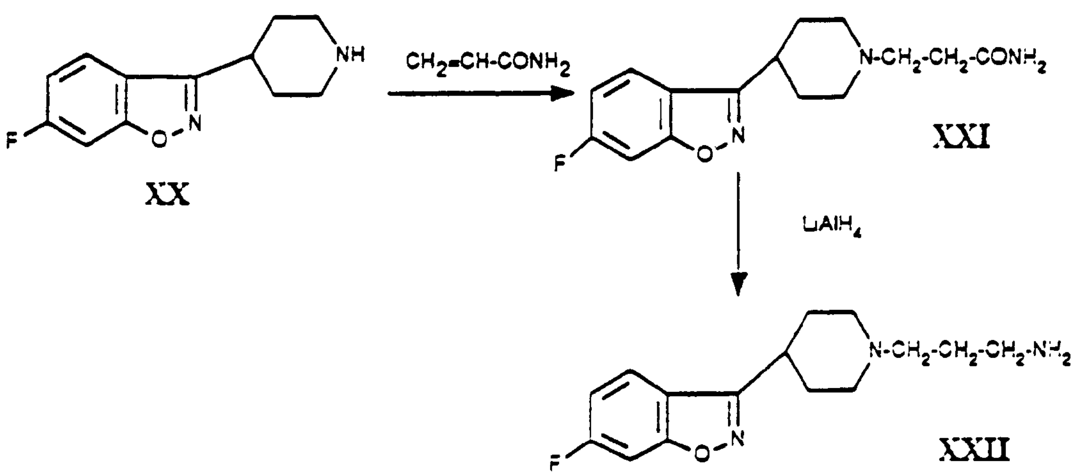


XX XXI XXII

La pipéridine XX réagit avec l'acrylamide dans un solvant inerte tel que l'éthanol, pour donner le propionamide de formule XXI qui est ensuite réduit par un hydrure tel que l'hydrure mixte de lithium et d'aluminium, en 3-[1-(3-aminopropyl)-4-pipéridinyl]-6-fluoro-1,2-benzisoxazole de formule XXII.

Dans le cas particulier où X est le groupe 6-fluoro-1,2-benzisoxazol-3-yle, Y un atome d'hydrogène et où m est égal à zéro et n est égal à 1, l'amine correspondante XXIV peut également être obtenue par réduction de l'acétonitrile de formule XXIII selon la réaction suivante :

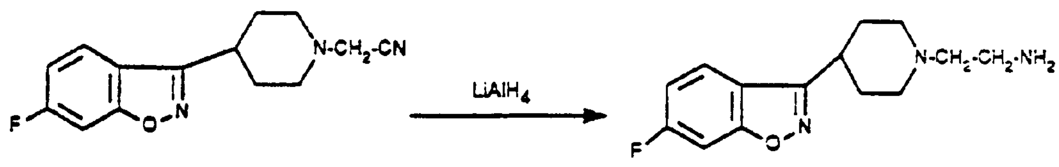


XXIII XXIV

Les dérivés intermédiaires de formule III sont pour la plupart d'entre eux des composés connus. Ceux qui sont nouveaux ont été préparés selon des techniques usuelles, à savoir : lorsque Q est un atome d'azote, R le groupe cyano et Z le radical méthylthio, les dérivés de formule III correspondants sont obtenus par condensation des amines de formule V sur le N-cyanodithioiminocarbonate de diméthyle, appelé couramment et ci-après N-cyanodithioiminocarbonate de méthyle. Dans la formule V, $R^1$ et $R^2$ ont les significations données précédemment.

Les dérivés intermédiaires de formule IV sont des produits nouveaux. Ils sont préparés par condensation d'une amine de formule II sur un dérivé de formule XXV.

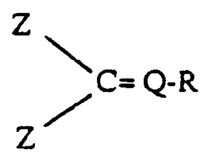

XXV

Dans les formules II et XXV, les substituants X, Y, Q, R, et m et n ont les significations données précédemment. Dans la formule XXV, Z représente un groupe facilement déplaçable par une amine et est de préférence un radical méthylthio ou un radical phénoxy.

Les composés représentés par la formule générale I possèdent de remarquables propriétés antihypertensives et antisérotonine susceptibles de rendre particulièrement intéressant leur usage dans la maladie hypertensive ainsi que dans les désordres organiques engendrés ou aggravés par un excès de sérotonine.

L'activité antihypertensive a été recherchée chez le rat génétiquement hypertendu dont la pression artérielle était régulièrement suivie : des rats mâles hypertendus et ayant une pression artérielle particulièrement stable sont groupés en lots de 10 et traités oralement avec les composés étudiés. Leur pression artérielle est mesurée 3 heures plus tard. On détermine ainsi pour chaque composé la dose minima abaissant significativement la pression artérielle moyenne du lot traité (D.M.A.).

L'antagonisme vis à vis de la sérotonine a été mis en évidence in vitro sur l'aorte isolée de rat : des rats mâles d'environ 300 g sont sacrifiés et saignés. On prélève rapidement l'aorte thoracique. Un fragment d'environ 1 cm est coupé en spirale puis placé dans une cuve renfermant 20 ml de milieu nutritif (Krebs-Henseleit) maintenu à 37°C et oxygéné ($O_2$ 95 %, $CO_2$ 5 %). La contraction est enregistrée par une jauge de contrainte isotonique. Après un repos de 45 min, des contractions supramaximales sont déclenchées par addition de sérotonine (20 $\mu$M/l) au milieu de survie. Un plateau est en général atteint après 10 min de contact, ce dernier ne dépassant pas 25 min. Un repos de 30 min et de nombreux rinçages séparent chaque contraction. Les produits sont ajoutés 10 min après la sérotonine et laissés 15 min, délai au bout duquel l'inhibition est mesurée. On détermine pour chaque composé étudié et sur un minimum de 3 aortes, la concentration inhibitrice 50 (CI 50).

Les résultats obtenus par ces deux méthodes pour quelques produits de l'invention et pour la kétansérine (3-[2-[4-(4-fluorobenzoyl)-1-pipéridinyl]éthyl]quinazolin-2,4(1H,3H)-dione) prise comme étalon sont consignés dans le tableau I.

Tableau I

| PRODUITS | Activité antihypertensive rat hypertendu D.M.A. (mg/kg/PO) | Activité antisérotonine aorte isolée de rat CI50 (µM/l) |
|---|---|---|
| Kétansérine | 25 | 0,15 |
| Exemple 1 | 10 | 0,53 |
| Exemple 10 | 10 | 0,46 |
| Exemple 12 | 5 | 0,17 |
| Exemple 13 | 5 | 1,95 |
| Exemple 14 | 10 | 0,67 |
| Exemple 18 | 2 | 0,09 |
| Exemple 19 | 0,5 | 0,05 |
| Exemple 20 | 0,5 | 0,04 |
| Exemple 21 | 2 | 0,10 |
| Exemple 22 | 5 | 0,02 |
| Exemple 24 | 10 | 2,90 |
| Exemple 26 | 5 | 0,38 |

Les composés de l'invention manifestent une faible toxicité. A titre d'illustration, la dose létale 50 déterminée sur le rat par voie orale, pour le composé décrit dans l'exemple 1, est de 1 700 mg/kg, soit 170 fois la dose active sur cette espèce.

La présente demande a également pour objet l'application des composés I à titre de médicaments et notamment de médicaments antihypertenseurs. Ces médicaments peuvent être administrés par voie orale sous forme de comprimés, comprimés dragéifiés ou de gélules ou par voie intraveineuse sous forme de soluté injectable. Le principe actif est associé à divers excipients pharmaceutiquement compatibles. Les posologies journalières peuvent varier de 2 à 100 mg de principe actif pris par voie orale et de 0,2 à 10 mg de principe actif pris par voie intraveineuse.

On donne ci-dessous, à titre d'exemples non limitatifs quelques formulations pharmaceutiques :

- Composition d'un comprimé :
  principe actif 10 mg
  excipient : lactose, amidon de blé, polyvidone, talc, stéarate de magnésium.
- Composition d'une gélule :
  principe actif 10 mg
  excipient : lactose, amidon de blé, talc, stéarate de magnésium.

Les exemples suivants illustrent l'invention à titre non limitatif. Dans les données de résonance magnétique nucléaire (R.M.N.), les abréviations suivantes ont été utilisées : s pour singulet, d pour doublet, t pour triplet, q pour quadruplet et m pour massif complexe ; les déplacements chimiques δ sont exprimés en ppm.

**Exemple 1 :**

**N-Cyano-N'-[2-[4-(4-fluorobenzoyl)-1-pipéridinyl]éthy]-N"-méthylguanidine**

a) N-Cyano-N'-[2-[4-(4-fluorobenzoyl)-1-pipéridinyl]éthyl]-S-méthylisothiourée

A une solution de 3,3 g (0,023 mole) de N-cyanodithioiminocarbonate de méthyle dans 52 ml d'éthanol, on ajoute goutte à goutte une solution de 5,7 g (0,023 mole) de 1-(2-aminoéthyl)-4-(4-fluorobenzoyl)-pipéridine [préparée selon A. Storni, brevet européen 124.476 ; C.A. (1985) 102, 131917b] dans 45 ml d'éthanol. L'agitation est poursuivie pendant 8 heures à température ambiante, après la fin de l'addition ; il se produit un dégagement de méthylmercaptan. Le milieu réactionnel est ensuite refroidi vers 0°C. Le précipité formé est isolé par filtration ; il est lavé à l'éther et recristallisé dans l'éthanol. Rdt : 5,5 g (69 %), F = 137 - 139°C.

| Analyse centésimale : $C_{17}H_{21}FN_4OS$ (M = 348,44) | | | | | |
|---|---|---|---|---|---|
| | C % | H % | F % | N % | S % |
| calculé | 58,60 | 6,08 | 5,45 | 16,08 | 9,20 |
| trouvé | 58,78 | 5,94 | 5,30 | 16,13 | 9,45 |

IR :
$\bar{\nu}$(C = O) = 1670 $cm^{-1}$
$\bar{\nu}$(C ≡ N) = 2160 $cm^{-1}$
R.M.N. ($CDCl_3$) : δ = 2,5 (3H,s) ; 1,6 - 3,7 (13H,m) ; 6,7 (1H,pic échangeable avec $D_2O$ ; 7,0 - 7,4 (2H,m) ; 7,8 - 8,2 (2H,m).

b) N-Cyano-N'-[2-(4-(4-fluorobenzoyl)-1-pipéridiny]éthyl]-N''-méthylguanidine

A une suspension de 5,5 g (0,016 mole) de N-cyano-N'-[2-[4-(4-fluorobenzoyl)-1-pipéridinyl]éthyl]-S-méthylisothiourée dans 32 ml d'éthanol, on ajoute goutte à goutte 32 ml d'une solution à 33 % de méthylamine dans l'éthanol absolu. La solution obtenue est portée à reflux pendant 1 heure. Il se produit un dégagement de méthylmercaptan. Après refroidissement, le précipité formé est filtré, lavé à l'hexane et séché. Il est recristallisé dans l'éthanol. Rdt : 4,3 g (82 %), F = 160 - 162°C.

Analyse centésimale : $C_{17}H_{22}FN_5O$ (M = 331,39)

| | C % | H % | F % | N % |
|---|---|---|---|---|
| calculé | 61,61 | 6,69 | 5,73 | 21,14 |

| | C % | H % | F % | N % |
|---|---|---|---|---|
| trouvé | 61,79 | 6,49 | 5,46 | 21,26 |

IR :
$\bar{\nu}$(C = O) = 1670 $cm^{-1}$
$\bar{\nu}$(C ≡ N) = 2160 $cm^{-1}$
R.M.N. ($CDCl_3$ + DMSO $d_6$) : δ = 2,7 (3H,d se transforme en s avec $D_2O$) ; 1,4 - 3,7 (13H, m) ; 6,3 (1H, pic échangeable avec $D_2O$) ; 6,9 - 7,4 (2H, m) ; 7,7 (1H, pic échangeable avec $D_2O$) ; 7,8 - 8,2 (2H,m).

**Exemple 2 :**

**N-Cyano-N'-[2-[4-(4-fluorobenzoyl)-1-pipéridinyl]éthyl]-N''-méthylguanidine**

a) N-Cyano-N'-[2-[4-(4-fluorobenzoyl)-1-pipéridinyl]éthyl]phénoxyformamidine

Un mélange de 9,5 g (0,040 mole)de N-cyanocarbonimidate de diphényle et de 10,0 g (0,040 mole) de 1-(2-aminoéthyl)-4-(4-fluorobenzoyl)pipéridine dans 100 ml d'isopropanol est agité pendant 15 heures à température ambiante. Le précipité formé est isolé par filtration ; il est lavé à l'éther et recristallisé dans l'acétate d'éthyle. Rdt : 11,0 g (70 %), F = 134 - 136°C.

| Analyse centésimale : $C_{22}H_{23}FN_4O_2$ (M = 394,45) | | | | |
|---|---|---|---|---|
| | C % | H % | F % | N % |
| calculé | 66,99 | 5,88 | 4,82 | 14,20 |
| trouvé | 66,93 | 5,82 | 4,83 | 14,08 |

IR :
$\bar{\nu}$(C = O) = 1655 $cm^{-1}$
$\bar{\nu}$(C ≡ N) = 2175 $cm^{-1}$
R.M.N. ($CDCl_3$ + $D_2O$) : δ = 1,5 - 3,8 (13H,m) ; 6,9 - 7,6 (7H,m) ; 7,7 - 8,1 (2H,m).

b) N-Cyano-N'-[2-[4-(4-fluorobenzoyl)-1-pipéridinyl]éthyl]-N''-méthylguanidine

A une suspension de 2,1 g (0,0053 mole) de N-cyano-N'-[2-[4-(4-fluorobenzoyl)-1-pipéridinyl]éthyl]-phénoxyformamidine dans 25 ml d'éthanol, on ajoute goutte à goutte 5,4 ml d'une solution à 33 % de méthylamine dans l'éthanol absolu. La solution obtenue est agitée pendant 1 heure à température ambiante. Le milieu réactionnel est ensuite refroidi vers 0°C. Le précipité formé est isolé par filtration ; il est lavé à l'éther et recristallisé dans l'éthanol. Rdt : 1,3 g (74 %), F = 160 - 162°C. Le produit est identique à celui obtenu dans l'exemple 1.

**Exemple 3 :**

**N-Cyano-N'-[2-[4-[2-(4-fluorophényl)-1,3-dioxolan-2-yl]-1-pipéridinyl]éthyl)-N''-méthylguanidine**

Un mélange de 10,4 g (0,035 mole) de 1-(2-aminoéthyl-4-[2-(4-fluorophényl)-1,3-dioxolan-2-yl]pipéridine [préparée selon J. Vandenbenrk et al., brevet européen 184.258 ; C.A. (1986) 105, 133907j] et de 4,5 g (0,035 mole) de N-cyano-N'-méthyl-S-méthylisothiourée dans 120 ml de 2-méthoxyéthanol est porté à reflux pendant 3 heures. Il se produit un dégagement de méthylmercaptan. Après refroidissement, la solution obtenue est concentrée à sec sous pression réduite. Le résidu solide est lavé à l'éther et recristallisé dans l'éthanol. Rdt : 4,1 g (31 %), F = 194 - 196°C.

| Analyse centésimale : $C_{19}H_{26}FN_5O_2$ (M = 375,44) | | | | |
|---|---|---|---|---|
| | C % | H % | F % | N % |
| calculé | 60,78 | 6,98 | 5,06 | 18,65 |
| trouvé | 60,75 | 6,93 | 4,99 | 18,71 |

IR :
$\bar{\nu}$(C ≡ N) = 2150 $cm^{-1}$
R.M.N. (DMSO $d_6$ + $CF_3COOD$) : δ = 2,7 (3H,s); 1,2 - 3,6 (13H, m) ; 3,4 - 4,1 (4H,m) ; 6,8 - 7,5 (4H,m).

**Exemple 4 :**

**N-Cyano-N'-[2-[4-[2-(4-fluorophényl)-1,3-dioxolan-2-yl]-1-pipéridinyl]éthyl]guanidine**

Un mélange de 4.0 g (0,0136 mole) de 1-(2-aminoéthyl)-4-[2-(4-fluorophényl)-1,3-dioxolan-2-yl]-pipéridine et de 1,6 g (0,0136 mole) de N-cyano-S-méthylisothiourée [préparée selon C.G. Mc Carty et al., J. Org. Chem. (1970) 35, 2067] dans 100 ml de 2-méthoxyéthanol est porté à reflux pendant 16 heures. Il se produit un dégagement de méthylmercaptan. Après refroidissement, le milieu réactionnel est concentré à sec sous pression réduite. Le résidu est chromatographié sur colonne de silice. Eluant : chloroforme 4 -méthanol 1. L'évaporation de l'éluat fournit la N-cyano-N'-[2-[4-[2-(4-fluorophényl)-1,3-dioxolan-2-yl]-1-pipéridinyl]éthyl]guanidine. Rdt : 1,0 g (20 %), F = 161 - 163°C (éthanol-éther isopropylique).

| Analyse centésimale : $C_{18}H_{24}FN_5O_2$ (M = 361,42) | | | | |
|---|---|---|---|---|
| | C % | H % | F % | N % |
| calculé<br>trouvé | 59,82<br>59,55 | 6,69<br>6,65 | 5,26<br>4,92 | 19,38<br>19,46 |

IR :
$\bar{\nu}$(C ≡ N) = 2150 $cm^{-1}$
R.M.N. ($CDCl_3$ + $CF_3COOD$) : δ = 1,5 - 3,7(13H,m) ; 3,5 - 4,1 (4H, m) ; 6,7 - 7,5 (4H,m).

**Exemple 5 :**

**N-Cyano-N'-[2-[4-[2-(4-fluorophényl)-1,3-dioxolan-2-yl]-1-pipéridinyl]éthyl]guanidine**

a) N-Cyano-N'-[2-[4-[2-(4-fluorophényl)-1,3-dioxolan-2-yl]-1-pipéridinyl]éthyl]-S-méthylisothiourée

Une solution de 12,0 g (0,041 mole) de 1-(2-aminoéthyl)-4-[2-(4-fluorophényl)-1,3-dioxolan-2-yl]- pipéridine dans 80 ml d'éthanol est ajoutée goutte à goutte à une solution de 6,0 g (0,041 mole) de N-cyanodithioiminocarbonate de méthyle dans 100 ml d'éthanol. L'agitation est poursuivie pendant 9 heures à température ambiante, après la fin de l'addition ; il se produit un dégagement de méthylmercaptan. La solution obtenue est ensuite concentrée à sec sous pression réduite. Le résidu est lavé à l'éther isopropylique et recristallisé dans un mélange d'éthanol et d'éther isopropylique. Rdt : 12,1 g (75 %), F = 133 - 134,5° C.

| Analyse centésimale : $C_{19}H_{25}FN_4O_2S$ (M = 392,49) | | | | | |
|---|---|---|---|---|---|
| | C % | H % | F % | N % | S % |
| calculé<br>trouvé | 58,14<br>57,76 | 6,42<br>6,66 | 4,84<br>4,79 | 14,27<br>13,98 | 8,17<br>8,03 |

IR :
$\bar{\nu}$(C ≡ N) = 2160 $cm^{-1}$
R.M.N. ($CDCl_3$ + $D_2O$) : δ = 2,4 (3H,s) ; 1,3 - 3,6 (13H, m); 3,5 - 4,1 (4H,m) ; 6,8 - 7,5 (4H,m).

b) N-Cyano-N'-[2-[4-[2-(4-fluorophényl)-1,3-dioxolan-2-yl]-1-pipéridinyl]éthyl]guanidine

Une solution de 4,0 g (0,010 mole) de N-cyano-N'-[2-[4-[2-(4-fluorophényl)-1,3-dioxolan-2-yl]-1-pipéridinyl]éthyl]-S-méthylisothiourée dans 100 ml d'éthanol est portée à reflux ; on fait barboter un courant d'ammoniac dans cette solution pendant 20 heures. Après refroidissement, le milieu réactionnel est concentré à sec sous pression réduite. Le résidu est concrétisé dans un mélange d'éther isopropylique et d'isopropanol. Il est purifié par recristallisation dans un mélange d'éthanol et d'éther isopropylique. Rdt : 0,6 g (17 %), F = 161 - 163° C. Le produit est identique à celui obtenu dans l'exemple 4.

**Exemple 6 :**

**N-Cyano-N'-[2-[4-(4-fluorobenzoyl)-1-pipéridinyl]éthyl]-N"-phénylguanidine**

Un mélange de 11,0 g (0,032 mole) de N-cyano-N'-[2-[4-(4-fluorobenzoyl)-1-pipéridinyl]éthyl]-S-méthylisothiourée et de 80 ml d'aniline est porté à 110 - 120°C pendant 7 heures puis à 135°C pendant 1 heure 45. Il se produit un dégagement de méthylmercaptan. Le milieu réactionnel est ensuite concentré à sec sous pression réduite. Le résidu est purifié par recristallisation dans l'éthanol. Rdt : 3,8 g (31 %), F = 199 -201°C.

<u>Analyse centésimale</u> : $C_{22}H_{24}FN_5O$ (M = 393,45)

| | C % | H % | F % | N % |
|---|---|---|---|---|
| calculé | 67,15 | 6,15 | 4,83 | 17,80 |

| | C % | H % | F % | N % |
|---|---|---|---|---|
| trouvé | 67,00 | 6,21 | 4,88 | 17,84 |

<u>IR</u> :
$\bar{\nu}$(C = O) = 1670 $cm^{-1}$
$\bar{\nu}$(C ≡ O) = 2160 $cm^{-1}$
<u>R.M.N.</u> (DMSO $d_6$ + $CF_3COOD$) : δ = 1,4 - 2,3 (4H,m) ; 2,6 - 4,0 (9H,m) ; 6,9 - 7,5 (7H,m) ; 7,8 - 8,2 (2H,m).

**<u>Exemple 7 :</u>**

**<u>N-Cyano-N'-[2-[4-(4-fluorobenzoyl)-1-pipéridinyl]éthyl]-N"-isopropylguanidine</u>**

Un mélange de 4,0 g (0,0115 mole) de N'-cyano-N-[2-[4-(4-fluorobenzoyl)-1-pipéridinyl]éthyl]-S-méthylisothiourée et de 40 ml d'isopropylamine est porté à léger reflux pendant 3 heures 30. On ajoute 40 ml d'éthanol et porte le mélange à reflux pendant 3 heures. Le dégagement de méthylmercaptan est très progressif. On rajoute 20 ml d'isopropylamine et continue le reflux pendant 8 heures. Après refroidissement, le précipité formé est isolé par filtration ; il est lavé à l'éther et recristallisé dans un mélange d'éthanol et d'éther isopropylique en présence de Norit. Rdt : 1,6 g (39 %), F = 158 -160°C.

| Analyse centésimale : $C_{19}H_{26}FN_5O$ (M = 359,44) | | | | |
|---|---|---|---|---|
| | C % | H % | F % | N % |
| calculé | 63,49 | 7,29 | 5,29 | 19,48 |
| trouvé | 63,33 | 7,15 | 5,25 | 19,25 |

<u>IR</u> :
$\bar{\nu}$(C = O) = 1670 $cm^{-1}$
$\bar{\nu}$(C ≡ N) = 2150 $cm^{-1}$
<u>R.M.N.</u> (DMSO $d_6$) : δ = 1,1 (6H,d) ; 1,2 - 4,1 (14H,m) ; 6,6 (1H,t échangeable avec $CF_3COOD$) ; 7,1 (1H,t échangeable avec $CF_3COOD$) ; 7,1 - 7,5 (2H,m) ; 7,8 - 8,2 (2H,m).

**<u>Exemple 8 :</u>**

**<u>N-t-Butyl-N'-cyano-N"-[2-[4-(4-fluorobenzoyl)-1-pipéridinyl]éthyl]guanidine</u>**

Obtenue en opérant comme dans l'exemple 3 à partir de 5,0 g (0,020 mole) de 1-(2-aminoéthyl)-4-(4-fluorobenzoyl)pipéridine et de 3,4 g (0,020 mole) de N-t-butyl-N'-cyano-S-méthylisothiourée [préparée selon H.J. Pertersen, brevet allemand 2.557.438 ; C.A. (1976) 85, 142993e] dans 120 ml de 2-méthoxyéthanol. Durée du reflux : 37 heures. Rdt : 1,6 g (21 %), F = 181,5 - 183,5°C (éthanol).

Analyse centésimale : $C_{20}H_{28}FN_5O$ (M = 373,47)

| | C % | H % | F % | N % |
|---|---|---|---|---|
| calculé | 64,32 | 7,56 | 5,09 | 18,75 |

| | C % | H % | F % | N % |
|---|---|---|---|---|
| trouvé | 64,60 | 7,56 | 5,01 | 18,81 |

IR :
$\bar{\nu}$(C = O) = 1665 $cm^{-1}$
$\bar{\nu}$(C ≡ N) = 2150 $cm^{-1}$
R.M.N. ($CDCl_3$) : δ = 1,4 (9H,s) ; 1,5 - 3,6 (13H,m) ; 5,6 - 6,1 (1H,m échangeable avec $CF_3COOD$) ; 6,2 (1H, pic échangeable avec $CF_3COOD$) ; 6,9 - 7,3 (2H,m) ; 7,7 - 8,1 (2H,m).

**Exemple 9 :**

**N-Cyano-N'-[2-[4-(4-fluorobenzoyl)-1-piperidinyl]éthyl-N"-(2,2,2-trifluoroéthyl)guanidine**

a) N-Cyano-N'-(2,2,2-trifluoroéthyl)-S-méthylisothiourée

Un mélange de 8,0 g (0,055 mole) de N-cyanodithioiminocarbonate de méthyle, de 8,1 g (0,060 mole) de chlorhydrate de 2,2,2-trifluoroéthylamine et de 6,0 g (0,059 mole) de triéthylamine dans 75 ml d'éthanol est porté à 40°C pendant 7 heures. Il se produit un dégagement de méthylmercaptan. On rajoute ensuite dans le milieu réactionnel 3,7 g (0,027 mole) de chlorhydrate de 2,2,2-trifluoroéthylamine et 2,7 g (0,027 mole) de triéthylamine et poursuit le chauffage à 40°C pendant 4 heures. Les mêmes quantités de réactifs sont à nouveau ajoutées et le chauffage à 40°C est poursuivi pendant encore 3 heures. Après refroidissement, un très léger précipité est éliminé par filtration. Le filtrat est concentré à sec sous pression réduite. Le résidu est lavé à l'eau et recristallisé dans l'éthanol. Rdt : 4,9 g (45 %), F = 151 - 153°C.

| Analyse centésimale : $C_5H_6F_3N_3S$ (M = 197,18) | | | | | |
|---|---|---|---|---|---|
| | C % | H % | F % | N % | S % |
| calculé | 30,46 | 3,07 | 28,91 | 21,31 | 16,26 |
| trouvé | 30,58 | 3,21 | 28,98 | 21,18 | 16,43 |

IR :
$\bar{\nu}$(C ≡ N) = 2160 $cm^{-1}$
R.M.N. (DMSO $d_6$ + $CF_3COOD$) : δ = 2,6 (3H,s) ; 4,2 (2H,q).

b) N-Cyano-N'-[2-[4-(4-fluorobenzoyl)-1-pipéridinyl]éthyl]-N"-(2,2,2-trifluoroéthyl)guanidine

Une solution de 2,8 g (0,011 mole) de 1-(2-aminoéthyl)-4-(4-fluorobenzoyl)pipéridine et de 2,2 g (0,011 mole) de N-cyano-N'-(2,2,2-trifluoroéthyl)-S-méthylisothiourée dans 50 ml d'éthanol est agitée pendant 4 heures à température ambiante, puis pendant 3 heures 30 vers 55°C et enfin pendant 14 heures 30 à reflux. Il se produit un dégagement de méthylmercaptan. Le milieu réactionnel est ensuite refroidi vers 0°C. Le précipité formé est isolé par filtration ; il est lavé à l'éther et recristallisé dans l'éthanol. Rdt : 2,9 g (66%), F = 156 - 158° C.

| Analyse centésimale : $C_{18}H_{21}F_4N_5O$ (M = 399,39) | | | | |
|---|---|---|---|---|
| | C % | H % | F % | N % |
| calculé | 54,13 | 5,30 | 19,03 | 17,54 |
| trouvé | 54,04 | 5,38 | 18,95 | 17,55 |

IR :
$\bar{\nu}$(C = O) = 1660 $cm^{-1}$
$\bar{\nu}$(C ≡ N) = 2150 $cm^{-1}$
R.M.N. ($CDCl_3$) : δ = 1,5 - 3,5 (13H,m) ; 3,6 - 4,2 (2H,m se transforme en q avec $D_2O$) ; 6,6 - 7,0 (1H,m échangeable avec $D_2O$) ; 6,9 - 7,3 (2H,m) ; 7,7 - 8,2 (2H,m) ; 9,5 - 9,9 (1H,m échangeable avec $D_2O$).

**Exemple 10 :**

**N'-Cyano-N,N-diméthyl-N"-[2-[4-(4-fluorobenzoyl)-1-pipéridinyl]éthyl]guanidine**

Obtenue en opérant comme dans l'exemple 4 à partir de 5,0 g (0,020 mole) de 1-(2-aminoéthyl)-4-(4-fluorobenzoyl)pipéridine et de 4,4 g (0,030 mole) de N'-cyano-N,N-diméthyl-S-méthylisothiourée [préparée selon B.T. Heitke et C.G. Mc Carty, J. Org. Chem. (1974) 39,1522] dans 110 ml d'éthanol. Durée du reflux : 34 heures. Eluant de chromatographie : chlorure de méthylène 95 - méthanol 5. Rdt : 1,3 g (19 %), F = 139 - 141°C (éthanol-éther isopropylique).

| Analyse centésimale : $C_{18}H_{24}FN_5O$ (M = 345,42) | | | | |
|---|---|---|---|---|
| | C % | H % | F % | N % |
| calculé | 62,59 | 7,00 | 5,50 | 20,28 |
| trouvé | 62,65 | 7,03 | 5,42 | 20,13 |

IR :
$\bar{\nu}$(C = O) = 1650 $cm^{-1}$
$\bar{\nu}$(C ≡ N) = 2150 $cm^{-1}$
R.M.N. ($CDCl_3$) : δ = 1,5 - 3,2 (11H,m) ; 3,0 (6H,s) ; 3,5 - 3,9 (2H,m) ; 5,8 (1H, pic échangeable avec $D_2O$) ; 6,9 - 7,3 (2H,m) ; 7,7- 8,2 (2H,m).

**Exemple 11 :**

**N-Cyano-N'-[3-[4-[2-(4-fluorophényl)-1,3-dioxolan-2-yl]-1-pipéridinyl]propyl]-N"-méthylguanidine**

a) 3-[4-[2-(4-Fluorophényl)-1,3-dioxolan-2-yl]-1-pipéridinyl]propionitrile

On ajoute goutte à goutte 3,2 g (0,060 mole) d'acrylonitrile à 12,6 g (0,050 mole) de 4-[2-(4-fluorophényl)-1,3-dioxolan-2-yl]pipéridine [préparée selon B. Dumaitre et al., brevet européen 14.295 ; C.A. (1981) 94, 65699v] maintenue entre 55 et 60°C. Au bout de 15 minutes environ, il se forme un précipité qui est filtré. Le solide obtenu est lavé à l'hexane et séché. Il est utilisé dans l'étape suivante sans autre purification. Rdt : 14,4 g (95 %), F = 107 - 109°C.
R.M.N. ($CDCl_3$) : δ = 1,1 - 3,1 (13H,m) ; 3,5 - 4,2 (4H,m) ; 6,8 - 7,5 (4H,m).

b) 1-(3-Aminopropyl)-4-[2-(4-fluorophényl)-1,3-dioxolan-2-yl]pipéridine

Un mélange de 14,0 g (0,046 mole) de 3-[4-[2-(4-fluorophényl)-1,3-dioxolan-2-yl]-1-pipéridinyl]-propionitrile, de 46 g d'ammoniac liquide et de 6 g de nickel de Raney dans 300 ml de méthanol est introduit dans un autoclave et hydrogéné à température ambiante pendant 6 heures, la pression initiale d'hydrogène étant fixée à 85 bars. Après dégazage, le milieu réactionnel est filtré et concentré à sec sous pression réduite. Le résidu est purifié par distillation sous pression réduite. Rdt : 12,1 g (85 %), $Eb_{1,5}$ = 168 - 175°C.
R.M.N. ($CDCl_3$) : δ = 1,2 (2H,s échangeable avec $D_2O$) ; 1,2 - 3,1 (15H,m) ; 3,5 - 4,1 (4H,m); 6,8 - 7,5 (4H,m).

c) N-Cyano-N'-[3-[4-[2-(4-fluorophényl)-1,3-dioxolan-2-yl]-1-pipéridinyl]propyl]-N"-méthylguanidine

Obtenue en opérant comme dans l'exemple 3 à partir de 10,0 g (0,032 mole) de 1-(3-aminopropyl)-4-[2-(4-fluorophényl)-1,3-dioxolan-2-yl]pipéridine et de 4,1 g (0,032 mole) de N-cyano-N'-méthyl-S-méthylisothiourée dans 100 ml de 2-méthoxyéthanol. Rdt : 4,5 g (36 %), F = 179 - 181°C (éthanol).

| Analyse centésimale : $C_{20}H_{28}FN_5O_2$ (M = 389,47) | | | | |
|---|---|---|---|---|
| | C % | H % | F % | N % |
| calculé | 61,68 | 7,25 | 4,88 | 17,98 |
| trouvé | 61,72 | 7,49 | 4,66 | 17,84 |

IR :
$\bar{\nu}(C \equiv N)$ = 2150 $cm^{-1}$
R.M.N. (DMSO $d_6$) : δ = 2,2 (1H,t échangeable avec $CF_3COOD$) ; 2,6 (3H,d se transforme en s avec $CF_3COOD$) ; 1,2 - 3,4 (15H,m) ; 3,4 - 4,1 (4H,m) ; 6,9 (1H,t échangeable avec $CF_3COOD$) ; 6,9 - 7,6 (4H,m).

**Exemple 12 :**

**N-Cyano-N'-[3-[4-(4-fluorobenzoyl)-1-pipéridinyl]propyl]-N"-méthylguanidine**

a) 1-(3-Aminopropyl)-4-(4-fluorobenzoyl)pipéridine

A une solution de 11,8 g (0,0382 mole) de 1-(3-aminopropyl)-4-[2-(4-fluorophényl)-1,3-dioxolan-2-yl]- pipéridine dans 100 ml d'isopropanol, on ajoute goutte à goutte 52,5 g d'acide chlorhydrique 22 %. Le mélange obtenu est porté à reflux pendant 8 heures. Après refroidissement, le milieu réactionnel est concentré à sec sous pression réduite. Le résidu est dissous dans 100 ml d'eau ; cette solution aqueuse est basifiée à l'ammoniaque et extraite au chlorure de méthylène. Les extraits organiques rassemblés sont séchés sur sulfate de sodium et concentrés à sec sous pression réduite. Le résidu est utilisé dans l'étape suivante sans autre purification. Rdt : 10,6 g (quantitatif).
R.M.N. ($CDCl_3$) : δ = 1,5 - 3,6 (17H, m) ; 6,8 - 7,4 (2H,m) ; 7,7 - 8,2 (2H,m).

b) N-Cyano-N'-[3-[4-(4-fluorobenzoyl)-1-pipéridinyl]propyl]-N"-méthylguanidine

Obtenue en opérant comme dans l'exemple 4 à partir de 10,1 g (0,0382 mole) de 1-(3-aminopropyl)-4-(4-fluorobenzoyl)pipéridine et de 4,7 g (0,0362 mole) de N-cyano-N'-méthyl-S-méthylisothiourée dans 100 ml de 2-méthoxyéthanol. Durée du reflux : 5 heures 30. Eluant de chromatographie : chlorure de méthylène 98 - méthanol 2. Rdt : 3,7 g (30 %), F = 171 - 174°C (éthanol).

| Analyse centésimale : $C_{18}H_{24}FN_5O$ (M = 345,42) | | | | |
|---|---|---|---|---|
| | C % | H % | F % | N % |
| calculé | 62,59 | 7,00 | 5,50 | 20,27 |
| trouvé | 62,40 | 7,22 | 5,30 | 20,09 |

IR :
$\bar{\nu}$(C = O) = 1665 $cm^{-1}$
$\bar{\nu}$(C ≡ N) = 2150 $cm^{-1}$
R.M.N. (DMSO $d_6$) : δ = 2,3 (1H,t échangeable avec $CF_3COOD$) ; 2,7 (2H,d se transforme en s avec $CF_3COOD$) ; 1,4 - 3,8 (15H,m) ; 7,0 (1H,t échangeable avec $CF_3COOD$); 7,1 - 7,6 (2H,m) ; 7,8 - 8,3 (2H,m).

**Exemple 13 :**

**N'-Cyano-N,N-diméthyl-N"-[3-[4-(4-fluorobenzoyl)-1-pipéridinyl]propyl]guanidine**

Obtenue en opérant comme dans l'exemple 4 à partir de 7,8 g (0,0295 mole) de 1-(3-aminopropyl)-4-(4-fluorobenzoyl)pipéridine et de 3,5 g (0,0244 mole) de N'-cyano-N,N-diméthyl-S-méthylisothiourée dans 100 ml d'éthanol. Durée du reflux : 12 heures. Eluant de chromatographie : chlorure de méthylène 9 - méthanol 1. Rdt : 3,6 g (41 %), F = 89 - 92°C (éthanol-éther éthylique).

| Analyse centésimale : $C_{19}H_{26}FN_5O$ (M = 359,45) | | | | |
|---|---|---|---|---|
| | C % | H % | F % | N % |
| calculé | 63,49 | 7,29 | 5,29 | 19,48 |
| trouvé | 63,26 | 7,33 | 5,16 | 19,35 |

IR :
$\bar{\nu}$(C = O) = 1650 $cm^{-1}$
$\bar{\nu}$(C ≡ N) = 2150 $cm^{-1}$
R.M.N. ($CDCl_3$) : δ= 1,5 - 3,9 (21H,m) dont 3,0 (6H,s) et 3,6 (2H,m se transforme en t par $D_2O$) ; 6,8 - 7,5 (3H,m dont 1H échangeable avec $D_2O$) ; 7,7 - 8,2 (2H,m).

**Exemple 14 :**

**N-Cyano-N'-[4-[4-(4-fluorobenzoyl)-1-pipéridinyl]butyl]-N"-méthylguanidine**

Obtenue en opérant comme dans l'exemple 4 à partir de 4,9 g (0,0176 mole) de 1-(4-aminobutyl)-4-(4-fluorobenzoyl)pipéridine [préparée selon J. Vandenberk et al., brevet européen 184.258 ; C.A. (1986) 105, 133907 j] et de 2,05 g (0,0159 mole) de N-cyano-N'-méthyl-S-méthylisothiourée dans 50 ml de 2-méthoxyéthanol. Durée du chauffage : 6 heures 30 à 100°C. Eluant de chromatographie : chlorure de méthylène 9 - méthanol 1. Rdt : 2,2 g (38 %), solide amorphe.

| Analyse centésimale : $C_{19}H_{26}FN_5O$ (M = 359,45) | | | | |
|---|---|---|---|---|
| | C % | H % | F % | N % |
| calculé | 63,49 | 7,29 | 5,29 | 19,48 |
| trouvé | 63,51 | 7,55 | 5,39 | 19,42 |

IR :
$\bar{\nu}$(C = O) = 1660 $cm^{-1}$
$\bar{\nu}$(C ≡ N) = 2140 $cm^{-1}$
R.M.N. ($CDCl_3$) :δ = 0,8 - 3,6 (17H,m) ; 2,9 (3H,d se transforme en s avec $D_2O$) ; 5,8 (2H,pic échangeable avec $D_2O$) ; 6,9 - 7,3 (2H,m) ; 7,7 - 8,1 (2H,m).

**Exemple 15 :**

**N-Cyano-N'-[6-[4-(4-fluorobenzoyl)-1-pipéridinyl]hexyl]-N"-méthylguanidine**

a) 2-[6-[4-[2-(4-Fluorophényl)-1,3,dioxolan-2-yl]-1-pipéridinyl]hexyl]-1H-isoindol-1,3 (2H)-dione

Un mélange de 18,8 g (0,0748 mole) de 4-[(4-fluorophényl)-1,3-dioxolan-2-yl]pipéridine, de 25,6 g (0,0825 mole) de 2-(6-bromohexyl)-1H-isoindol-1,3(2H)-dione [préparée selon E.F. Elslager et al., J. Am. Chem. Soc. (1957) 79, 4699] et de 11,4 g (0,0825 mole) de carbonate de potassium dans 150 ml d'acétonitrile est porté à reflux pendant 3 heures. Après élimination par filtration du solide en suspension, la solution est concentrée à sec sous pression réduite. Le résidu obtenu est agité dans 500 ml d'éther éthylique. La suspension formée est filtrée ; le filtrat éthéré est évaporé pour fournir la 2-[6-[4-[2-(4-fluorophényl)-1,3-dioxolan-2-yl]-1-pipéridinyl]hexyl]-1H-isoindol-1,3(2 H)-dione. Rdt : 31,0 g (86 %), F = 72 - 76°C.
IR :
$\nu$(C = O) = 1700 $cm^{-1}$
R.M.N. ($CDCl_3$) : $\delta$ = 1,0 - 2,6 (17H,m) ; 2,7 - 3,2 (2H,m) ; 3,5 - 4,1 (6H,m) ; 6,7 - 8,0 (8H,m).

b) 1-(6-Aminohexyl)-4-[2-(4-fluorophényl)-1,3-dioxolan-2-yl]pipéridine

On ajoute goutte à goutte à température ambiante 3,6 g (0,0719 mole) d'hydrate d'hydrazine à une suspension de 30,5 g (0,0635 mole) de 2-[6-[4-[2-(4-fluorophényl)-1,3-dioxolan-2-yl]-1-pipéridinyl]hexyl]-1H-isoindol-1,3(2H)-dione dans 600 ml d'éthanol. L'agitation est poursuivie pendant 23 heures à température ambiante. On ra joute ensuite 1,0 g (0,0200 mole) d'hydrate d'hydrazine et poursuit l'agitation pendant encore 16 heures. Après élimination d'un précipité par filtration, la solution est concentrée sous pression réduite. La résidu est repris à l'eau et extrait au chlorure de méthylène. Les extraits organiques rassemblés sont séchés sur sulfate de sodium et concentrés à sec sous pression réduite. Le résidu est utilisé dans l'étape suivante sans autre purification. Rdt : 19,0 g (85 %).
R.M.N. ($CDCl_3$) : $\delta$ = 0,9 - 4,1 (27 H,m dont 2H échangeables avec $D_2O$) ; 6,7 - 7,6 (4H,m).

c) 1 -(6-Aminohexyl)-4-(4-fluorobenzoyl)pipéridine

A une solution de 19,0 g (0,0542 mole) de 1-(6-aminohexyl)-4-[2-(4-fluorophényl)-1,3-dioxolan-2-yl]-pipéridine dans 140 ml d'isopropanol, on ajoute goutte à goutte 75,0 g d'acide chlorhydrique 22 %. Le mélange obtenu est porté à reflux pendant 8 heures. Après refroidissement, le milieu réactionnel est concentré à sec sous pression réduite. Le résidu lavé à l'éther et séché, fournit le dichlorhydrate de 1-(6-aminohexyl)-4-(4-fluorobenzoyl)pipéridine. Rdt : 18,6 g (90 %), F = 205 -208°C.
IR :
$\nu$(C = O) = 1660 $cm^{-1}$
R.M.N. (DMSO $d_6$) : $\delta$ = 1,0 - 4,1 (23H,m dont 2H échangeables avec $CF_3COOD$) ; 7,1 - 7,6 (2H,m) ; 7,7 - 8,3 (2H,m); 11,0 (2H,pic échangeable avec $CF_3COOD$).
La base est obtenue par reprise du dichlorhydrate dans l'ammoniaque diluée et extraction au chlorure de méthylène. Rdt : 15,0 g (quantitatif).
IR :
$\nu$(C = O) = 1660 $cm^{-1}$
R.M.N. ($CDCl_3$) : $\delta$ = 0,9 - 3,6 (23H,m dont 2H échangeables avec $D_2O$) ; 6,8 - 7,3 (2H,m) ; 7,8 - 8,1 (2H,m).

d) N-Cyano-N'-[6-[4-(4-fluorobenzoyl)-1-pipéridinyl]hexyl]-N"-méthylguanidine

Obtenue en opérant comme dans l'exemple 4 à partir de 6,0 g (0,0196 mole) de 1-(6-aminohexyl)-4-(4-fluorobenzoyl)pipéridine et de 2,3 g (0,0178 mole) de N-cyano-N'-méthyl-S-méthylisothiourée dans 50 ml de 2-méthoxyéthanol. Durée du chauffage : 2 heures à 85°C puis 7 heures 30 à 115°C. Eluant de chromatographie : chlorure de méthylène 9 - méthanol 1. Rdt : 1,2 g (17 %), F = 121 - 123°C.

| Analyse centésimale : $C_{21}H_{30}FN_5O$ (M = 387,50) | | | | |
|---|---|---|---|---|
| | C % | H % | F % | N % |
| calculé | 65,09 | 7,80 | 4,90 | 18,07 |
| trouvé | 65,03 | 7,95 | 4,86 | 17,95 |

IR :
$\bar{\nu}$(C = O) = 1660 $cm^{-1}$
$\bar{\nu}$(C ≡ N) = 2160 $cm^{-1}$
R.M.N. ($CDCl_3$) : δ = 0,8 - 2,7 (17H,m) ; 2,8 (3H,d se transforme en s avec $D_2O$); 2,8 -3,5 (4H,m) ; 5,2 (1H,pic échangeable avec $D_2O$) ; 5,5 (1H, pic échangeable avec $D_2O$) ; 6,9 - 7,3 (2H,m) ; 7,7 - 8,1 (2H,m).

**Exemple 16 :**

**N,N'-Bis [2-[4-(4-fluorobenzoyl)-1-pipéridinyllethyl]-N"-cyanoguanidine**

Une solution de 3,2 g (0,0127 mole) de 1-(2-aminoéthyl)-4-(4-fluorobenzoyl)pipéridine dans 50 ml d'éthanol est ajoutée goutte à goutte à une suspension de 5,0 g (0,0127 mole) de N-cyano-N'-[2-[4-(4-fluorobenzoyl)-1-pipéridinyl]éthyl]phénoxyformamidine dans 60 ml d'éthanol. Le milieu réactionnel est agité pendant 11 heures à température ambiante, puis est concentré à sec sous pression réduite. Le résidu est purifié par chromatographie sur colonne de silice. Eluant : chlorure de méthylène 9 - méthanol 1. L'évaporation de l'éluat fournit la N,N'-bis[2-[4-(4-fluorobenzoyl)-1-pipéridinyl]éthyl]-N"-cyanoguanidine. Rdt : 1,1 g (16 %), F = 152 - 156°C (éthanol).

| Analyse centésimale : $C_{30}H_{36}F_2N_6O_2$ (M = 550,65) | | | | |
|---|---|---|---|---|
| | C % | H % | F % | N % |
| calculé | 65,44 | 6,59 | 6,90 | 15,26 |
| trouvé | 65,25 | 6,66 | 6,68 | 15,20 |

IR :
$\bar{\nu}$(C = O) = 1665 $cm^{-1}$
$\bar{\nu}$(C ≡ N) = 2160 $cm^{-1}$
R.M.N. ($CDCl_3$) : δ = 1,5 - 3,6 (26H,m) ; 6,7 - 7,3 (6H,m dont 2H échangeables avec $D_2O$); 7,6 - 8,2 (4H,m).

**Exemple 17 :**

**N-Cyano-N'-[3-[4-(4-fluorobenzoyl)-1-pipéridinyl]-2-hydroxy]propyl]-N"-méthylguanidine**

a) 1-Chloro-3-[4-[2-(4-fluorophényl)-1,3-dioxolan-2-yl]-1-pipéridinyl]propan-2-ol

A une suspension de 55,2 g (0,220 mole) de 4-[2-(4-fluorophényl)-1,3-dioxolan-2-yl]pipéridine dans 220 ml d'éther éthylique, on ajoute goutte à goutte 20,4 g (0,220 mole) d'épichlorhydrine. L'agitation est poursuivie à température ambiante pendant 12 heures après la fin de l'addition. Le précipité formé est isolé par filtration et séché. Il est utilisé dans l'étape suivante sans autre purification. Rdt : 60.0 g (79 %).
R.M.N. ($CDCl_3$ + $D_2O$) : δ = 1,2 - 4,1 (18H,m) ; 6,7 - 7,5 (4H,m).

b) 2-[3-[4-[2-(4-Fluorophényl)-1,3-dioxolan-2-yl]-1-pipéridinyl]-2-hydroxy]propyl]-1H-isoindol-1,3(2H)-dione

Un mélange de 59,6 g (0,173 mole) de 1-chloro-3-[4-[2-(4-fluorophényl)-1,3-dioxolan-2-yl]-1-pipéridinyl]-propan-2-ol et de 32,1 g (0,173 mole) de phtalimidate de potassium dans 710 ml de N,N-diméthylformamide

est porté à reflux pendant 2 heures. Après refroidissement, le milieu réactionnel est jeté dans 4,3 l d'eau. La suspension obtenue est alors agitée pendant 2 heures à température ambiante. Le précipité est ensuite isolé par filtration. Il est lavé à l'hexane et recristallisé dans un mélange d'hexane et d'acétate d'éthyle en présence de Norit. Rdt : 31,5 g (40 %), F = 124 -126°C.
IR :
$\bar{\nu}$(C = O) = 1690 $cm^{-1}$
R.M.N. ($CDCl_3$) : δ = 1,2 - 4,2 (18H,m) ; 6,7 - 7,5 (4H,m) ; 7,5 - 7,9 (4H,m).

c) 1-Amino-3-[4-[2-(4-fluorophényl)-1,3-dioxolan-2-yl]-1-pipéridinyl]propan-2-ol

On ajoute goutte à goutte à température ambiante 3,9 g (0,0779 mole) d'hydrate d'hydrazine à une suspension de 31,5 g (0,0693 mole) de 2-[3-[4-[2-(4-fluorophényl)-1,3-dioxolan-2-yl]-1-pipéridinyl]-2-hydroxy]propyl]-1H-isoindol-1,3(2H)-dione dans 650 ml d'éthanol. L'agitation est poursuivie pendant 60 heures à température ambiante. La solution obtenue est concentrée sous pression réduite. Le résidu est repris par 1 l d'éther éthylique. Le solide formé est isolé par filtration et lavé à l'éther. Il est ensuite extrait par 3 x 500 ml de chloroforme bouillant. La concentration de ces extraits chloroformiques fournit le 1-amino-3-[4-[2-(4-fluorophényl)-1,3-dioxolan-2-yl]-1-pipéridinyl]propan-2-ol que l'on utilise dans l'étape suivante sans autre purification. Rdt : 18,5 g (82 %), F = 132 - 136°C.
R.M.N. ($CDCl_3$) : δ = 1,3 - 4,1 (21H dont 3H échangeables avec $D_2O$) ; 6,8 - 7,5 (4H,m).

d) 1-Amino-3-[4-(4-fluorobenzoyl)-1-pipéridinyl]propan-2-ol

A une solution de 10,5 g (0,0324 mole) de 1-amino-3-[4-[2-(4-fluorophényl)-1,3-dioxolan-2-yl]-1-pipéridinyl]propan-2-ol dans 85 ml d'isopropanol, on ajoute goutte à goutte 44,1 g d'acide chlorhydrique 22 %. Le mélange obtenu est porté à reflux pendant 10 heures. Après refroidissement, le milieu réactionnel est concentré à sec sous pression réduite. Le résidu, recristallisé dans un mélange d'éthanol et d'éther éthylique, fournit le dichlorhydrate de 1-amino-3-[4-(4-fluorobenzoyl)-1-pipéridinyl]propan-2-ol. Rdt : 9,2 g (80 %), F = 189 - 192°C.

| Analyse centésimale : $C_{15}H_{23}Cl_2FN_2O_2$ (M = 353,26) | | | | | |
|---|---|---|---|---|---|
| | C % | H % | Cl % | F % | N % |
| calculé | 51,00 | 6,56 | 20,07 | 5,38 | 7,93 |
| trouvé | 50,75 | 6,69 | 19,98 | 5,17 | 7,82 |

IR :
$\bar{\nu}$(C = O) = 1665 $cm^{-1}$
R.M.N. (DMSO $d_6$ + $CF_3COOD$) : δ = 1,7 - 2,2 (4H,m) ; 2,6 - 4,6 (10H,m) ; 7,0 - 7,4 (2H,m) ; 7,8 - 8,2 (2H,m).
La base est obtenue par reprise du dichlorhydrate dans l'ammoniaque diluée et extraction au chlorure de méthylène. Rdt : 6,9 g (95 %), F = 89 - 92°C.
IR :
$\bar{\nu}$(C = O) = 1655 $cm^{-1}$
R.M.N. ($CDCl_3$) : δ = 1,6 - 4,0 (17H,m dont 3H échangeables avec $D_2O$) ; 6,9 - 7,3 (2H,m) ; 7,7 - 8,1 (2H,m).

e) N-Cyano-N'-[3-[4-(4-fluorobenzoyl)-1-pipéridinyl]-2-hydroxy]propyl]-N"-méthylguanidine

Une solution de 4,7 g (0,0168 mole) de 1-amino-3-[4-(4-fluorobenzoyl)-1-pipéridinyl]propan-2-ol dans 100 ml d'éthanol est ajoutée goutte à goutte à température ambiante à une solution de 2,5 g (0,0168 mole) de N-cyanodithioiminocarbonate de méthyle dans 100 ml d'éthanol. Le milieu réactionnel est agité pendant 24 heures à température ambiante. Il se produit un dégagement de méthylmercaptan. On ajoute ensuite goutte à goutte 14,4 ml (0,116 mole) d'une solution de méthylamine à 33 % dans l'éthanol. Le milieu réactionnel est agité pendant 20 heures à température ambiante. Il se produit un nouveau dégagement de méthylmercaptan. La solution obtenue est concentrée à sec sous pression réduite. Le résidu est purifié par chromatographie sur colonne de silice. Eluant : chlorure de méthylène 9 - méthanol 1. L'évaporation de l'éluat fournit la N-cyano-N'-[3-[4-(4-fluorobenzoyl)-1-pipéridinyl]-2-hydroxy]propyl]-N"-méthylguanidine. Rdt : 3,4 g (28 %), F = 141 - 145°C (acétate d'éthyle-éthanol).

| Analyse centésimale : $C_{18}H_{24}FN_5O_2$ (M = 361,42) | | | | |
|---|---|---|---|---|
| | C % | H % | F % | N % |
| calculé | 59,82 | 6,69 | 5,26 | 19,38 |
| trouvé | 59,64 | 6,53 | 5,35 | 19,60 |

IR :
$\bar{\nu}(C = O) = 1660\ cm^{-1}$
$\bar{\nu}(C \equiv N) = 2160\ cm^{-1}$
R.M.N. ($CDCl_3$) : δ = 1,7 - 2,6 (8H,m) ; 2,9 (3H, d se transforme en s avec $D_2O$) ; 2,8 -3,6 (5H,m) ; 3,7 - 4,3 (2H,m dont 1H échangeable avec $D_2O$) ; 6,2 (1H,pic échangeable avec $D_2O$) ; 6,9 (1H,pic échangeable avec $D_2O$) ; 6,9 - 7,4 (2H,m) ; 7,8 - 8,2 (2H,m).

**Exemple 18 :**

**N-Cyano-N'-[2-[4-(6-fluoro-1,2-benzisoxazol-3-yl)-1-pipéridinyl)éthyl]-N"-méthylguanidine**

a) 6-Fluoro-3-[1-(2-phtalimidoéthyl)-4-pipéridinyl]-1,2-benzisoxazole

Une suspension de 20,0 g (0,091 mole) de 6-fluoro-3-(4-pipéridinyl)-1,2-benzisoxazole [préparé selon J.T. Strupczewski et al., J. Med. Chem. (1985) 28, 761], de 23,0 g (0,091 mole) de N-(2-bromoéthyl)-phtalimide et de 12,6 g (0,091 mole) de carbonate de potassium dans 2,6 l d'acétonitrile est porté à reflux pendant 4 heures. Le milieu réactionnel est ensuite filtré à chaud et le gâteau est lavé à l'acétonitrile. Le filtrat et la solution de lavage rassemblés sont concentrés à sec sous pression réduite. Le résidu obtenu est repris à l'eau et extrait au chlorure de méthylène. Ces extraits organiques sont lavés à l'eau, séchés sur sulfate de sodium et concentrés à sec sous pression réduite. Le résidu est purifié par recristallisation dans l'acétate d'éthyle en présence de Norit. Rdt : 13,7 g (38 %), F = 143 - 145°C.

| Analyse centésimale : $C_{22}H_{20}FN_3O_3$ (M = 393,42) | | | | |
|---|---|---|---|---|
| | C % | H % | F % | N % |
| calculé | 67,17 | 5,12 | 4,83 | 10,68 |
| trouvé | 67,03 | 5,30 | 4,93 | 10,60 |

IR :
$\bar{\nu}(C = O) = 1690\ cm^{-1}$
R.M.N. ($CDCl_3$) : δ = 1,7 - 3,5 (11H,m) dont 2,7 (2H,t) ; 3,9 (2H,t) ; 6,7 - 8,1 (7H,m).

b) 3-[1-(2-Aminoéthyl)-4-pipéridinyl]-6-fluoro-1,2-benzisoxazole

On ajoute goutte à goutte 1,8 g (0,0360 mole) d'hydrate d'hydrazine à une suspension de 12,3 g (0,0313 mole) de 6-fluoro-3-[1-(2-phtalimidoéthyl)-4-pipéridinyl]-1,2-benzisoxazole dans 180 ml d'éthanol. Après 20 heures d'agitation à température ambiante, on additionne au milieu réactionnel 45 ml de potasse 10 % et on élimine l'éthanol par concentration sous pression réduite. La solution aqueuse résiduelle est extraite au chlorure de méthylène. Les extraits organiques sont séchés sur sulfate de sodium et concentrés à sec sous pression réduite. Le résidu solide obtenu est utilisé dans l'étape suivante sans autre purification. Rdt : 7,8 g (95 %), F= 61 - 65°C.
R.M.N. ($CDCl_3$) : δ = 1,5 - 3,5 (15H,m) dont 1,7 (2H, pic échangeable avec $D_2O$) ; 6,8 - 7,8 (3H,m).

c) N-Cyano-N'-[2-[4-(6-fluoro-1,2-benzisoxazol-3-yl)-1-pipéridinyl]éthyl]-S-méthylisothiourée

Obtenue en opérant comme dans le paragraphe a de l'exemple 1 à partir d'une solution de 2,1 g (0,0144 mole) de N-cyanocarbonimidodithioate de méthyle dans 40 ml d'éthanol et d'une solution de 4,0 g (0,0152 mole) de 3-[1-(2-aminoéthyl)-4-pipéridinyl]-6-fluoro-1,2-benzisoxazole dans 40 ml d'éthanol. Durée de l'agitation : 20 heures. Rdt : 4,2 g (81 %), F = 148 - 151°C (éthanol).

Analyse centésimale : $C_{17}H_{20}FN_5OS$ (M = 361,44)

| | C % | H % | F % | N % | S % |
|---|---|---|---|---|---|
| calculé | 56,49 | 5,58 | 5,26 | 19,38 | 8,87 |
| | C % | H % | F % | N % | S % |
| trouvé | 56,78 | 5,57 | 5,38 | 19,35 | 9,00 |

IR :
$\bar{\nu}$(C ≡ N) = 2150 $cm^{-1}$
R.M.N. ($CDCl_3$) : δ = 1,6 - 3,7 (16H,m) dont 2,5 (3H,s) et 3,5 (2H,m se transforme en t avec $D_2O$); 6,6 - 7,4 (3H,m dont 1H échangeable avec $D_2O$); 7.4 - 8,0 (1H,m).

d) N-Cyano-N'-[2-[4-(6-fluoro-1,2-benzisoxazol-3-yl)-1-pipéridinyl]éthyl]-N"-méthylguanidine

On ajoute 1,2 g (0,00332 mole) de N-cyano-N'-[2-[4-(6-fluoro-1,2-benzisoxazol-3-yl)-1-pipéridinyl]éthyl]-S-méthylisothiourée à 100 ml (0,803 mole) d'une solution de méthylamine à 33 % dans l'éthanol absolu. L'agitation est poursuivie pendant 20 heures à température ambiante ; il se produit un dégagement de méthylmercaptan. La solution obtenue est ensuite concentrée à sec sous pression réduite. Le résidu est lavé à l'éther et recristallisé dans l'éthanol en présence de Norit. Rdt : 0,8 g (70 %), F = 166 - 168°C.

| Analyse centésimale : $C_{17}H_{21}FN_6O$ (M = 344,39) | | | | |
|---|---|---|---|---|
| | C % | H % | F % | N % |
| calculé | 59,29 | 6,15 | 5,52 | 24,40 |
| trouvé | 59,27 | 6,25 | 5,21 | 24,27 |

IR :
$\bar{\nu}$(C ≡ N) = 2150 $cm^{-1}$
R.M.N ($CDCl_3$) : δ = 1,6 - 2,8 (8H,m) ; 2,8 (3H, d se transforme en s avec $D_2O$) ; 2,9 - 3,6 (5H,m) ; 6.0 (1H,pic échangeable avec $D_2O$) ; 6,8 - 8,0 (3H,m) ; 7,9 (1H, pic échangeable avec $D_2O$).

**Exemple 19 :**

**N-Cyano-N'-[3-[4-(6-fluoro-1,2-benzisoxazol-3-yl)-1-pipéridinyl]propyl]-N"-méthylguanidine**

a) 6-Fluoro-3-[1-(3-phtalimidopropyl)-4-pipéridinyl]-1,2-benzisoxazole

Obtenu en opérant comme dans le paragraphe a de l'exemple 18 à partir de 288,0 g (1,308 mole) de 6-fluoro-3-(4-pipéridinyl)-1,2-benzisoxazole, de 350,6 g (1,308 mole) de N-(3-bromopropyl)phtalimide et de 180,6 g (1,307 mole) de carbonate de potassium dans 2,6 l d'acétonitrile. Durée du reflux : 5 heures 30. Rdt : 357,7 g (67 %), F = 128 - 130°C (acétate d'éthyle).

| Analyse centésimale : $C_{23}H_{22}FN_3O_3$ (M = 407,44) | | | | |
|---|---|---|---|---|
| | C % | H % | F % | N % |
| calculé | 67,80 | 5,44 | 4,66 | 10,31 |
| trouvé | 67,60 | 5,42 | 4,73 | 10,30 |

IR :
$\bar{\nu}$(C = O) = 1690 $cm^{-1}$
R.M.N. ($CDCl_3$) : δ = 1,6 - 3,3 (13H,m); 3,75 (2H,t) ; 6,7 - 7,3 (2H,m) ; 7,4 - 8.0 (5H,m).

b) 3-[1-(3-Aminopropyl)-4-pipéridinyl]-6-fluoro-1,2-benzisoxazole

Obtenu en opérant comme dans le paragraphe b de l'exemple 18 à partir de 46,6 g (0,931 mole) d'hydrate d'hydrazine et de 330,0 g (0,810 mole) de 6-fluoro-3-[1(3-phtalimidopropyl)-4-pipéridinyl]-1,2-benzisoxazole dans 4,7 l d'éthanol. Rdt : 204,6 g (91 %), F = 59 - 62°C.
R.M.N. ($CDCl_3$) : δ = 1,4 - 3,6 (17H,m) dont 1,8 (2H, pic échangeable avec $D_2O$) ; 6,8 - 7,4 (2H,m) ; 7,5 -7,9 (1H,m).

c) N-Cyano-N'-[3-[4-(6-fluoro-1,2-benzisoxazol-3-yl)-1-pipéridinyl]propyl]-N"-méthylguanidine

Une solution de 130,2 g (0,469 mole) de 3-[1-(3-aminopropyl)-4-pipéridinyl]-6-fluoro-1,2-benzisoxazole dans 450 ml d'éthanol est ajoutée goutte à goutte à une solution de 64,5 g (0,441 mole) de N-cyanocarbonimidodithioate de méthyle dans 1,2 l d'éthanol. L'agitation est poursuivie pendant 22 heures à température ambiante ; il se produit un dégagement de méthylmercaptan. Le milieu réactionnel est ensuite additionné de 520 ml (4,176 moles) d'une solution de méthylamine à 33 % dans l'éthanol absolu et porté à 60°C pendant 3 heures. Il se produit un nouveau dégagement de méthylmercaptan. Après refroidissement vers -10°C. le précipité formé est isolé par filtration. Rdt : 137,0 g (87 %), F = 177 - 180°C (éthanol).

| Analyse centésimale : $C_{18}H_{23}FN_6O$ (M = 358,42) | | | | |
|---|---|---|---|---|
| | C % | H % | F % | N % |
| calculé | 60,32 | 6,47 | 5,30 | 23,45 |
| trouvé | 60,23 | 6,77 | 5,48 | 23,26 |

IR :
$\bar{\nu}$(C ≡ N) = 2150 $cm^{-1}$
R.M.N. (DMSO $d_6$) : δ = 1,5 - 2,6 (10H,m) ; 2,7 (3H, d se transforme en s avec $D_2O$); 2,7 - 3,4 (5H,m) ; 7,0 (2H, pic échangeable avec $D_2O$) ; 6,9 - 8,1 (3H,m).

**Exemple 20 :**

**N-Cyano-N'-éthyl-N"-[3-[4-(6-fluoro-1,2-benzisoxazol-3-yl)-1-pipéridinyl]propyl]guanidine**

a) N-Cyano-N'-[3-[4-(6-fluoro-1,2-benzisoxazol-3-yl)-1-pipéridinyl]propyl]-S-méthylisothiourée

Obtenue en opérant comme dans le paragraphe a de l'exemple 1 à partir d'une solution de 12,8 g (0,0875 mole) de N-cyanocarbonimidodithioate de méthyle dans 240 ml d'éthanol et d'une solution de 25,8 g (0,0930 mole) de 3-[1-(3-aminopropyl)-4-pipéridinyl]-6-fluoro-1,2-benzisoxazole dans 240 ml d'éthanol. Durée de l'agitation : 18 heures. Rdt : 24,1 g (73 %), F = 142 - 145°C (éthanol).

| Analyse centésimale : $C_{18}H_{22}FN_5OS$ (M = 375,47) | | | | | |
|---|---|---|---|---|---|
| | C % | H % | F % | N % | S % |
| calculé | 57,58 | 5,91 | 5,06 | 18,65 | 8,54 |
| trouvé | 57,42 | 6,08 | 5,38 | 18,62 | 8,27 |

IR :
$\bar{\nu}$(C ≡ N) = 2160 $cm^{-1}$
R.M.N. ($CDCl_3$ + $D_2O$) : δ = 1,6 - 3,3 (16H,m) ; 3,5 (2H,t) ; 6,8 - 7,4 (2H,m) ; 7,4 - 8,2 (1H,m).

b) N-Cyano-N'-éthyl-N"-[3-[4-(6-fluoro-1,2-benzisoxazol-3-yl)-1-pipéridinyl]propyl]guanidine

Un mélange de 3,0 g (0,00799 mole) de N-cyano-N'-[3-[4-(6-fluoro-1,2-benzisoxazol-3-yl)-1-pipéridinyl]-propyl]-S-méthylisothiourée et de 25 ml (0,377 mole) d'éthylamine est agité pendant 3 heures à température ambiante. Il se produit un dégagement de méthylmercaptan. L'éthylamine s'étant évaporée, on en rajoute la même quantité que précédemment et poursuit l'agitation pendant 1 heure. Après évaporation de l'éthylamine, le résidu est recristallisé dans l'éthanol. Rdt : 2,6 g (87 %), F= 153 -155°C.

| Analyse centésimale : $C_{19}H_{25}FN_6O$ (M = 372,45) | | | | |
|---|---|---|---|---|
| | C % | H % | F % | N % |
| calculé | 61,27 | 6,77 | 5,10 | 22,56 |
| trouvé | 61,16 | 6,79 | 5,16 | 22,65 |

IR :
$\bar{\nu}$(C ≡ N) = 2150 $cm^{-1}$
R.M.N. (DMSO $d_6$) : δ = 1,1 (3H,t) ; 1,4 - 2,5 (10H,m) ; 2,7 - 3,5 (7H,m) ; 6,7 - 8,2 (5H,m dont 2H échangeables avec $D_2O$).

**Exemple 21 :**

**N'-Cyano-N,N-diméthyl-N"-[3-[4-(6-fluoro-1,2-benzisoxazol-3-yl)-1-pipéridinyl]propyl]guanidine**

Obtenue en opérant comme dans l'exemple 4 à partir de 5,8 g (0,0209 mole) de 3-[1-(3-aminopropyl)-4-pipéridinyl]-6-fluoro-1,2-benzisoxazole et de 3,0 g (0,0209 mole) de N'-cyano-N,N-diméthyl-S-méthylisothiourée dans 70 ml d'éthanol. Durée du reflux : 9 heures 30. Eluant de chromatographie : chlorure de méthylène 95 - méthanol 5. Rdt : 3,8 g (49 %), F = 108 - 111°C.

Analyse centésimale : $C_{19}H_{25}FN_6O$ (M = 372,45)

| | C % | H % | F % | N % |
|---|---|---|---|---|
| calculé | 61,27 | 6,77 | 5,10 | 22,56 |
| trouvé | 61,24 | 6,97 | 5,10 | 22,57 |

IR :
$\bar{\nu}$(C ≡ N) = 2140 $cm^{-1}$

R.M.N. ($CDCl_3$) : δ = 1,6 - 3,3 (19H,m) dont 3,0 (6H,s); 3,4 - 3,9 (2H,m se transforme en t avec $D_2O$) ; 6,7 - 7,4 (3H,m dont 1H échangeable avec $D_2O$) ; 7,4 - 7,9 (1H,m).

**Exemple 22 :**

**N-Cyano-N'-[3-[4-(6-fluoro-1,2-benzisoxazol-3-yl)-1-pipéridinyl]propyl]guanidine**

Une solution de 1,5 g (0,00541 mole) de 3-[1-(3-aminopropyl)-4-pipéridinyl]-6-fluoro-1,2-benzisoxazole et de 0,6 g (0,00521 mole) de N-cyano-S-méthylisothiourée dans 50 ml d'éthanol est portée à reflux pendant 8 heures. Après refroidissement vers -10°C, le précipité formé est isolé par filtration. Il est purifié par chromatographie sur colonne de silice (éluant : chlorure de méthylène 95 - méthanol 5) puis par recristallisation dans l'éthanol. Rdt : 0,5 g (28 %), F = 178 - 181°C.

| Analyse centésimale : $C_{17}H_{21}FN_6O$ (M = 344,39) | | | | |
|---|---|---|---|---|
| | C % | H % | F % | N % |
| calculé | 59,29 | 6,15 | 5,52 | 24,40 |
| trouvé | 59,01 | 6,51 | 5,71 | 24,70 |

IR :
$\bar{\nu}(C \equiv N)$ = 2155 $cm^{-1}$
R.M.N. (DMSO $d_6$) : δ = 1,3 - 3,5 (15H,m) ; 6,7 (3H,pic élargi échangeable avec $CF_3COOD$) ; 6,9 - 8,2 (3H,m).

**Exemple 23 :**

**N-Cyano-N'-(3,3-diméthyl-2-butyl)-N"-[3-[4-(6-fluoro-1,2-benzisoxazol-3-yl)-1-pipéridinyl]propyl]guanidine**

a) N-Cyano-N'-(3,3-diméthyl-2-butyl)-S-méthylisothiourée

Une solution de 2,0 g (0,0137 mole) de N-cyanodithioiminocarbonate de méthyle et de 1,4 g (0,0138 mole) de 2-amino-3,3-diméthylbutane dans 50 ml d'éthanol est agitée pendant 3 jours à température ambiante. Il se produit un dégagement de méthylmercaptan. Après refroidissement vers -10°C, le précipité formé est isolé par filtration. Rdt : 1,6 g (59 %), F = 143 - 146°C (éthanol).

| Analyse centésimale : $C_9H_{17}N_3S$ (M = 199,32) | | | | |
|---|---|---|---|---|
| | C % | H % | N % | S % |
| calculé | 54,23 | 8,60 | 21,08 | 16,09 |
| trouvé | 54,27 | 8,64 | 21,02 | 16,18 |

IR :
$\bar{\nu}(C \equiv N)$ = 2150 $cm^{-1}$
R.M.N. ($CDCl_3$) : δ = 0,9 (9H,s) ; 1,2 (3H,d) ; 2,5 (3H,s) ; 3,7 (1H,m) ; 5,9 (1H,pic élargi échangeable avec $D_2O$).

b) N-Cyano-N'-(3,3-diméthyl-2-butyl)-N"-[3-[4-(6-fluoro-1,2-benzisoxazol-3-yl)-1-pipéridinyl]propyl]guanidine

Obtenue en opérant comme dans l'exemple 4 à partir de 1,2 g (0,00433 mole) de 3-[1-(3-aminopropyl)-4-pipéridinyl]-6-fluoro-1,2-benzisoxazole et de 0,8 g (0,00401 mole) de N-cyano-N'-(3,3-diméthyl-2-butyl)-S-méthylisothiourée dans 50 ml de 2-méthoxyéthanol. Durée du reflux : 10 heures. Eluant de chromatographie : chlorure de méthylène 95 - méthanol 5. Rdt : 0,7 g (41 %), F= 164 - 166°C (éthanol).

| Analyse centésimale : $C_{23}H_{33}FN_6O$ (M = 428,56) | | | | |
|---|---|---|---|---|
| | C % | H % | F % | N % |
| calculé | 64,46 | 7,76 | 4,43 | 19,61 |
| trouvé | 64,65 | 7,74 | 4,35 | 19,39 |

IR :
$\bar{\nu}(C \equiv N) = 2150\ cm^{-1}$
R.M.N. ($CDCl_3$) : δ = 1,0 (9H,s) ; 1,2 (3H,d) ; 1,3 - 4,0 (15H,m) ; 4,7 - 5,4 (1H,m) ; 6,7 - 7,4 (4H,m dont 2H échangeables par $D_2O$) ; 7,6 - 8,0 (1H,m).

**Exemple 24 :**

**N-[2-[4-(4-Fluorobenzoyl)-1-pipéridinyl]éthyl]-N'-méthyl-2-nitro-1,1-éthènediamine**

a) N-[2-[4-(4-Fluorobenzoyl)-1-pipéridinyl]éthyl]-1-(méthylthio)-2-nitroéthènamine

Une suspension de 11,0 g (0,044 mole) de 1-(2-aminoéthyl)-4-(4-fluorobenzoyl)pipéridine et de 7,3 g (0,044 mole) de 1,1-bis(méthylthio)-2-nitroéthylène dans 100 ml d'acétonitrile est portée à reflux pendant 7 heures. Il se produit un dégagement de méthylmercaptan. Après refroidissement, la solution obtenue est concentrée à sec sous pression réduite. Le résidu est concrétisé dans l'acétate d'éthyle glacé et recristallisé dans l'isopropanol. Rdt : 7,4 (46 %), F = 106 - 110°C. Un échantillon analytique a été préparé par chromatographie sur colonne de silice. Eluant : chlorure de méthylène 98 -méthanol 2. F = 123 - 125°C (éthanol).

| Analyse centésimale : $C_{17}H_{22}FN_3O_3S$ (M = 367,44) | | | | | |
|---|---|---|---|---|---|
| | C % | H % | F % | N % | S % |
| calculé | 55,57 | 6,04 | 5,17 | 11,44 | 8,73 |
| trouvé | 55,42 | 6,03 | 5,05 | 11,34 | 8,66 |

IR :
$\bar{\nu}(C = O) = 1665\ cm^{-1}$
R.M.N. ($CDCl_3$) : δ = 1,6 - 3,3 (11H,m) 2,4 (3H,s) ; 3,5 (2H,q se transforme en t avec $D_2O$) ; 6,5 (1H,s) ; 6,9 - 7,3 (2H,m) ; 7,8 - 8,1 (2H,m); 10,6 (1H,pic échangeable avec $D_2O$).

b) N-[2-[4-(4-Fluorobenzoyl)-1-pipéridinyl]éthyl]-N'-méthyl-2-nitro-1,1-éthènediamine

On ajoute 6,4 g (0,0174 mole) de N-[2-[4-(4-fluorobenzoyl)-1-pipéridinyl]éthyl]-1-(méthylthio)-2-nitroéthènamine à 18 ml (0,145 mole) d'une solution de méthylamine à 33 % dans l'éthanol. La solution obtenue est tiédie très progressivement. Dès 30°C, un précipité abondant apparaît, accompagné d'un important dégagement de méthylmercaptan. L'agitation est ensuite poursuivie pendant 2 heures à température ambiante. Le précipité formé est isolé par filtration. Il est lavé à l'éther isopropylique et recristallisé dans l'éthanol en présence de Norit. Rdt : 3,6 g (59 %), F = 157 - 159°C.

| Analyse centésimale : $C_{17}H_{23}FN_4O_3$ (M = 350,39) | | | | |
|---|---|---|---|---|
| | C % | H % | F % | N % |
| calculé | 58,27 | 6,62 | 5,42 | 15,99 |
| trouvé | 58,39 | 6,66 | 5,35 | 16,09 |

IR :
$\bar{\nu}$(C = O) = 1660 $cm^{-1}$
R.M.N. ($CDCl_3$) : δ = 1,5 - 3,6 (13H,m) ; 2,8 (3H, d se transforme en s avec $D_2O$) ; 6,5 (1H,s) ; 6,8 - 7,3 (2H,m) ; 7,7 - 8,2 (2H,m) ; 9,4 (1H,pic échangeable avec $D_2O$); 10,2 (1H,pic échangeable avec $D_2O$).

**Exemple 25 :**

**N-[3-[4-(4-Fluornbenzoyl)-1-pipéridinyl]propyl]-N'-méthyl-2-nitro-1,1-éthènediamine**

a) N-[3-[4-(4-Fluorobenzoyl)-1-pipéridinyl]propyl]-1-(méthylthio)-2-nitroéthènamine

Un mélange de 6,0 g (0,0227 mole) de 1-(3-aminopropyl)-4-(4-fluorobenzoyl)pipéridine et de 3,1 g (0,0188 mole) de 1,1-bis(méthylthio)-2-nitroéthylène dans 80 ml d'acétonitrile est porté à reflux pendant 6 heures. Il se produit un dégagement de méthylmercaptan. Après refroidissement, la solution obtenue est concentrée à sec sous pression réduite. Le résidu huileux est purifié par chromatographie sur colonne de silice. Eluant : chlorure de méthylène 95 - méthanol 5. Rdt : 1,9 g (26 %), F = 92 - 94°C (éthanol).

| Analyse centésimale : $C_{18}H_{24}FN_3O_3S$ (M = 381,47) | | | | | |
|---|---|---|---|---|---|
| | C % | H % | F % | N % | S % |
| calculé | 56,68 | 6,34 | 4,98 | 11,02 | 8,40 |
| trouvé | 56,45 | 6,47 | 4,85 | 10,83 | 8,70 |

IR :
$\bar{\nu}$(C = O) = 1660 $cm^{-1}$
R.M.N. ($CDCl_3$) : δ = 1,5 - 3,8 (18H,m) dont 2,4 (3H,s) et 3,5 (2H,q se transforme en t avec $D_2O$) ; 6,5 (1H,s) ; 6,9 - 7,3 (2H,m) ; 7,8 - 8,2 (2H,m) ; 10,6 (1H,pic échangeable avec $D_2O$).

b) N-[3-[4-(4-Fluorobenzoyl)-1-pipéridinyl]propyl]-N'-méthyl-2-nitro-1,1-éthènediamine

Un mélange de 1,7 g (0,00446 mole) de N-[3-[4-(4-fluorobenzoyl)-1-pipéridinyl]propyl]-1-(méthylthio)-2-nitroéthènamine et de 20 ml (0,161 mole) d'une solution de méthylamine à 33 % dans l'éthanol absolu est agité pendant 6 heures à température ambiante. Après deux jours de repos, le milieu réactionnel est filtré. Le gâteau est lavé à l'éther et purifié par chromatographie sur colonne de silice. Eluant : chlorure de méthylène 95 - méthanol 5. Rdt : 1,0 g (62 %), F= 160 - 162°C.

| Analyse centésimale : $C_{18}H_{25}FN_4O_3$ (M = 364,42) | | | | |
|---|---|---|---|---|
| | C % | H % | F % | N % |
| calculé | 59,33 | 6,92 | 5,21 | 15,37 |
| trouvé | 59,32 | 7,03 | 5,33 | 15,56 |

IR :
$\bar{\nu}$(C = O) = 1665 $cm^{-1}$
R.M.N. ($CDCl_3$) : δ = 1,5 - 3,7 (18H,m) ; 6,6 (1H,s) ; 6,9 - 7,4 (2H,m) ; 7,6 (1H,pic échangeable avec $D_2O$) ; 7,8 - 8,2 (2H,m) ; 10,2 (1H,pic échangeable avec $D_2O$).

**Exemple 26 :**

**N-[2-[4-(6-Fluoro-1,2-benzisoxazol-3-yl)-1-pipéridinyl]éthyl]-N'-méthyl-2-nitro-1,1-éthènediamine**

a) N-[2-[4-(6-Fluoro-1,2-benzisoxazol-3-yl)-1-pipéridinyl]éthyl]-1-(méthylthio)-2-nitroéthènamine

Obtenue en opérant comme dans le paragraphe a de l'exemple 25 à partir de 3,7 g (0,0141 mole) de 3-[1-(2-aminoéthyl)-4-pipéridinyl]-6-fluoro-1,2-benzisoxazole et de 2,2 g (0,0133 mole) de 1,1-bis(méthylthio)-2-nitroéthylène dans 55 ml d'acétonitrile. Durée du reflux : 13 heures 30. Eluant de chromatographie : chlorure de méthylène 98 -méthanol 2. Rdt : 1,3 g (26 %), F = 150 - 154°C.

| Analyse centésimale : $C_{17}H_{21}FN_4O_3S$ (M = 380,44) | | | | | |
|---|---|---|---|---|---|
| | C % | H % | F % | N % | S % |
| calculé | 53,67 | 5,56 | 4,99 | 14,73 | 8,43 |
| trouvé | 53,61 | 5,73 | 4,93 | 14,39 | 8,41 |

R.M.N. ($CDCl_3$) : δ = 1,8 - 3,3 (14H,m) dont 2,4 (3H,s) et 2,7 (2H,t) ; 3,5 (2H,q se transforme en t avec $D_2O$) ; 6,5 (1H,s) ; 6,8 - 7,3 (2H,m) ; 7,6 - 8,0 (1H,m) ; 10,8 (1H,pic échangeable avec $D_2O$).

b) N-[2-[4-(6-Fluoro-1,2-benzisoxazol-3-yl)-1-pipéridinyl]éthyl]-N'-méthyl-2-nitro-1,1-éthènediamine

Un mélange de 1,0 g (0,00263 mole) de N-[2-[4-(6-fluoro-1,2-benzisoxazol-3-yl)-1-pipéridinyl]éthyl]-1-(méthylthio)-2-nitroéthènamine et de 75 ml d'une solution de méthylamine à 33 % dans l'éthanol absolu est agité pendant 48 heures à température ambiante. Il se produit un dégagement de méthylmercaptan. Après refroidissement vers -10°C, le précipité formé est isolé par filtration ; il est lavé à l'éther et recristallisé dans l'éthanol. Rdt : 0,7 g (73 %), F= 181 - 182,5°C.

| Analyse centésimale : $C_{17}H_{22}FN_5O_3$ (M = 363,39) | | | | |
|---|---|---|---|---|
| | C % | H % | F % | N % |
| calculé | 56,19 | 6,10 | 5,23 | 19,27 |
| trouvé | 56,44 | 5,97 | 5,51 | 19,26 |

R.M.N. ($CDCl_3$) : δ = 1,5 - 3,7 (16H,m) dont 2,8 (3H,s après échange avec $D_2O$) ; 6,5 (1H,s) ; 6,8 - 7,4 (2H,m) ; 7,4 - 7,8 (1H,m) ; 9,1 (1H,pic échangeable avec $D_2O$) ; 10,2 (1H,pic échangeable avec $D_2O$).

**Exemple 27 :**

**N-Cyano-N'-[2-[4-[2-(4-flurorophényl)-1,3-dioxolan-2-yl]-1-ipéridinyl]éthyl]pipéridinoformamidine**

Obtenue en opérant comme dans l'exemple 3 à partir de 2,0 g (0,068 mole) de 1-(2-aminoéthyl)-4-[2-(4-fluorophényl)-1,3-dioxolan-2-yl]pipéridine et de 1,25 g (0,068 mole) de N-cyano-S-méthyl-pipéridinothioformamide [préparé selon R. Neidlein et U. Askani, Arch. Pharm (Weinheim) (1977) 310, 820] dans 40 ml d'éthanol. Durée du reflux : 34 heures. Rdt : 1,0 g (34 %), F = 155 - 157°C (éthanol-éther éthylique).

| Analyse centésimale : $C_{23}H_{32}FN_5O_2$ (M = 429,54) | | | | |
|---|---|---|---|---|
| | C % | H % | F % | N % |
| calculé<br>trouvé | 64,31<br>64,07 | 7,51<br>7,47 | 4,42<br>4,32 | 16,30<br>16,03 |

IR :
$\bar{\nu}(C \equiv N) = 2150\ cm^{-1}$
R.M.N. ($CDCl_3$) : δ = 1,3 - 3,6 (23H,m) ; 3,5 - 4,1 (4H,m) ; 5,6 - 5,9 (1H,m échangeable avec $CF_3$ COOD) ; 6,7 - 7,5 (4H,m).

**Exemple 28 :**

**N-Cyano-N'-[2-[4-(4-flurorobenzoyl)-1-pipéridinyl]éthyl]-pipéridinoformamidine**

Obtenue en opérant comme dans l'exemple 4 à partir de 5,0 g (0,020 mole) de 1-(2-aminoéthyl)-4-(4-fluorobenzoyl)pipéridine et de 3,7 g (0,020 mole) de N-cyano-S-méthyl-pipéridinothioformamide dans 50 ml d'éthanol. Durée du reflux : 13 heures. Eluant de chromatographie : chlorure de méthylène 98 - méthanol 2. Rdt : 1,6 g (21 %), F = 139 - 141°C (éthanol-éther isopropylique).

| Analyse centésimale : $C_{21}H_{28}FN_5O$ (M = 385,48) | | | | |
|---|---|---|---|---|
| | C % | H % | F % | N % |
| calculé<br>trouvé | 65,43<br>65,33 | 7,32<br>7,32 | 4,93<br>4,87 | 18,17<br>18,19 |

IR :
$\bar{\nu}(C = O) = 1670\ cm^{-1}$
$\bar{\nu}(C \equiv N) = 2150\ cm^{-1}$
R.M.N. ($CDCl_3$) : δ = 1,4 - 3,8 (23H,m) ; 5,7 (1H,pic échangeable avec $D_2O$); 6,9 - 7,4 (2H,m) ; 7,8 - 8,2 (2H,m).

**Exemple 29 :**

**N-Cyano-N'-méthyl-[4-(4-fluorobenzoyl)-1-pipéridinyl]formamidine**

a) N-Cyano-S-méthyl-[4-(4-fluorobenzoyl)-1-pipéridinyl]thioformamide

Une solution de 3,0 g (0,0145 mole) de 4-(4-fluorobenzoyl) pipéridine dans 30 ml d'éthanol est ajoutée goutte à goutte à une solution de 2,1 g (0,0145 mole) de N-cyanocarbonimidodithioate de méthyle dans 50 ml d'éthanol. L'agitation est poursuivie pendant 7 heures à température ambiante. Il se produit un dégagement de méthylmercaptan. La solution obtenue est ensuite concentrée à sec sous pression réduite. Le résidu est lavé à l'éther isopropylique et recristallisé dans un mélange d'éthanol et d'éther isopropylique. Rdt : 2,5 g (56 %), F = 101 - 103°C (éthanol).
IR :
$\bar{\nu}(C = O) = 1670\ cm^{-1}$
$\bar{\nu}(C \equiv N) = 2160\ cm^{-1}$
R.M.N. ($CDCl_3$) : δ = 1,7 - 2,2 (4H,m) ; 2,8 (3H,s) ; 3,1 - 3,8 (3H,m) ; 4,3 - 4,8 (2H,m) ; 7,0 - 7,4 (2H,m) ; 7,8 - 8,2 (2H,m).

b) N-Cyano-N'-méthyl-[4-(4-fluorobenzoyl)-1-pipéridinyl]thioformamidine

A une suspension de 2,4 g (0,0079 mole) de N-cyano-S-méthyl-[4-(4-fluorobenzoyl)-1-pipéridinyl]-thioformamide dans 50 ml d'éthanol, on ajoute goutte à goutte 16 ml (0,129 mole) d'une solution de méthylamine à 33 % dans l'éthanol. La solution obtenue est agitée pendant 5 heures à température ambiante. Il se produit un dégagement de méthylmercaptan. Le milieu réactionnel est ensuite concentré à sec sous pression réduite. Le résidu est lavé à l'éther isopropylique et recristallisé dans un mélange d'éthanol et d'éther isopropylique en présence de Norit. Rdt : 1,3 g (57 %), F = 128 - 130°C.

Analyse centésimale : $C_{15}H_{17}FN_4O$ (M = 288,33)

| | C % | H % | F % | N % |
|---|---|---|---|---|
| calculé | 62,49 | 5,94 | 6,59 | 19,43 |

| | C % | H % | F % | N % |
|---|---|---|---|---|
| trouvé | 62,42 | 6,01 | 6,85 | 19,58 |

IR :
$\bar{\nu}$(C = O) = 1665 $cm^{-1}$
$\bar{\nu}$(C ≡ N) = 2150 $cm^{-1}$
R.M.N. ($CDCl_3$) : δ = 1,6 - 2,1 (4H,m) ; 2,9 - 3,8 (3H,m) ; 3,0 (3H,d se transforme en s avec $CF_3COOD$) ; 3,8 - 4,3 (2H,m) ; 5,6 - 6,1 (1H,m échangeable avec $CF_3COOD$) ; 6,9 - 7,4 (2H,m) ; 7,7 - 8,2 (2H,m).

**Exemple 30 :**

**N-Cyano-N'-[2-[4-(4-fluorobenzoyl)-1-pipéridinyl]éthyl]-[4-(4-fluorobenzoyl)-1-pipéridinyl]formamidine**

Obtenue en opérant comme dans l'exemple 4 à partir de 5,0 g (0,020 mole) de 1-(2-aminoéthyl)-4-(4-fluorobenzoyl)pipéridine et de 3,6 g (0,012 mole) de N-cyano-S-méthyl-[4-(4-fluorobenzoyl)-1-pipéridinyl]-thioformamide dans 100 ml d'éthanol. Durée du reflux : 21 heures 30. Eluant de chromatographie : chlorure de méthylène 95 -méthanol 5. Rdt : 1,1 g (18 %) , F = 137 - 139°C (éthanol-éther isopropylique).

| Analyse centésimale : $C_{28}H_{31}F_2N_5O_2$ (M = 507,58) | | | | |
|---|---|---|---|---|
| | C % | H % | F % | N % |
| calculé | 66,26 | 6,16 | 7,49 | 13,80 |
| trouvé | 65,99 | 6,22 | 7,44 | 13,71 |

IR :
$\bar{\nu}$(C = O) = 1665 $cm^{-1}$
$\bar{\nu}$(C ≡ N) = 2150 $cm^{-1}$
R.M.N. ($CDCl_3$) : δ = 1,5 - 4,4 (22H,m) ; 5,9 (1H,pic échangeable avec $D_2O$) ; 6,9 - 7,4 (4H,m) ; 7,7 - 8,2 (4H,m).

**Exemple 31 :**

**N-Cyano-N'-méthyl-[4-(6-fluoro-1,2-benzisoxazol-3-yl)-1-pipéridinyl]formamidine**

a) N-Cyano-S-méthyl-[4-(6-fluoro-1,2-benzisoxazol-3-yl)-1-pipéridinyl]thioformamide

Une suspension de 4,0 g (0,0182 mole) de 6-fluoro-3-(4-pipéridinyl)-1,2-benzisoxazole dans 30 ml d'éthanol est ajoutée goutte à goutte à une solution de 2,4 g (0,0164 mole) de N-cyanocarbonimidodithioate de méthyle dans 40 ml d'éthanol. L'agitation est poursuivie pendant 2 heures à température ambiante. Il se produit un dégagement de méthylmercaptan. Le milieu réactionnel est ensuite porté à 60°C pendant 1 heure ; après refroidissement, il est concentré à sec sous pression réduite. Le résidu est recristallisé dans l'éthanol en présence de Norit. Rdt : 3,1 g (59 %), F = 148 - 151°C.

Analyse centésimale : $C_{15}H_{15}FN_4OS$ (M = 318,37)

| | C % | H % | F % | N % | S % |
|---|---|---|---|---|---|
| calculé | 56,59 | 4,75 | 5,97 | 17,60 | 10,07 |
| trouvé | 56,36 | 4,88 | 6,13 | 17,37 | 9,77 |

IR :
$\bar{\nu}$(C ≡ N) = 2160 $cm^{-1}$
R.M.N. ($CDCl_3$) : δ = 1,6 - 2,5 (4H,m) ; 2,8 (3H,s) ; 3.2- 3,8 (3H,m) ; 4,3 - 4,9 (2H,m) ; 6,8 - 7,9 (3H,m).

b) N-Cyano-N'-méthyl-[4-(6-fluoro-1,2-benzisoxazol-3-yl)-1-pipéridinyl]formamidine

A une suspension de 1,6 g (0,00503 mole) de N-cyano-S-méthyl-[4-(6-fluoro-1,2-benzisoxazol-3-yl)-1-pipéridinyl]thioformamide dans 30 ml d'éthanol, on ajoute 5,1 ml (0,0410 mole) d'une solution de méthylamine à 33 % dans l'éthanol absolu. L'agitation est poursuivie pendant 16 heures à température ambiante ; il se produit un dégagement de méthylmercaptan. On rajoute ensuite la même quantité de solution de méthylamine que précédemment et poursuit l'agitation pendant encore 8 heures. Après refroidissement vers -10°C, le précipité formé est isolé par filtration ; il est lavé à l'éther et séché. Rdt : 1,3 g (86 %), F = 164 - 167°C (éthanol).

| Analyse centésimale : $C_{15}H_{16}FN_5O$ (M = 301,32) | | | | |
|---|---|---|---|---|
| | C % | H % | F % | N % |
| calculé | 59,79 | 5,35 | 6,30 | 23,24 |
| trouvé | 59,61 | 5,47 | 6,39 | 23,20 |

IR :
$\bar{\nu}$(C ≡ N) = 2150 $cm^{-1}$
R.M.N. (DMSO $d_6$) : δ = 1,5 - 2,3 (4H,m) ; 2,9 (3H,d se transforme en s avec $CF_3COOD$) ; 3,0 - 4,4 (SH,m) ; 7,0 - 8,2 (4H, m dont 1H échangeable avec $CF_3COOD$).

**Exemple 32 :**

**Dimaléate de N-carbamoyl-N'-[2-[4-(4-fluorobenzoyl)-1-pipéridinyl]éthyl]-N"-méthylguanidine**

Une solution de 3,0 g (0,0080 mole) de N-cyano-N'-[2-[4-[2-(4-fluorophényl)-1,3-dioxolan-2-yl]-1-pipéridinyl]éthyl]-N"-méthylguanidine dans 69 ml d'acide chlorhydrique 1N est amenée au reflux en 15 minutes et maintenue ainsi pendant 2 minutes. Après refroidissement rapide par un bain d'eau, le milieu réactionnel est basifié par de la soude 30 % puis extrait au chlorure de méthylène. Les extraits organiques rassemblés sont lavés par une solution aqueuse saturée de chlorure de sodium, séchés sur sulfate de sodium et concentrés à sec sous pression réduite. Le résidu est solubilisé dans 100 ml d'éthanol et additionné d'une solution de 2,1 g (0,018 mole) d'acide maléique dans 50 ml d'éthanol. La solution ainsi formée est concentrée à sec sous pression réduite. Le résidu est purifié par recristallisation dans l'éthanol. Rdt : 3,0 g (64 %), F = 148 -150°C (décomposition).

| Analyse centésimale : $C_{25}H_{32}FN_5O_{10}$ (M = 581,55) | | | | |
|---|---|---|---|---|
| | C % | H % | F % | N % |
| calculé | 51,63 | 5,55 | 3,27 | 12,04 |
| trouvé | 51,84 | 5,72 | 3,27 | 12,04 |

IR :
$\bar{\nu}$(C = O) = 1665 $cm^{-1}$
R.M.N. (DMSO $d_6$ + $CF_3COOD$) : $\delta$ = 2,9 (3H,s) ; 1,4 - 4,0 (13H,m) ; 6,2 (4H,s) ; 7,0 -7,5 (2H,m) ; 7,8 - 8,3 (2H,m).

**Exemple 33 :**

**Dimaléate de N-carbamoyl-N'-[3-[4-(4-fluorobenzoyl)-1-pipéridinyl]propyl-N"-méthylguanidine**

Obtenu en opérant comme dans l'exemple 32 à partir de 2,0 g (0,0051 mole) de N-cyano-N'-[3-[4-[2-(4-fluorophényl)-1,3-dioxolan-2-yl]-1-pipéridinyl]propyl]-N"-méthylguanidine et de 44 ml d'acide chlorhydrique 1N et addition ensuite d'une solution de 1,2 g (0,010 mole) d'acide maléique dans 50 ml d'éthanol. Rdt : 1,8 g (59 %), F = 149 - 151°C (décomposition) (éthanol).

| Analyse centésimale : $C_{26}H_{34}FN_5O_{10}$ (M = 595,58) | | | | |
|---|---|---|---|---|
| | C % | H % | F % | N % |
| calculé | 52,43 | 5,75 | 3,19 | 11,76 |
| trouvé | 52,53 | 5,76 | 3,12 | 11,50 |

IR :
$\bar{\nu}$(C = O) = 1660 $cm^{-1}$
R.M.N. (DMSO $d_6$ + $CF_3COOD$) : $\delta$ = 2,8 (3H,s) ; 1,5 - 4,0 (15H,m) ; 6,2 (4H,s) ; 7,0-7,5 (2H,m) ; 7,8 - 8,2 (2H,m).

**Exemple 34 :**

**Dimaléate de N-carbamoyl-N'-[3-[4-(6-fluoro-1,2-benzisoxazol-3-yl)-1-pipéridinyl]propyl]-N"-méthylguanidine**

Obtenu en opérant comme dans l'exemple 32 à partir de 2,0 g (0,00558 mole) de N-cyano-N'-[3-[4-(4-fluorobenzoyl)-1-pipéridinyl]propyl]-N"-méthylguanidine et de 47 ml d'acide chlorhydrique 1N et addition ensuite d'une solution de 0,86 g (0,00741 mole) d'acide maléique dans 50 ml d'éthanol. Rdt : 1,5 g (44 %), F = 136°C (décomposition) (éthanol).

Analyse centésimale : $C_{26}H_{33}FN_6O_{10}$ (M = 608,58)

| | C % | H % | F % | N % |
|---|---|---|---|---|
| calculé | 51,31 | 5,47 | 3,12 | 13,81 |

| | C % | H % | F % | N % |
|---|---|---|---|---|
| trouvé | 51,66 | 5,52 | 2,86 | 13,55 |

R.M.N. (DMSO $d_6$ + $CF_3COOD$) : δ = 1,5 - 4,0 (18H,m) dont 2,9 (3H,s) ; 6,2 (4H,s) ; 7,0 - 8,1 (3H,m).

**Exemple 35 :**

**N-Cyano-N'-[3-[4-(6-fluoro-1,2-benzisoxazol-3-yl)-1-pipéridlnyl]propyl]-N"-méthylguanidine**

a) 3-[4-(6-Fluoro-1,2-benzisoxazol-3-yl)-1-pipéridinyl]propionamide

Une solution de 3,4 g (0,0156 mole) de 6-fluoro-3-(4-pipéridinyl)-1,2-benzisoxazole et de 1,3 g (0,0187 mole) d'acrylamide dans 25 ml d'éthanol est portée à reflux pendant 5 heures. Le milieu réactionnel est alors refroidi à 0°C. Le précipité formé est isolé par filtration, lavé à l'éther et séché. Rdt : 3,8 g (84 %), F = 144 - 147°C.
IR :
ν(C = O) = 1650 $cm^{-1}$
R.M.N. ($CDCl_3$) : δ = 1,8 - 3,3 (13H,m) ; 5,8 (1H,pic échangeable avec $D_2O$) ; 6,8 - 7,7 (3H,m) ; 7,8 (1H,pic échangeable avec $D_2O$).

b) 3-[1-(3-Aminopropyl)-4-pipéridinyl]-6-fluoro-1,2-benzisoxazole

A une suspension de 1,9 g (0,050 mole) d'hydrure de lithium et d'aluminium dans 100 ml de tétrahydrofurane, on ajoute par fractions 3,8 g (0,013 mole) de 3-[4-(6-fluoro-1,2-benzisoxazol-3-yl)-1-pipéridinyl]propionamide en suspension dans 50 ml de tétrahydrofurane. Le milieu réactionnel est agité sous atmosphère d'azote pendant 20 heures. Après refroidissement vers 10°C, l'hydrure métallique en excès est détruit par l'addition de 15 ml d'acétate d'éthyle puis de 15 ml de soude 10 %. Les hydroxydes formés sont filtrés et lavés au chlorure de méthylène. Le filtrat et les solutions de lavage rassemblés sont lavés par de la saumure, séchés sur sulfate de sodium et concentrés à sec sous pression réduite. Le résidu est utilisé dans l'étape suivante sans autre purification. Il est identique au produit obtenu dans le paragraphe b de l'exemple 19. Rdt : 3,6 g (quantitatif).

c) N-Cyano-N'-[3-[4-(6-fluoro-1,2-benzisoxazol-3-yl)-1-pipéridinyl]propyl]-S-méthylisothiourée

Une solution de 3,6 g (0,013 mole) de 3-[1-(3-aminopropyl)-4-pipéridinyl]-6-fluoro-1,2-benzisoxazole dans 90 ml d'éthanol est ajoutée goutte à goutte à une solution de 1,5 g (0,0103 mole) de N-cyanocarbonimidodithioate de méthyle dans 90 ml d'éthanol. Le mélange est agité pendant 24 heures à température ambiante. Il se produit un dégagement de méthylmercaptan. La solution obtenue est concentrée à sec sous pression réduite. Le résidu est purifié par chromatographie sur colonne de silice. Eluant : chlorure de méthylène 95 - méthanol 5. L'évaporation de l'éluat fournit un produit identique à celui obtenu dans le paragraphe a de l'exemple 20. Rdt : 1,3 g (27 %).

d) N-Cyano-N'-[3-[4-(6-fluoro-1,2-benzisoxazol-3-yl)-1-pipéridinyl]propyl]-N"-méthylguanidine

Une solution de 1,3 g (0,0035 mole) de N-cyano-N'-[3-[4-(6-fluoro-1,2-benzisoxazol-3-yl)-1-pipéridinyl]-propyl]-S-méthylisothiourée dans 100 ml d'une solution de méthylamine à 33 % dans l'éthanol est agitée à température ambiante pendant 20 heures. Il se produit un dégagement de méthylmercaptan. La solution obtenue est ensuite concentrée jusqu'à un volume d'environ 30 ml et refroidie vers -20°C. Le précipité formé est isolé par filtration ; il est lavé à l'éther et recristallisé dans l'éthanol. Il est identique au produit obtenu dans le paragraphe c de l'exemple 19. Rdt : 0,8 g (64 %).

| Analyse centésimale : $C_{18}H_{23}FN_6O$ (M = 358,42) | | | | |
|---|---|---|---|---|
| | C % | H % | F % | N % |
| calculé | 60,32 | 6,47 | 5,30 | 23,45 |
| trouvé | 60,13 | 6,50 | 5,01 | 23,46 |

**Exemple 36 :**

**N-Cyano-N'-[2-[4-(6-fluoro-1,2-benzisoxazol-3-yl)-1-pipéridinyl]éthyl]-N"-méthylguanidine**

a) 3-[1-(2-Aminoéthyl)-4-pipéridinyl]-6-fluoro-1,2-benzisoxazole

A une suspension de 1,3 g (0,034 mole) d'hydrure de lithium et d'aluminium dans 85 ml de tétrahydrofurane, on ajoute goutte à goutte, à 15°C. une solution de 3,0 g (0,0116 mole) de [4-(6-fluoro-1,2-benzisoxazol-3-yl)-1-pipéridinyl]acétonitrile [préparé selon J.T. Strupczewski et al., brevet U.S. 4.355.037 ; C.A. (1983) 98, 53870p] dans 50 ml de tétrahydrofurane. Le milieu réactionnel est agité sous atmosphère d'azote pendant 20 heures. Après refroidissement vers 15°C. l'hydrure métallique en excès est détruit par l'addition de 20 ml d'acétate d'éthyle puis de 20 ml d'eau. Les hydroxydes formés sont filtrés et lavés au chlorure de méthylène. Le filtrat et les solutions de lavage rassemblés sont lavés par de la saumure, séchés sur sulfate de sodium et concentrés à sec sous pression réduite. Le résidu obtenu est utilisé dans l'étape suivante sans autre purification. Il est identique au produit obtenu dans le paragraphe b de l'exemple 18. Rdt : 2,8 g (92 %).

b) N-Cyano-N'-[2-[4-(6-fluoro-1,2-benzisoxazol-3-yl)-1-pipéridinyl]éthyl]S-méthylisothiourée

Obtenue en opérant comme dans le paragraphe c de l'exemple 35 à partir de 1,7 g (0,0065 mole) de 3-[1-(2-aminoéthyl)-4-pipéridinyl]-6-fluoro-1,2-benzisoxazole dans 50 ml d'éthanol et de 0, 95 g (0,0065 mole) de N-cyanocarbonimidodithioate de méthyle dans 50 ml d'éthanol. Eluant de chromatographie : chlorure de méthylène 95 -méthanol 5. L'évaporation de l'éluat fournit un produit identique à celui obtenu dans le paragraphe c de l'exemple 18. Rdt : 1,2 g (51 %).

c) N-Cyano-N'-[2-[4-(6-fluoro-1,2-benzisoxazol-3-yl)-1-pipéridinyl]éthyl]-N"-méthylguanidine

Obtenue en opérant comme dans le paragraphe d de l'exemple 35 à partir de 1,2 g (0,0033 mole) de N-cyano-N'-[2-[4-(6-fluoro-1,2-benzisoxazol-3-yl)-1-pipéridinyl]éthyl]-S-méthylisothiourée et de 100 ml d'une solution de méthylamine à 33 % dans l'éthanol. La recristallisation dans l'éthanol fournit un produit identique à celui obtenu dans le paragraphe d de l'exemple 18.

## Revendications

**Revendications pour les Etats contractants suivants : AT, BE, CH, DE, DK, FR, GB, IT, LI, LU, NL, SE**

1. Pipéridines caractérisées par la formule

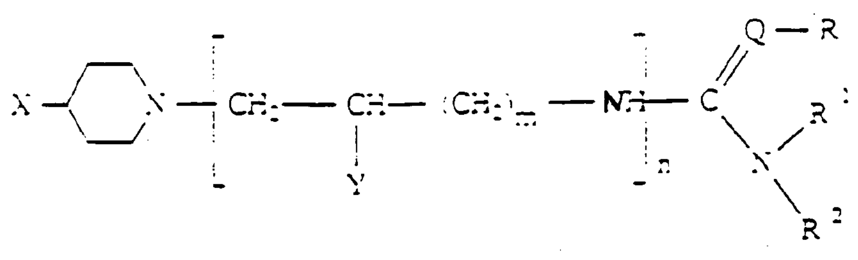

I

dans laquelle X est le groupe 4-fluorobenzoyle, 2-(4-fluorophényl)-1,3-dioxolan-2-yle ou 6-fluoro-1,2-benzisoxazol-3-yle, Y est un atome d'hydrogène ou le groupe hydroxy; m est un nombre entier compris entre 0 et 4 inclus, n est 0 ou 1, Q est un atome d'azote ou le groupe méthine ; lorsque Q est un atome d'azote, R est le groupe cyano ou le groupe carbamoyle ; lorsque Q est le groupe méthine, R est le groupe nitro ; $R^1$ et $R^2$ sont identiques ou différents et sont l'hydrogène, un radical alcoyle inférieur, le radical phényle, le groupe 2,2.2-trifluoroéthyle ou 2-[4-(4-fluorobenzoyl)-1-pipéridinyl]éthyle ; ou le motif structural $NR^1 R^2$ est le radical pipéridino ou le groupe 4-(4-fluorobenzoyl)-1-pipéridinyle ; et leurs sels d'acides minéraux ou organiques pharmaceutiquement acceptables.

2. Pipéridines selon la revendication 1, caractérisées en ce que X est le groupe 4-fluorobenzoyle ou 6-fluoro-1,2-benzisoxazol-3-yle, Y est un atome d'hydrogène, m est égal à 0,1 ou 2 et n est égal à 1 ; et leurs sels d'acides minéraux ou organiques pharmaceutiquement acceptables.

3. Pipéridine selon la revendication 1, la N-cyano-N'-[2-[4-(4-fluorobenzoyl)-1-pipéridinyl]éthyl]-N"-méthylguanidine, et ses sels d'acides minéraux ou organiques pharmaceutiquement acceptables.

4. Pipéridine selon la revendication 1, la N-cyano-N'-[2-[4-(6-fluoro-1,2-benzisoxazol-3-yl)-1-pipéridinyl]-éthyl]-N"-méthylguanidine, et ses sels d'acides minéraux ou organiques pharmaceutiquement acceptables.

5. Pipéridine selon la revendication 1, la N-cyano-N'-[3-[4-(6-fluoro-1,2-benzisoxazol-3-yl)-1-pipéridinyl]-propyl)-N"-méthylguanidine, et ses sels d'acides minéraux ou organiques pharmaceutiquement acceptables.

6. Pipéridine selon la revendication 1, la N-cyano-N'-éthyl-N-[3-[4-(6-fluoro-1,2-benzisoxazol-3-yl)-1-pipéridinyl]propyl]guanidine, et ses sels d'acides minéraux ou organiques pharmaceutiquement acceptables.

7. Pipéridine selon la revendication 1, la N'-cyano-N,N-diméthyl-N"-[3-[4-(6-fluoro-1,2-benzisoxazol-3-yl)-1-pipéridinyl]propyl]guanidine, et ses sels d'acides minéraux ou organiques pharmaceutiquement acceptables.

8. Pipéridine selon la revendication 1, la N-cyano-N'-[3-[4-(6-fluoro-1,2-benzisoxazol-3-yl)-1-pipéridinyl]-propyl]guanidine, et ses sels d'acides minéraux ou organiques pharmaceutiquement acceptables.

9. Pipéridine selon la revendication 1, la N-[2-[4-(6-fluoro-1,2-benzisoxazol-3-yl)-1-pipéridinyl]éthyl]-N'-méthyl-2-nitro-1,1-éthènediamine.

10. Procédé de préparation des composés selon la revendication 1 caractérisé en ce qu'on traite une amine de formule :

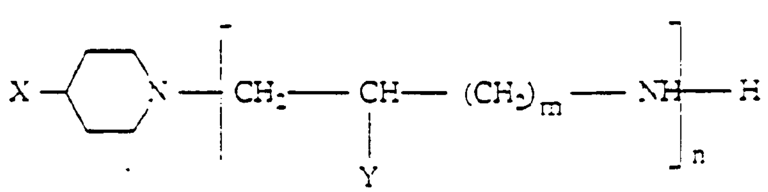

II

par un dérivé de formule :

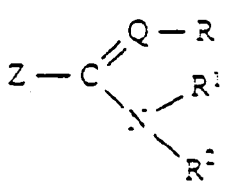

III

X, Y, Q, R, $R^1$, $R^2$, m et n ayant les significations données dans la revendication 1, Z représentant le radical méthylthio ou le radical phénoxy.

11. Procédé selon la revendication 10. caractérisé en ce qu'on effectue la réaction dans un solvant inerte, de préférence l'éthanol ou le 2-méthoxyéthanol.

12. Procédé de préparation des composés selon la revendication 1 caractérisé en ce qu'on traite un dérivé de formule :

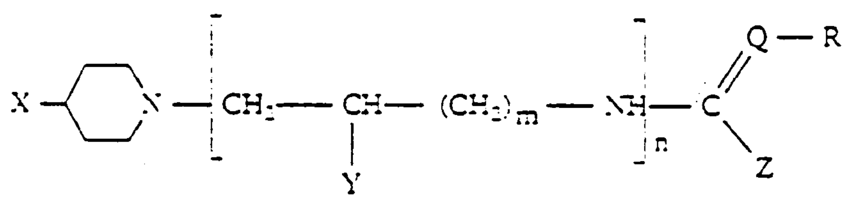 IV

par une amine de formule $HNR^1R^2$, X, Y, Q, R, $R^1$, $R^2$, m et n ayant les significations données dans la revendication 1, Z représentant le radical méthylthio ou le radical phénoxy.

**13.** Procédé de préparation selon la revendication 12 caractérisé en ce qu'on effectue la réaction dans un solvant inerte, de préférence l'éthanol ou sans solvant, dans un excès d'amine de formule $HNR^1R^2$.

**14.** Produits intermédiaires dans la préparation des composés selon la revendication 12 caractérisés par la formule :

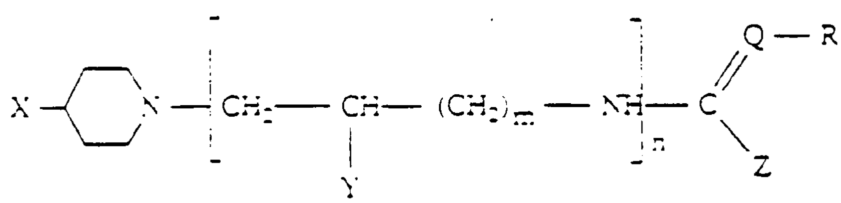 IV

dans laquelle X, Y, Q, R, m et n ont les significations données dans la revendication 1 et Z est le radical méthylthio ou le radical phénoxy.

**15.** Médicament contenant comme principe actif une pipéridine caractérisée par la formule :

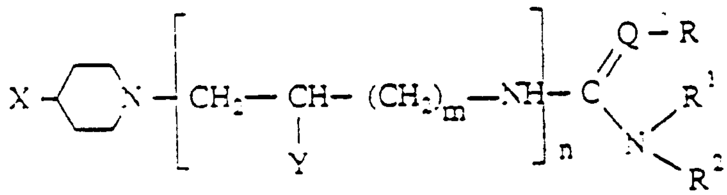 I

dans laquelle X est le groupe 4-fluorobenzoyle,2-(4-fluorophényl)-1,3-dioxolan-2-yle ou 6-fluoro-1,2-benzisoxazol-3-yle, Y est un atome d'hydrogène ou le groupe hydroxy, m est un nombre entier compris entre 0 et 4 inclus, n est 0 ou 1, Q est un atome d'azote ou le groupe méthine ; lorsque Q est un atome d'azote, R est le groupe cyano ou le groupe carbamoyle ; lorsque Q est le groupe méthine, R est le groupe nitro ; $R^1$ et $R^2$ peuvent être identiques ou différents et sont l'hydrogène, un radical alcoyle inférieur, le radical phényle, le groupe 2,2,2-trifluoroéthyle ou 2-[4-(4-fluorobenzoyl)-1-pipéridinyl]éthyle ; ou le motif structural $NR^1R^2$ est le radical pipéridino ou le groupe 4-(4-fluorobenzoyl)-1-pipéridinyle ; et leurs sels d'acides minéraux ou organiques pharmaceutiquement acceptables.

**16.** Composition pharmaceutique caractérisée en ce qu'elle comporte au moins une pipéridine de formule I définie à la revendication 1 et un excipient pharmaceutiquement acceptable.

**17.** Composition pharmaceutique selon la revendication 16, caractérisée en ce qu'elle est sous forme de comprimés, comprimés dragéifiés, gélules ou soluté injectable.

**18.** Utilisation des composés de formule I selon la revendication 1 pour la préparation de médicament utile dans le traitement et la prophylaxie de l'hypertension et des désordres organiques engendrés ou aggravés par un excès de sérotonine.

**Revendications pour l'Etat contractant suivant : ES**

1. Procédé de préparation des composés de formule I

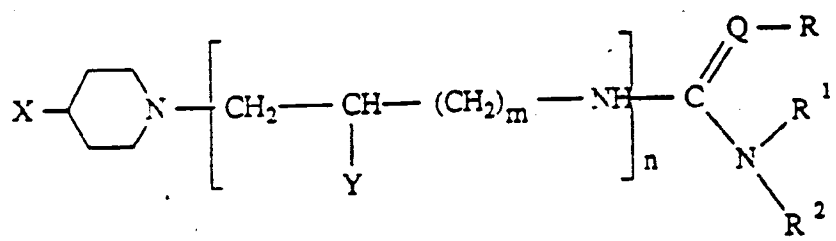

I

dans laquelle X est le groupe 4-fluorobenzoyle, 2-(4-fluorophényl)-1,3-dioxolan-2-yle ou 6-fluoro-1,2-benzisoxazol-3-yle, Y est un atome d'hydrogène ou le groupe hydroxy, m est un nombre entier compris entre 0 et 4 inclus, n est 0 ou 1, Q est un atome d'azote ou le groupe méthine ; lorsque Q est un atome d'azote, R est le groupe cyano ou le groupe carbamoyle ; lorsque Q est le groupe méthine. R est le groupe nitro ; $R^1$ et $R^2$ sont identiques ou différents et sont l'hydrogène, un radical alcoyle inférieur, le radical phényle, le groupe 2,2,2-trifluoroéthyle ou 2-[4-(4-fluorobenzoyl)-1-pipéridinyl]éthyle ; ou le motif structural $NR^1R^2$ est le radical pipéridino ou le groupe 4-(4-fluorobenzoyl)-1-pipéridinyle ; et leurs sels d'acides minéraux ou organiques pharmaceutiquement acceptables, caractérisé en ce que l'on réalise au moins l'une des étapes suivantes :

A) le traitement d'une amine de formule II

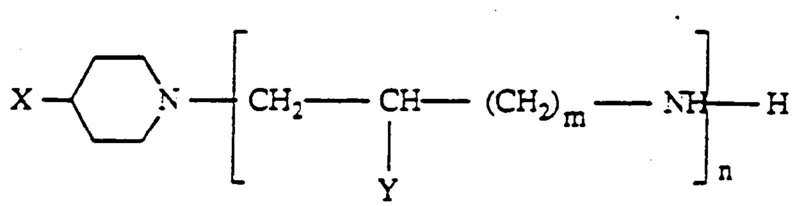

II

par un dérivé de formule :

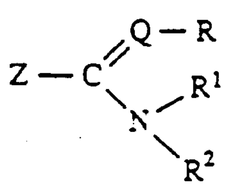

III

X, Y, Q, R, $R^1$, $R^2$, m et n ayant les significations données ci-dessus, Z représentant le radical méthylthio ou le radical phénoxy, ou

B) le traitement d'un dérivé de formule IV

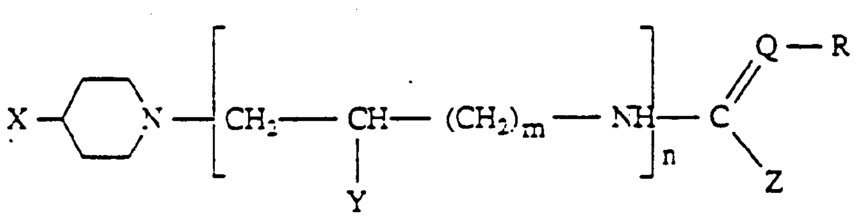

IV

par une amine de formule $HNR^1R^2$, X, Y, Q, R, $R^1$, $R^2$, m et n ayant les significations données ci-dessus , Z représentant le radical méthylthio ou le radical phénoxy, ou

C) au cas où Q représente un atome d'azote et R un groupement carbamoyle, et X représente le groupe 4-fluorobenzoyle, l'hydrolyse des composés I correspondants où X représente le groupe 2-(4-fluorophényl)-1,3-dioxolan-2-yle et R le groupe cyano, ou
D) au cas où Q représente un atome d'azote et R un groupement carbamoyle, et X représente le groupe 6-fluoro-1,2-benzisoxasol-3-yle, l'hydrolyse de composés I correspondants où R représente le groupe cyano.

2. Procédé selon la revendication 1, caractérisé en ce qu'on effectue le traitement A) dans un solvant inerte, de préférence l'éthanol ou le 2-méthoxyethanol.

3. Procédé de préparation selon la revendication 1, caractérisé en ce qu'on effectue le traitement B) dans un solvant inerte, de préférence l'éthanol ou sans solvant, dans un excès d'amine de formule $HNR^1R^2$.

4. Procédé selon la revendication 1, caractérisé en ce que les composés sont ceux où X est le groupe 4-fluorobenzoyle ou 6-fluoro-1,2-benzisoxazol-3-yle, Y est un atome d'hydrogène, m est égal à 0,1 ou 2 et n est égal à 1 ; et leurs sels d'acides minéraux ou organiques pharmaceutiquement acceptables.

5. Procédé selon la revendication 1, caractérisé en ce que le composés est la N-cyano-N'-[2-[4-(4-fluorobenzoyl)-1-pipéridinyl]éthyl]-N"-méthylguanidine, et ses sels d'acides minéraux ou organiques pharmaceutiquement acceptables.

6. Procédé selon la revendication 1, caractérisé en ce que le composé est la N-cyano-N'-[2-[4-(6-fluoro-1,2-benzisoxazol-3-yl)-1-pipéridinyl]éthyl]-N"-méthylguanidine, et ses sels d'acides minéraux ou organiques pharmaceutiquement acceptables.

7. Procédé selon la revendication 1, caractérisé en ce que le composé est la N-cyano-N'-[3-[4-(6-fluoro-1,2-benzisoxazol-3-yl)-1-pipéridinyl]propyl]-N"-méthylguanidine, et ses sels d'acides minéraux ou organiques pharmaceutiquement acceptables.

8. Procédé selon la revendication 1, caractérisé en ce que le composé est la N-cyano-N'-éthyl-N"-[3-[4-(6-fluoro-1,2-benzisoxazol-3-yl)-1-pipéridinyl]propyl]guanidine, et ses sels d'acides minéraux ou organiques pharmaceutiquement acceptables.

9. Procédé selon la revendication 1, caractérisé en ce que le composé est la N'-cyano-N,N-diméthyl-N"-[3-[4-(6-fluoro-1,2-benzisoxazol-3-yl)-1-pipéridinyl]propyl]guanidine, et ses sels d'acides minéraux ou organiques pharmaceutiquement acceptables.

10. Procédé selon la revendication 1, caractérisé en ce que le composé est la N-cyano-N'-[3-[4-(6-fluoro-1,2-benzisoxazol-3-yl)-1-pipéridinyl]propyl]guanidine, et ses sels d'acides minéraux ou organiques pharmaceutiquement acceptables.

11. Procédé selon la revendication 1, caractérisé en ce que le composé est la N-[2-[4-(6-fluoro-1,2-benzisoxazol-3-yl)-1-pipéridinyl]éthyl]-N'-méthyl-2-nitro-1,1-éthènediamine.

12. Procédé de préparation de produits intermédiaires de formule IV

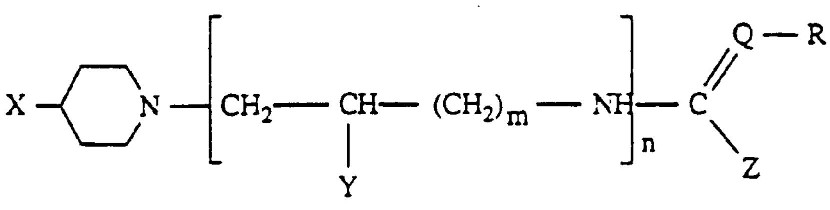
IV

dans laquelle X, Y, Q, R, m et n ont les significations données dans la revendication 1 et Z est le radical méthylthio ou le radical phénoxy, ou intermédiaires dans la préparation des composés de formule I selon la revendication 1, caractérisé en ce que l'on réalise une condensation de l'amine de formule II sur un dérivé de formule XXV, où Z, Q et R ont les significations ci-dessus :

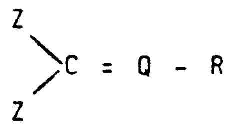

XXV

**13.** Procédé de préparation de compositions pharmaceutiques, caractérisé en ce que l'on mélange au moins un composé de formule I

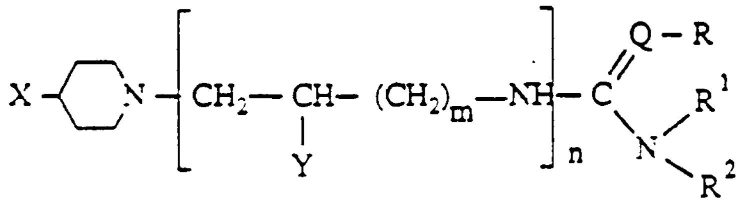

I

dans laquelle X est le groupe 4-fluorobenzoyle, 2-(4-fluorophényl)-1,3-dioxolan-2-yle ou 6-fluoro-1,2-benzisoxazol-3-yle, Y est un atome d'hydrogène ou le groupe hydroxy, m est un nombre entier compris entre 0 et 4 inclus, n est 0 ou 1, Q est un atome d'azote ou le groupe méthine ; lorsque Q est un atome d'azote, R est le groupe cyano ou le groupe carbamoyle ; lorsque Q est le groupe méthine, R est le groupe nitro ; $R^1$ et $R^2$ peuvent être identiques ou différents et sont l'hydrogène, un radical alcoyle inférieur, le radical phényle, le groupe 2,2,2-trifluoroéthyle ou 2-[4-(4-fluorobenzoyl)-1-pipéridinyl]éthyle ; ou le motif structural $NR^1R^2$ est le radical pipéridino ou le groupe 4-(4-fluorobenzoyl)-1-pipéridinyle ; ou ses sels d'acides minéraux ou organiques pharmaceutiquement acceptables, avec un excipient pharmaceutiquement acceptable.

**14.** Procédé de préparation selon la revendication 13, caractérisé en ce que l'excipient est propre à la préparation de composition sous forme de comprimés, comprimés dragéifiés, gélules ou soluté injectable .

**Revendications pour l'Etat contractant suivant : GR**

**1.** Pipéridines caractérisées par la formule

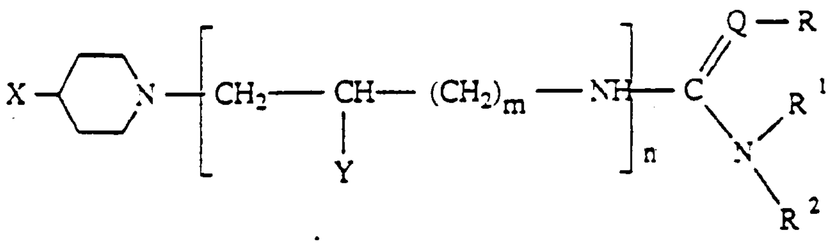

I

dans laquelle X est le groupe 4-fluorobenzoyle, 2-(4-fluorophényl)-1,3-dioxolan-2-yle ou 6-fluoro-1,2-benzisoxazol-3-yle, Y est un atome d'hydrogène ou le groupe hydroxy, m est un nombre entier compris entre 0 et 4 inclus, n est 0 ou 1, Q est un atome d'azote ou le groupe méthine ; lorsque Q est un atome d'azote, R est le groupe cyano ou le groupe carbamoyle ; lorsque Q est le groupe méthine, R est le groupe nitro ; $R^1$ et $R^2$ sont identiques ou différents et sont l'hydrogène, un radical alcoyle inférieur, le radical phényle, le groupe 2,2,2-trifluoroéthyle ou 2-[4-(4-fluorobenzoyl)-1-pipéridinyl]éthyle ; ou le motif structural $NR^1R^2$ est le radical pipéridino ou le groupe 4-(4-fluorobenzoyl)-1-pipéridinyle ; et leurs sels d'acides minéraux ou organiques pharmaceutiquement acceptables.

**2.** Pipéridines selon la revendication 1, caractérisées en ce que X est le groupe 4-fluorobenzoyle ou 6-fluoro-1,2-benzisoxazol-3-yle, Y est un atome d'hydrogène, m est égal à 0,1 ou 2 et n est égal à 1 ; et leurs sels d'acides minéraux ou organiques pharmaceutiquement acceptables.

**3.** Pipéridine selon la revendication 1, la N-cyano-N'-[2-[4-(4-fluorobenzoyl)-1-pipéridinyl]éthyl]-N"-méthylguanidine, et ses sels d'acides minéraux ou organiques pharmaceutiquement acceptables.

4. Pipéridine selon la revendication 1, la N-cyano-N'-[2-[4-(6-fluoro-1,2-benzisoxazol-3-yl)-1-pipéridinyl]-éthyl-N"-méthylguanidine, et ses sels d'acides minéraux ou organiques pharmaceutiquement acceptables.

5. Pipéridine selon la revendication 1, la N-cyano-N'-[3-[4-(6-fluoro-1,2-benzisoxazol-3-yl)-1-pipéridinyl]-propyl]-N"-méthylguanidine, et ses sels d'acides minéraux ou organiques pharmaceutiquement acceptables.

6. Pipéridine selon la revendication 1, la N-cyano-N'-éthyl-N"-[3-[4-(6-fluoro-1,2-benzisoxazol-3-yl)-1-pipéridinyl]propyl]guanidine, et ses sels d'acides minéraux ou organiques pharmaceutiquement acceptables.

7. Pipéridine selon la revendication 1, la N'-cyano-N,N-diméthyl-N"-[3-[4-(6-fluoro-1,2-benzisoxazol-3-yl)-1-pipéridinyl]propyl]guanidine, et ses sels d'acides minéraux ou organiques pharmaceutiquement acceptables.

8. Pipéridine selon la revendication 1, la N-cyano-N'-[3-[4-(6-fluoro-1,2-benzi soxazol-3-yl)-1-pipéridinyl]-propyl]guanidine, et ses sels d'acides minéraux ou organiques pharmaceutiquement acceptables.

9. Pipéridine selon la revendication 1, la N-[2-[4-(6-fluoro-1,2-benzisoxazol-3-yl)-1-pipéridinyl]éthyl]-N'-méthyl-2-nitro-1,1-éthènediamine.

10. Procédé de préparation des composés selon la revendication 1 caractérisé en ce qu'on traite une amine de formule :

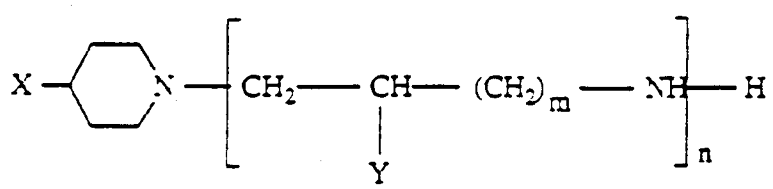

II

par un dérivé de formule :

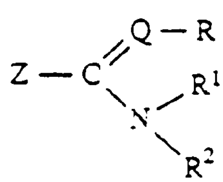

III

X, Y, Q, R, $R^1$, $R^2$, m et n ayant les significations données dans la revendication 1, Z représentant le radical méthylthio ou le radical phénoxy.

11. Procédé selon la revendication 10, caractérisé en ce qu'on effectue la réaction dans un solvant inerte, de préférence l'éthanol ou le 2-méthoxyéthanol.

12. Procédé de préparation des composés selon la revendication 1 caractérisé en ce qu'on traite un dérivé de formule :

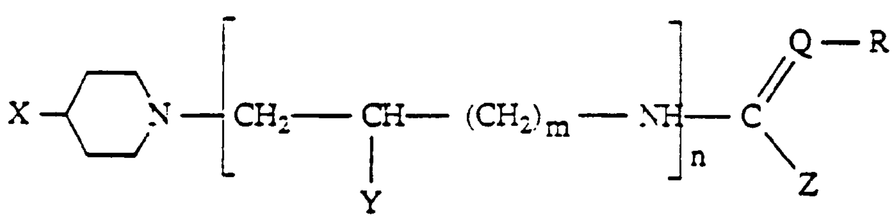

IV

par une amine de formule $HNR^1R^2$, X, Y, Q, R, $R^1$, $R^2$, m et n ayant les significations données dans la

revendication 1, Z représentant le radical méthylthio ou le radical phénoxy.

**13.** Procédé de préparation selon la revendication 12 caractérisé en ce qu'on effectue la réaction dans un solvant inerte, de préférence l'éthanol ou sans solvant, dans un excès d'amine de formule $HNR^1R^2$.

**14.** Produits intermédiaires dans la préparation des composés selon la revendication 12 caractérisés par la formule :

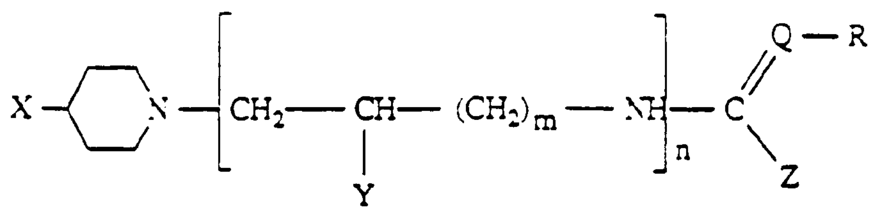

IV

dans laquelle X, Y, Q, R, m et n ont les significations données dans la revendication 1 et Z est le radical méthylthio ou le radical phénoxy.

**15.** Procédé de préparation de compositions pharmaceutiques, caractérisé en ce que l'on mélange au moins un composé de formule I

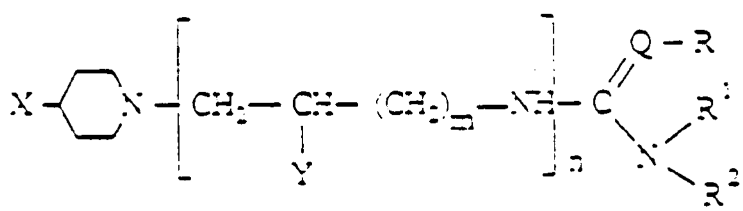

I

dans laquelle X est le groupe 4-fluorobenzoyle, 2-(4-fluorophényl)-1,3-dioxolan-2-yle ou 6-fluoro-1,2-benzisoxazol-3-yle, Y est un atome d'hydrogène ou le groupe hydroxy, m est un nombre entier compris entre 0 et 4 inclus, n est 0 ou 1, Q est un atome d'azote ou le groupe méthine ; lorsque Q est un atome d'azote, R est le groupe cyano ou le groupe carbamoyle ; lorsque Q est le groupe méthine, R est le groupe nitro ; $R^1$ et $R^2$ peuvent être identiques ou différents et sont l'hydrogène, un radical alcoyle inférieur, le radical phényle, le groupe 2,2,2-trifluoroéthyle ou 2-[4-(4-fluorobenzoyl)-1-pipéridinyl]éthyle ; ou le motif structural $NR^1R^2$ est le radical pipéridino ou le groupe 4-(4-fluorobenzoyl)-1-pipéridinyle ; et ses sels d'acides minéraux ou organiques pharmaceutiquement acceptables, avec un excipient pharmaceutiquement acceptable.

**16.** Procédé de préparation selon la revendication 15, caractérisé en ce que l'excipient est propre à la préparation de composition sous forme de comprimés, comprimés dragéifiés, gélules ou soluté injectable.

## Claims

### Claims for the following Contracting States : AT, BE, CH, DE, DK, FR, GB, IT, LI, LU, NL, SE

**1.** Piperidines characterized by the formula

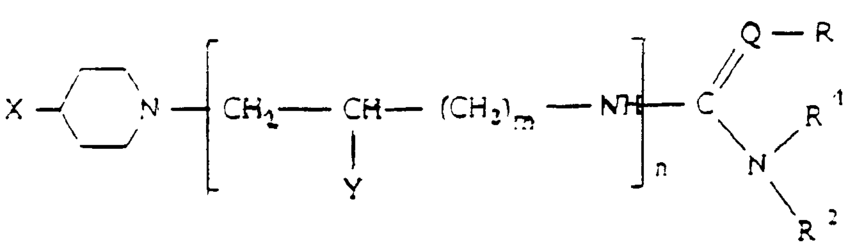

I

in which X is the 4-fluorobenzoyl, 2-(4-fluorophenyl)-1,3-dioxolan-2-yl or 6-fluoro-1,2-benzisoxazol-3-yl group, Y is a hydrogen atom or the hydroxyl group, m is an integer between 0 and 4 inclusive, n is 0 or 1, Q is a nitrogen atom or the methine group; when Q is a nitrogen atom, R is the cyano group or the carbamoyl group; when Q is the methine group, R is the nitro group; $R^1$ and $R^2$ may be identical or different and are hydrogen, a lower alkyl radical, the phenyl radical or the 2,2,2-trifluoroethyl or 2-[4-(4-fluorobenzoyl)-1-piperidinyl]ethyl group; or the $NR^1R^2$ structural unit is the piperidino radical or the 4-(4-fluorobenzoyl)-1-piperidinyl group; and their pharmaceutically acceptable inorganic or organic acid salts.

2. Piperidines according to Claim 1, characterized in that X is the 4-fluorobenzoyl or 6-fluoro-1,2-benzisoxazol-3-yl group, Y is a hydrogen atom, m is equal to 0, 1 or 2 and n is equal to 1; and their pharmaceutically acceptable inorganic or organic acid salts.

3. Piperidine according to Claim 1, N-cyano-N'-[2-[4-(4-fluorobenzoyl)-1-piperidinyl]ethyl]-N"-methylguanidine, and its pharmaceutically acceptable inorganic or organic acid salts.

4. Piperidine according to Claim 1, N-cyano-N'-[2-[4-(6-fluoro-1,2-benzisoxazol-3-yl)-1-piperidinyl]ethyl]-N"-methylguanidine, and its pharmaceutically acceptable inorganic or organic acid salts.

5. Piperidine according to Claim 1, N-cyano-N'-[3-[4-(6-fluoro-1,2-benzisoxazol-3-yl)-1-piperidinyl]-propyl]-N"-methylguanidine, and its pharmaceutically acceptable inorganic or organic acid salts.

6. Piperidine according to Claim 1, N-cyano-N'-ethyl-N"-[3-[4-(6-fluoro-1,2-benzisoxazol-3-yl)-1-piperidinyl]propyl]guanidine, and its pharmaceutically acceptable inorganic or organic acid salts.

7. Piperidine according to Claim 1, N'-cyano-N,N-dimethyl-N"-[3-[4-(6-fluoro-1,2-benzisoxazol-3-yl)-1-piperidinyl]propyl]guanidine, and its pharmaceutically acceptable inorganic or organic acid salts.

8. Piperidine according to Claim 1, N-cyano-N'-[3-[4-(6-fluoro-1,2-benzisoxazol-3-yl)-1-piperidinyl]-propyl]guanidine, and its pharmaceutically acceptable inorganic or organic acid salts.

9. Piperidine according to Claim 1, N-[2-[4-(6-fluoro-1,2-benzisoxazol-3-yl)-1-piperidinyl]ethyl]-N'- methyl-2-nitro-1,1-ethenediamine.

10. Process for the preparation of the compounds according to Claim 1, characterized in that an amine of formula

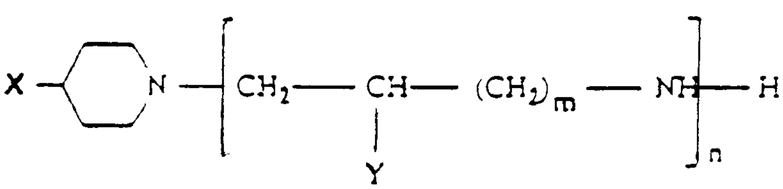

II

is treated with a derivative of formula:

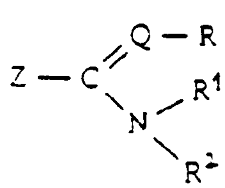

III

X, Y, Q, R, $R^1$, $R^2$, m and n having the meanings given in claim 1, Z denoting the methylthio radical or the phenoxy radical.

11. Process according to Claim 10, characterized in that the reaction is carried out in an inert solvent, preferably ethanol or 2-methoxyethanol.

12. Process for the preparation of the compounds according to Claim 1, characterized in that a derivative of

formula:

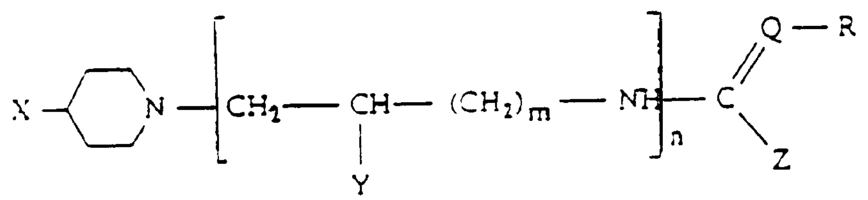

IV

is treated with an amine of formula $HNR^1R^2$, X, Y, Q, R, $R^1$, $R^2$, m and n having the meanings given in Claim 1, Z denoting the methylthio radical or the phenoxy radical.

**13.** Process for preparation according to Claim 12, characterized in that the reaction is carried out in an inert solvent, preferably ethanol or without solvent, in an excess of amine of formula $HNR^1R^2$.

**14.** Intermediate products in the preparation of the compounds according to Claim 12, characterized by the formula

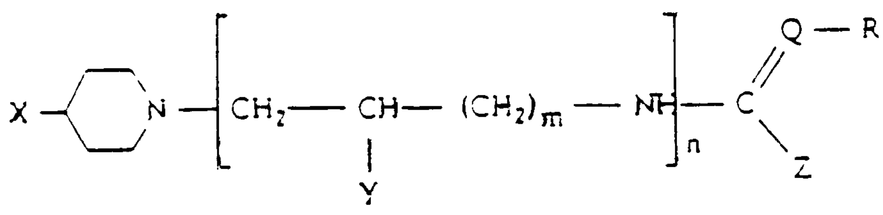

IV

in which X, Y, Q, R, m and n have the meanings given in Claim 1 and Z is the methylthio radical or the phenoxy radical.

**15.** Medication containing as active principle a piperidine characterized by the formula:

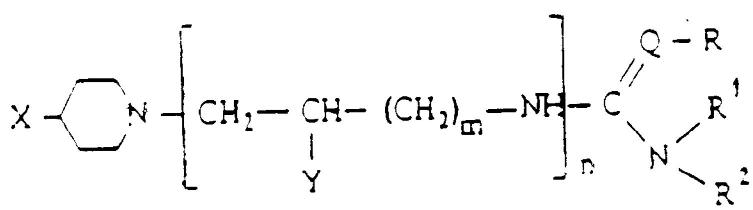

I

in which X is the 4-fluorobenzoyl, 2-(4-fluorophenyl)-1,3-dioxolan-2-yl or 6-fluoro-1,2-benzisoxazol-3-yl group, Y is a hydrogen atom or the hydroxyl group, m is an integer between 0 and 4 inclusive, n is 0 or 1, Q is a nitrogen atom or the methine group; when Q is a nitrogen atom R is the cyano group or the carbamoyl group; when Q is the methine group R is the nitro group; $R^1$ and $R^2$ may be identical or different and are hydrogen, a lower alkyl radical, the phenyl radical or the 2,2,2-trifluoroethyl or 2-[4-(4-fluorobenzoyl)-1-piperidinyl]ethyl group; or the $NR^1R^2$ structural unit is the piperidino radical or the 4-(4-fluorobenzoyl)-1-piperidinyl group; and their pharmaceutically acceptable inorganic or organic acid salts.

**16.** Pharmaceutical composition characterized in that it comprises at least one piperidine of formula I defined in Claim 1 and a pharmaceutically acceptable excipient.

**17.** Pharmaceutical composition according to Claim 16, characterized in that it is in the form of tablets, coated tablets, gelatin capsules or injectable solution.

**18.** Use of the compounds of formula I according to Claim 1 for the preparation of a medication useful in the treatment and prophylaxis of hypertension and of organic disorders generated or aggravated by an excess of serotonin.

**Claims for the following Contracting State : ES**

1. Process for preparation of the compounds of formula I

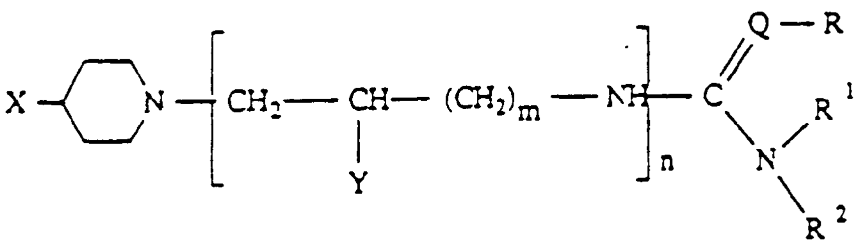

I

in which X is the 4-fluorobenzoyl, 2-(4-fluorophenyl)-1,3-dioxolan-2-yl or 6-fluoro-1,2-benzisoxazol-3-yl group, Y is a hydrogen atom or the hydroxyl group, m is an integer between 0 and 4 inclusive, n is 0 or 1, Q is a nitrogen atom or the methine group; when Q is a nitrogen atom, R is the cyano group or the carbamoyl group; when Q is the methine group, R is the nitro group; $R^1$ and $R^2$ may be identical or different and are hydrogen, a lower alkyl radical, the phenyl radical or the 2,2,2-trifluoroethyl or 2-[4-(4-fluorobenzoyl)-1-piperidin-yl]ethyl group; or the $NR^1R^2$ structural unit is the piperidino radical or the 4-(4-fluorobenzoyl)-1-piperidinyl group; and their pharmaceutically acceptable inorganic or organic acid salts, characterized in that at least one of the following steps is carried out:

A) an amine of formula II

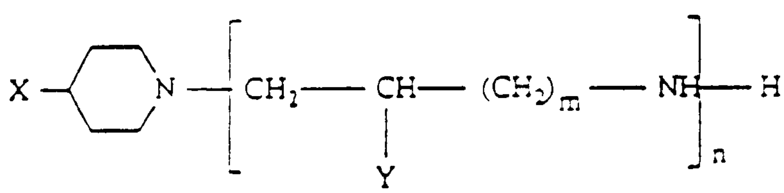

II

is treated with a derivative of formula:

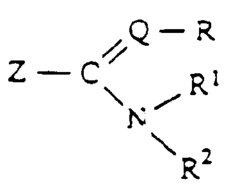

III

X, Y, Q, R, $R^1$, $R^2$, m and n having the meanings given above, Z denoting the methylthio radical or the phenoxy radical, or

B) a derivative of formula IV

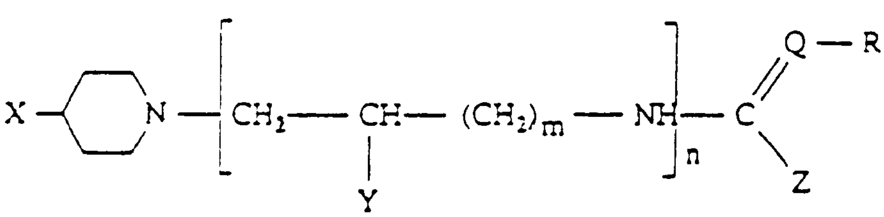

IV

is treated with an amine of formula $HNR^1R^2$, X, Y, Q, R, $R^1$, $R^2$, m and n having the meanings given above, Z denoting the methylthio radical or the phenoxy radical, or

C) in the case where Q denotes a nitrogen atom and R a carbamoyl group, and X denotes the 4-fluorobenzoyl group, the corresponding compounds 1 where X denotes the 2-(4-fluorophenyl)-1,3-dioxolan-2-yl group and R the cyano group are hydrolysed, or

D) in the case where Q denotes a nitrogen atom and R a carbamoyl group, and X denotes the 6-fluoro-1,2-benzisoxazol-3-yl group, corresponding compounds I where R denotes the cyano group are hydrolysed.

2. Process according to Claim 1, characterized in that the treatment A) is carried out in an inert solvent, preferably ethanol or 2-methoxyethanol.

3. Process for preparation according to Claim 1, characterized in that the treatment B) is carried out in an inert solvent, preferably ethanol or without solvent, in an excess of amine of formula $HNR^1R^2$.

4. Process according to Claim 1, characterized in that the compounds are those where X is the 4-fluorobenzoyl or 6-fluoro-1,2-benzisoxazol-3-yl group, Y is a hydrogen atom, m is equal to 0, 1 or 2 and n is equal to 1; and their pharmaceutically acceptable inorganic or organic acid salts.

5. Process according to Claim 1, characterized in that the compound is N-cyano-N'-[2-[4-(4-fluorobenzoyl)-1-piperidinyl]ethyl]-N"-methylguanidine, and its pharmaceutically acceptable inorganic or organic acid salts.

6. Process according to Claim 1, characterized in that the compound is N-cyano-N'- [2-[4-(6-fluoro-1,2-benzisoxazol-3-yl)-1-piperidinyl]ethyl]-N"-methylguanidine, and its pharmaceutically acceptable inorganic or organic acid salts.

7. Process according to Claim 1, characterized in that the compound is N-cyano-N'-[3-[4-(6-fluoro-1,2-benzisoxazol-3-yl)-1-piperidinyl]propyl]-N"-methylguanidine, and its pharmaceutically acceptable inorganic or organic acid salts.

8. Process according to Claim 1, characterized in that the compound is N-cyano-N'-ethyl-N"-[3-[4-(6-fluoro-1,2-benzisoxazol-3-yl)-1-piperidinyl]propyl]guanidine, and its pharmaceutically acceptable inorganic or organic acid salts.

9. Process according to Claim 1, characterized in that the compound is N'-cyano-N,N-dimethyl-N"-[3-[4-(6-fluoro-1,2-benzisoxazol-3-yl)-1-piperidinyl]-propyl]guanidine, and its pharmaceutically acceptable inorganic or organic acid salts.

10. Process according to Claim 1, characterized in that the compound is N-cyano-N'-[3-[4-(6-fluoro-1,2-benzisoxazol-3-yl)-1-piperidinyl]propyl]guanidine, and its pharmaceutically acceptable inorganic or organic acid salts.

11. Process according to Claim 1, characterized in that the compound is N-[2-[4-(6-fluoro-1,2-benzisoxazol-3-yl)-1-piperidinyl]ethyl]-N'-methyl-2-nitro-1,1-ethenediamine.

12. Process for preparation of intermediate products of formula IV

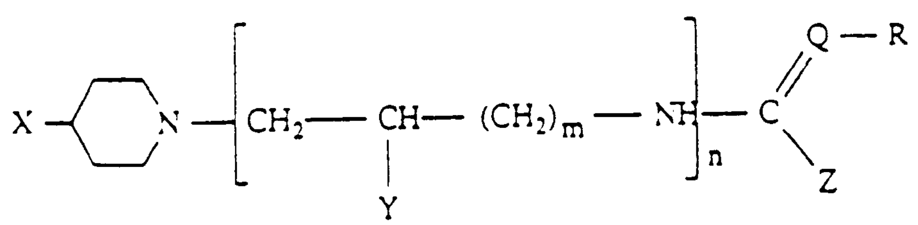

IV

in which X, Y, Q, R, m and n have the meanings given in Claim 1 and Z is the methylthio radical or the phenoxy radical, or intermediates in the preparation of the compounds of formula 1 according to Claim 1, characterized in that the amine of formula II is condensed with a derivative of formula XXV, where Z, Q and R have the above meanings:

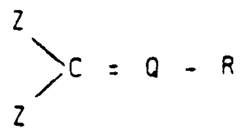

XXV

**13.** Process for preparation of pharmaceutical compositions, characterized in that at least one compound of formula I

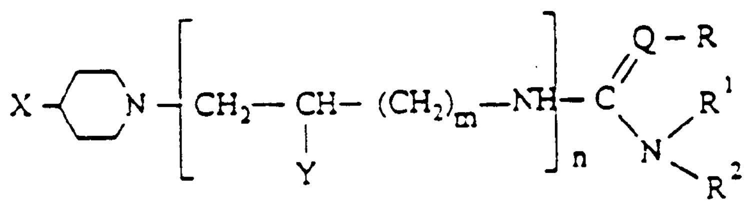

I

in which X is the 4-fluorobenzoyl, 2-(4-fluorophenyl)-1,3-dioxolan-2-yl or 6-fluoro-1,2-benzisoxazol-3-yl group, Y is a hydrogen atom or the hydroxyl group, m is an integer between 0 and 4 inclusive, n is 0 or 1, Q is a nitrogen atom or the methine group; when Q is a nitrogen atom R is the cyano group or the carbamoyl group; when Q is the methine group R is the nitro group; $R^1$ and $R^2$ may be identical or different and are hydrogen, a lower alkyl radical, the phenyl radical or the 2,2,2-trifluoroethyl or 2-[4-(4-fluorobenzoyl)-1-piperidinyl]ethyl group; or the $NR^1R^2$ structural unit is the piperidino radical or the 4-(4-fluorobenzoyl)-1-piperidinyl group; or its pharmaceutically acceptable inorganic or organic acid salts, is mixed with a pharmaceutically acceptable excipient.

**14.** Process for preparation according to Claim 13, characterized in that the excipient is suitable for the preparation of composition in the form of tablets, coated tablets, gelatin capsules or injectable solution.

**Claims for the following Contracting State : GR**

**1.** Piperidines characterized by the formula

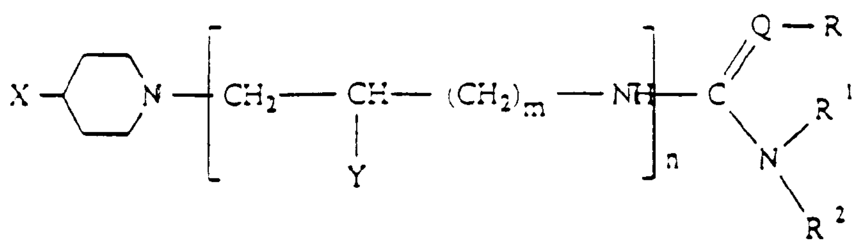

I

in which X is the 4-fluorobenzoyl, 2-(4-fluorophenyl)-1,3-dioxolan-2-yl or 6-fluoro-1,2-benzisoxazol-3-yl group, Y is a hydrogen atom or the hydroxyl group, m is an integer between 0 and 4 inclusive, n is 0 or 1, Q is a nitrogen atom or the methine group; when Q is a nitrogen atom, R is the cyano group or the carbamoyl group; when Q is the methine group, R is the nitro group; $R^1$ and $R^2$ may be identical or different and are hydrogen, a lower alkyl radical, the phenyl radical or the 2,2,2-trifluoroethyl or 2-[4-(4-fluorobenzoyl)-1-piperidinyl]ethyl group; or the $NR^1R^2$ structural unit is the piperidino radical or the 4-(4-fluorobenzoyl)-1-piperidinyl group; and their pharmaceutically acceptable inorganic or organic acid salts.

**2.** Piperidines according to Claim 1, characterized in that X is the 4-fluorobenzoyl or 6-fluoro-1,2-benzisoxazol-3-yl group, Y is a hydrogen atom, m is equal to 0, 1 or 2 and n is equal to 1; and their pharmaceutically acceptable inorganic or organic acid salts.

**3.** Piperidine according to Claim 1, N-cyano-N'-[2-[4-(4-fluorobenzoyl)-1-piperidinyl]ethyl]-N"-methylguanidine, and its pharmaceutically acceptable inorganic or organic acid salts.

**4.** Piperidine according to Claim 1, N-cyano-N'-[2-[4-(6-fluoro-1,2-benzisoxazol-3-yl)-1-piperidinyl]ethyl]-N"-methylguanidine, and its pharmaceutically acceptable inorganic or organic acid salts.

5. Piperidine according to Claim 1, N-cyano-N'-[3-[4-(6-fluoro-1,2-benzisoxazol-3-yl)-1-piperidinyl]-propyl]-N"-methylguanidine, and its pharmaceutically acceptable inorganic or organic acid salts.

6. Piperidine according to Claim 1, N-cyano-N'-ethyl-N"-[3-[4-(6-fluoro-1,2-benzisoxazol-3-yl)-1-piperidinyl]propyl]guanidine, and its pharmaceutically acceptable inorganic or organic acid salts.

7. Piperidine according to Claim 1, N'-cyano-N,N-dimethyl-N"-[3-[4-(6-fluoro-1,2-benzisoxazol-3-yl)-1-piperidinyl]propyl]guanidine, and its pharmaceutically acceptable inorganic or organic acid salts.

8. Piperidine according to Claim 1, N-cyano-N'-[3-[4-(6-fluoro-1,2-benzisoxazol-3-yl)-1-piperidinyl]-propyl]guanidine, and its pharmaceutically acceptable inorganic or organic acid salts.

9. Piperidine according to Claim 1, N-[2-[4-(6-fluoro-1,2-benzisoxazol-3-yl)-1-piperidinyl]ethyl]-N'-methyl-2-nitro-1,1-ethenediamine.

10. Process for the preparation of the compounds according to Claim 1, characterized in that an amine of formula

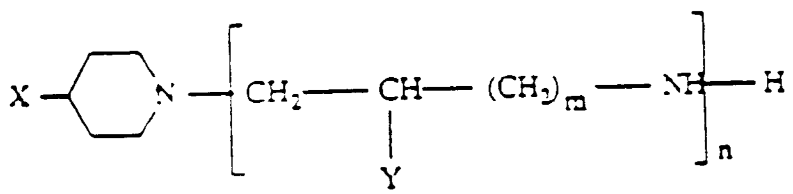

II

is treated with a derivative of formula:

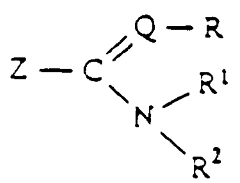

III

X, Y, Q, R, $R^1$, $R^2$, m and n having the meanings given in Claim 1, Z denoting the methylthio radical or the phenoxy radical.

11. Process according to Claim 10, characterized in that the reaction is carried out in an inert solvent, preferably ethanol or 2-methoxyethanol.

12. Process for the preparation of the compounds according to Claim 1, characterized in that a derivative of formula:

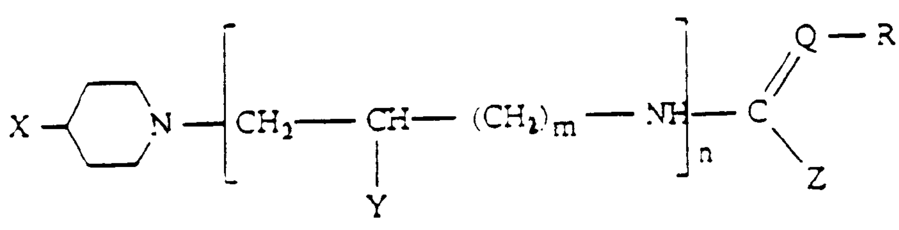

IV

is treated with an amine of formula $HNR^1R^2$, X, Y, Q, R, $R^1$, $R^2$, m and n having the meanings given in Claim 1, Z denoting the methylthio radical or the phenoxy radical.

13. Process for preparation according to Claim 12, characterized in that the reaction is carried out in an inert

solvent, preferably ethanol or without solvent, in an excess of amine of formula $HNR^1R^2$.

14. Intermediate products in the preparation of the compounds according to Claim 12, characterized by the formula

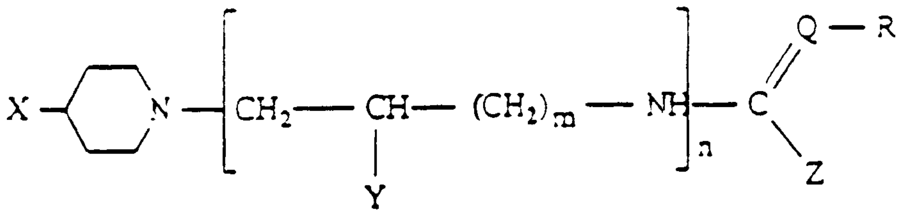

IV

in which X, Y, Q, R, m and n have the meanings given in Claim 1 and Z is the methylthio radical or the phenoxy radical.

15. Process for preparation of pharmaceutical compositions, characterized in that at least one compound of formula I

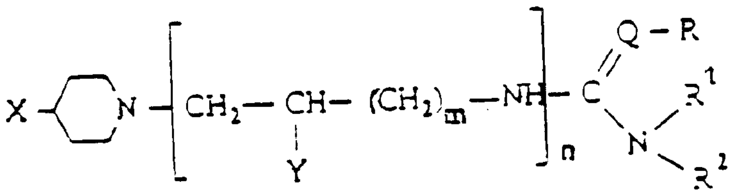

I

in which X is the 4-fluorobenzoyl, 2-(4-fluorophenyl)-1,3-dioxolan-2-yl or 6-fluoro-1,2-benzisoxazol-3-yl group, Y is a hydrogen atom or the hydroxyl group, m is an integer between 0 and 4 inclusive, n is 0 or 1, Q is a nitrogen atom or the methine group; when Q is a nitrogen atom R is the cyano group or the carbamoyl group; when Q is the methine group R is the nitro group; $R^1$ and $R^2$ may be identical or different and are hydrogen, a lower alkyl radical, the phenyl radical or the 2,2,2-trifluoroethyl or 2-[4-(4-fluorobenzoyl)-1-piperidinyl]ethyl group; or the $NR^1R^2$ structural unit is the piperidino radical or the 4-(4-fluorobenzoyl)-1-piperidinyl group; and its pharmaceutically acceptable inorganic or organic acid salts, is mixed with a pharmaceutically acceptable excipient.

16. Process for preparation according to Claim 15, characterized in that the excipient is suitable for the preparation of composition in the form of tablets, coated tablets, gelatin capsules or injectable solution.

## Patentansprüche

### Patentansprüche für folgende Vertragsstaaten : AT, BE, CH, DE, DK, FR, GB, IT, LI, LU, NL, SE

1. Piperidine, **gekennzeichnet durch** die Formel

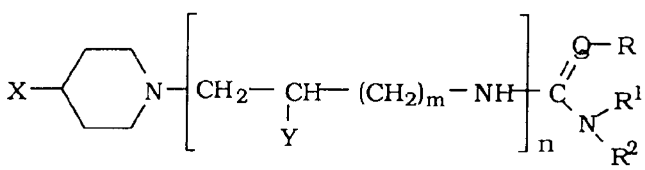

I

in der X eine 4-Fluorbenzoylgruppe, eine 2-(4-Fluorphenyl)-1,3-dioxolan-2-yl-gruppe oder eine 6-Fluor-1,2-benzisoxazol-3-yl-gruppe, Y ein Wasserstoffatom oder eine Hydroxygruppe, m eine ganze Zahl mit einem Wert zwischen 0 und 4 einschließlich, n 0 oder 1, Q ein Stickstoffatom oder eine Methingruppe; wenn Q ein Stickstoffatom darstellt, R eine Cyanogruppe oder eine Carbamoylgruppe; wenn Q eine Methingruppe darstellt, R eine Nitrogruppe; $R^1$ und $R^2$, die gleichartig oder verschieden sind, Wasserstoffatome, Niedrigalkylgruppen, Phenylgruppen, 2,2,2-Trifluorethylgruppen oder 2-[4-(4-Fluorbenzoyl)-1-piperidinyl]-ethylgruppen bedeuten; oder der Rest $NR^1R^2$ einen Piperidinorest oder einen 4-(4-Fluorbenzoyl)-1-piperidinylrest bedeutet; sowie deren Salze mit anorganischen oder organischen, pharmazeutisch annehmbaren Säuren.

2. Piperidine nach Anspruch 2, **dadurch gekennzeichnet,** daß X eine 4-Fluorbenzoylgruppe oder 6-Fluor-1,2-benzisoxazol-3-ylgruppe, Y ein Wasserstoffatom, m 0, 1 oder 2 und n 1 bedeuten, sowie deren Salze mit anorganischen oder organischen, pharmazeutisch annehmbaren Säuren.

3. Piperidin nach Anspruch 1, nämlich N-Cyano-N'-[2-[4-(4-fluorbenzoyl)-1-piperidinyl]-ethyl]-N"-methylguanidin und dessen Salze mit anorganischen oder organischen, pharmazeutisch annehmbaren Säuren.

4. Piperidin nach Anspruch 1, nämlich N-Cyano-N'-[2-[4-(6-fluor-1,2-benzisoxazol-3-yl)-1-piperidinyl]-ethyl]-N"-methylguanidin und dessen Salze mit anorganischen oder organischen, pharmazeutisch annehmbaren Säuren.

5. Piperidin nach Anspruch 1, nämlich N-Cyano-N'-[3-[4-(6-fluor-1,2-benzisoxazol-3-yl)-1-piperidinyl]-propyl]-N"-methylguanidin und dessen Salze mit anorganischen oder organischen, pharmazeutisch annehmbaren Säuren.

6. Piperidin nach Anspruch 1, nämlich N-Cyano-N'-ethyl-N"-[3-[4-(6-fluor-1,2 -benzisoxazol-3-yl)-1-piperidinyl]-propyl]-guanidin und dessen Salze mit anorganischen oder organischen, pharmazeutisch annehmbaren Säuren.

7. Piperidin nach Anspruch 1, nämlich N'-Cyano-N,N-dimethyl-N"-[3-[4-(6-fluor-1,2-benzisoxazol-3-yl)-1-piperidinyl]-propyl]-guanidin und dessen Salze mit anorganischen oder organischen, pharmazeutisch annehmbaren Säuren.

8. Piperidin nach Anspruch 1, nämlich N-Cyano-N'-[3-[4-(6-fluor-1,2-benzisoxazol-3-yl)-1-piperidinyl]-propyl]-guanidin und dessen Salze mit anorganischen oder organischen, pharmazeutisch annehmbaren Säuren.

9. Piperidin nach Anspruch 1, nämlich N-[2-[4-(6-Fluor-1,2-benzisoxazol-3-yl)-1-piperidinyl]-ethyl]-N'-methyl-2-nitro-1,1-ethendiamin.

10. Verfahren zur Herstellung der Verbindungen nach Anspruch 1, **dadurch gekennzeichnet,** daß man ein Amin der Formel

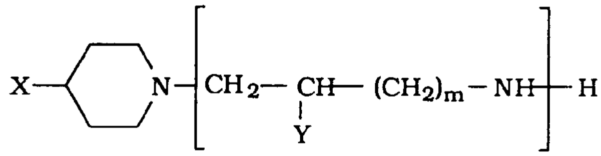

II

mit einem Derivat der Formel

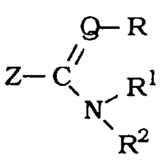

III

worin X. Y, Q, R, $R^1$, $R^2$, m und n die in Anspruch 1 angegebenen Bedeutungen besitzen und Z eine Methylthiogruppe oder eine Phenoxygruppe bedeutet, behandelt.

11. Verfahren nach Anspruch 10, **dadurch gekennzeichnet,** daß man die Umsetzung in einem inerten Lösungsmittel, vorzugsweise Ethanol oder 2-Methoxyethanol, durchführt.

**12.** Verfahren zur Herstellung der Verbindungen nach Anspruch 1, **dadurch gekennzeichnet,** daß man ein Derivat der Formel

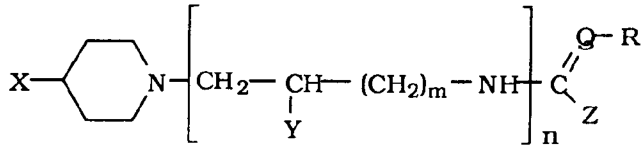

IV

mit einem Amin der Formel $HNR^1R^2$, worin X, Y, Q, R, $R^1$, $R^2$, m und n die in Anspruch 1 angegebenen Bedeutungen besitzen und Z eine Methylthiogruppe oder eine Phenoxygruppe darstellt, behandelt.

**13.** Verfahren nach Anspruch 12, **dadurch gekennzeichnet,** daß man die Umsetzung in einem inerten Lösungsmittel, vorzugsweise Ethanol, oder ohne Lösungsmittel in einem Überschuß des Amins der Formel $HNR^1R^2$ durchführt.

**14.** Zwischenprodukte für die Herstellung der Verbindungen gemäß Anspruch 12, **gekennzeichnet durch** die Formel

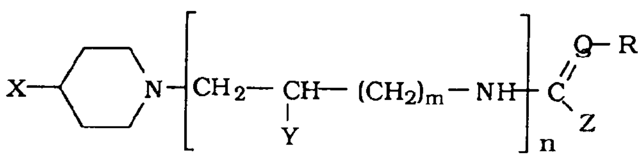

IV

in der X, Y, Q, R, m und n die in Anspruch 1 angegebenen Bedeutungen besitzen und Z eine Methylthiogruppe oder eine Phenoxygruppe bedeuten.

**15.** Arzneimittel enthaltend als Wirkstoff ein Piperidin der Formel

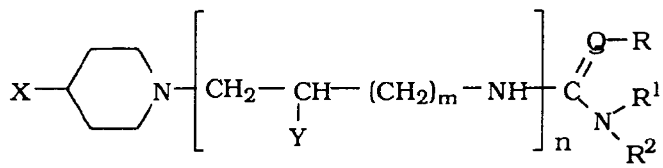

I

in der X eine 4-Fluorbenzoylgruppe, eine 2-(4-Fluorphenyl)-1,3-dioxolan-2-yl-gruppe oder eine 6-Fluor-1,2-benzisoxazol-3-yl-gruppe, Y ein Wasserstoffatom oder eine Hydroxygruppe, m eine ganze Zahl mit einem Wert zwischen 0 und 4 einschließlich, n 0 oder 1, Q ein Stickstoffatom oder eine Methingruppe; wenn Q ein Stickstoffatom darstellt, R eine Cyanogruppe oder eine Carbamoylgruppe; wenn Q eine Methingruppe darstellt, R eine Nitrogruppe; $R^1$ und $R^2$, die gleichartig oder verschieden sind, Wasserstoffatome, Niedrigalkylgruppen, Phenylgruppen, 2,2,2-Trifluorethylgruppen oder 2-[4-(4-Fluorbenzoyl)-1-piperidi- nyl]-ethylgruppen bedeuten; oder der Rest $NR^1R^2$ einen Piperidinorest oder einen 4-(4-Fluorbenzoyl)-1-piperidinylrest bedeutet; sowie deren Salze mit anorganischen oder organischen. pharmazeutisch annehmbaren Säuren.

**16.** Pharmazeutische Zubereitung, **dadurch gekennzeichnet,** daß sie mindestens ein Piperidin der in Anspruch 1 definierten Formel I und einen geeigneten pharmazeutischen Träger umfaßt.

**17.** Pharmazeutische Zubereitung nach Anspruch 16, **dadurch gekennzeichnet,** daß sie in Form von Tabletten, dragierten Tabletten, Gelkapseln oder injizierbaren Lösungen vorliegt.

**18.** Verwendung der Verbindungen der Formel I nach Anspruch 1 zur Herstellung eines Arzneimittels für die Behandlung und die Prophylaxe der Hypertension und von organischen Störungen, die durch einen Serotoninüberschuß verursacht oder verstärkt werden.

**Patentansprüche für folgenden Vertragsstaat : ES**

1. Verfahren zur Herstellung von Verbindungen der Formel I

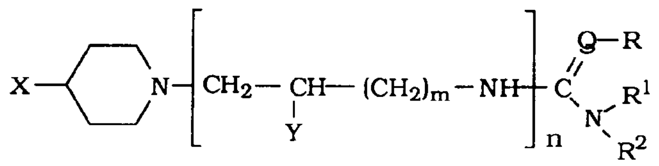

I

in der X eine 4-Fluorbenzoylgruppe, eine 2-(4-Fluorphenyl)-1,3-dioxolan-2-yl-gruppe oder eine 6-Fluor-1,2-benzisoxazol-3-yl-gruppe, Y ein Wasserstoffatom oder eine Hydroxygruppe, m eine ganze Zahl mit einem Wert zwischen 0 und 4 einschließlich, n 0 oder 1, Q ein Stickstoffatom oder eine Methingruppe; wenn Q ein Stickstoffatom darstellt, R eine Cyanogruppe oder eine Carbamoylgruppe; wenn Q eine Methingruppe darstellt, R eine Nitrogruppe; $R^1$ und $R^2$, die gleichartig oder verschieden sind, Wasserstoffatome, Niedrigalkylgruppen, Phenylgruppen, 2,2,2-Trifluorethylgruppen oder 2-[4-(4-Fluorbenzoyl)-1-piperidinyl]-ethylgruppen bedeuten; oder der Rest $NR^1R^2$ einen Piperidinorest oder einen 4-(4-Fluorbenzoyl)-1-piperidinylrest bedeutet; sowie von deren Salzen mit anorganischen oder organischen, pharmazeutisch annehmbaren Säuren, **dadurch gekennzeichnet,** daß man mindestens eine der folgenden Stufen durchführt:

A) Behandlung eines Amins der Formel II

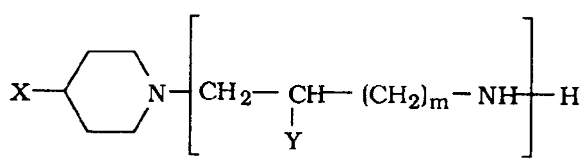

II

mit einem Derivat der Formel

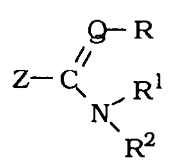

III

worin X, Y, Q, R, $R^1$, $R^2$, m und n die oben angegebenen Bedeutungen besitzen und Z eine Methylthiogruppe oder eine Phenoxygruppe bedeutet, oder

B) Behandlung eines Derivats der Formel IV

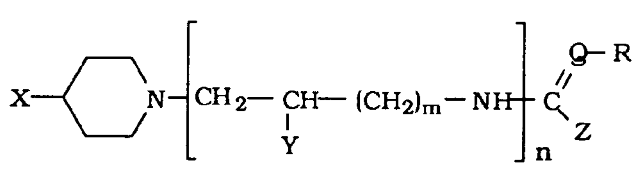

IV

mit einem Amin der Formel $HNR^1R^2$, worin X, Y, Q, R, $R^1$, $R^2$, m und n die oben angegebenen Bedeutungen besitzen und Z eine Methylthiogruppe oder eine Phenoxygruppe darstellt, oder

C) dann, wenn Q ein Stickstoffatom, R eine Carbamoylgruppe und X eine 4-Fluorbenzoylgruppe bedeuten, Hydrolyse der entsprechenden Verbindungen I, worin X eine 2-(4-Fluorphenyl)-1,3-dioxolan-2-yl-gruppe und R eine Cyanogruppe bedeuten, oder

D) dann, wenn Q ein Stickstoffatom, R eine Carbamoylgruppe und X eine 6-Fluor-1,2-benzisoxazol-3-yl-gruppe bedeuten, Hydrolyse der entsprechenden Verbindungen I, worin R eine Cyanogruppe darstellt.

2. Verfahren nach Anspruch 1, **dadurch gekennzeichnet,** daß man die Behandlung A) in einem inerten Lösungsmittel, vorzugsweise Ethanol oder 2-Methoxyethanol, durchführt.

3. Verfahren nach Anspruch 1, **dadurch gekennzeichnet,** daß man die Behandlung B) in einem inerten Lösungsmittel, vorzugsweise Ethanol, oder ohne Lösungsmittel in einem Überschuß des Amins der Formel $HNR^1R^2$ durchführt.

4. Verfahren nach Anspruch 1, **dadurch gekennzeichnet,** daß die Verbindungen jene sind, worin X eine 4-Fluorbenzoylgruppe oder eine 6-Fluor-1,2-benzisoxazol-3-yl-gruppe, Y ein Wasserstoffatom, m 0, 1 oder 2 und n 1 bedeuten, sowie deren Salze mit anorganischen oder organischen, pharmazeutisch annehmbaren Säuren.

5. Verfahren nach Anspruch 1, **dadurch gekennzeichnet,** daß die Verbindung N-Cyano-N'-[2-[4-(4-fluorbenzoyl)-1-piperidinyl]-ethyl]-N"-methylguanidin ist und dessen Salze mit anorganischen oder organischen, pharmazeutisch annehmbaren Säuren.

6. Verfahren nach Anspruch 1, **dadurch gekennzeichnet,** daß die Verbindung N-Cyano-N'-[2-[4-(6-fluor-1,2-benzisoxazol-3-yl)-1-piperidinyl]-ethyl]-N"-methylguanidin ist und dessen Salze mit anorganischen oder organischen, pharmazeutisch annehmbaren Säuren.

7. Verfahren nach Anspruch 1, **dadurch gekennzeichnet,** daß die Verbindung N-Cyano-N'-[3-[4-(6-fluor-1,2-benzisoxazol-3-yl)-1-piperidinyl]-propyl]-N"-methylguanidin ist und dessen Salze mit anorganischen oder organischen, pharmazeutisch annehmbaren Säuren.

8. Verfahren nach Anspruch 1, **dadurch gekennzeichnet,** daß die Verbindung N-Cyano-N'-ethyl-N"-[3-[4-(6-fluor-1,2-benzisoxazol-3-yl)-1-piperidinyl]-propyl]-guanidin ist und dessen Salze mit anorganischen oder organischen, pharmazeutisch annehmbaren Säuren.

9. Verfahren nach Anspruch 1, **dadurch gekennzeichnet,** daß die Verbindung N'-Cyano-N,N-dimethyl-N"-[3-[4-[6-fluor-1,2-benzisoxazol-3-yl)-1-piperidinyl]-propyl]-guanidin ist und dessen Salze mit anorganischen oder organischen, pharmazeutisch annehmbaren Säuren.

10. Verfahren nach Anspruch 1, **dadurch gekennzeichnet,** daß die Verbindung N-Cyano-N'-[3-[4-(6-fluor-1,2-benzisoxazol-3-yl)-1-piperidinyl]-propyl]-guanidin ist und dessen Salze mit anorganischen oder organischen, pharmazeutisch annehmbaren Säuren.

11. Verfahren nach Anspruch 1, **dadurch gekennzeichnet,** daß die Verbindung N-[2-[4-(6-Fluor-1,2-benzisoxazol-3-yl)-1-piperidinyl]-ethyl]-N'-methyl-2-nitro-1,1 -ethendiamin ist.

12. Verfahren zur Herstellung der Zwischenprodukte der Formel IV

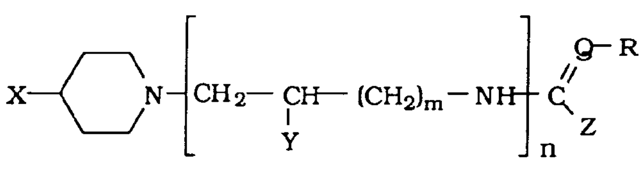

IV

worin X, Y, Q. R, m und n die in Anspruch 1 angegebenen Bedeutungen besitzen und Z eine Methylthiogruppe oder eine Phenoxygruppe darstellt, oder auch die Zwischenprodukte für die Herstellung der Verbindungen der Formel I nach Anspruch 1, **dadurch gekennzeichnet,** daß man eine Kondensation des Amins der Formel II mit einem Derivat der Formel XXV

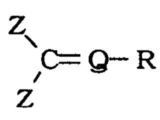

XXV

worin Z, Q und R die oben angegebenen Bedeutungen besitzen, bewirkt.

13. Verfahren zur Herstellung von pharmazeutischen Zubereitungen, **dadurch gekennzeichnet,** daß man mindestens eine Verbindung der Formel I

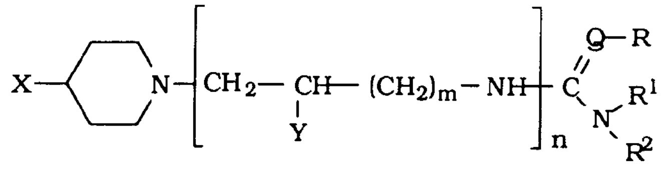

I

in der X eine 4-Fluorbenzoylgruppe, eine 2-(4-Fluorphenyl)-1,3-dioxolan-2-yl-gruppe oder eine 6-Fluor-1,2-benzisoxazol-3-yl-gruppe, Y ein Wasserstoffatom oder eine Hydroxygruppe, m eine ganze Zahl mit einem Wert zwischen 0 und 4 einschließlich, n 0 oder 1, Q ein Stickstoffatom oder eine Methingruppe; wenn Q ein Stickstoffatom darstellt, R eine Cyanogruppe oder eine Carbamoylgruppe; wenn Q eine Methingruppe darstellt, R eine Nitrogruppe; $R^1$ und $R^2$, die gleichartig oder verschieden sind, Wasserstoffatome, Niedrigalkylgruppen, Phenylgruppen, 2,2,2-Trifluorethylgruppen oder 2-[4-(4-Fluorbenzoyl)-1-piperidinyl]-ethylgruppen bedeuten; oder der Rest $NR^1R^2$ einen Piperidinorest oder einen 4-(4-Fluorbenzoyl)-1-piperidinylrest bedeutet; oder ein Salz dieser Verbindung mit anorganischen oder organischen, pharmazeutisch annehmbaren Säuren mit einem geeigneten pharmazeutischen Träger vermischt.

14. Verfahren nach Anspruch 13, **dadurch gekennzeichnet,** daß der Träger zur Herstellung der Zubereitung in Form von Tabletten, dragierten Tabletten, Gelkapseln oder injizierbaren Lösungen geeignet ist.

**Patentansprüche für folgenden Vertragsstaat : GR**

1. Piperidine, **gekennzeichnet durch** die Formel

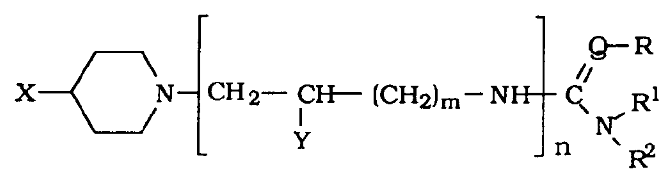

I

in der X eine 4-Fluorbenzoylgruppe, eine 2-(4-Fluorphenyl)-1,3-dioxolan-2-yl-gruppe oder eine 6-Fluor-1,2-benzisoxazol-3-yl-gruppe, Y ein Wasserstoffatom oder eine Hydroxygruppe, m eine ganze Zahl mit einem Wert zwischen 0 und 4 einschließlich, n 0 oder 1, Q ein Stickstoffatom oder eine Methingruppe; wenn Q ein Stickstoffatom darstellt, R eine Cyanogruppe oder eine Carbamoylgruppe; wenn Q eine Methingruppe darstellt, R eine Nitrogruppe; $R^1$ und $R^2$, die gleichartig oder verschieden sind, Wasserstoffatome, Niedrigalkylgruppen, Phenylgruppen, 2,2,2-Trifluorethylgruppen oder 2-[4-(4-Fluorbenzoyl)-1-piperidinyl]-ethylgruppen bedeuten; oder der Rest $NR^1R^2$ einen Piperidinorest oder einen 4-(4-Fluorbenzoyl)-1-piperidinylrest bedeutet; sowie deren Salze mit anorganischen oder organischen, pharmazeutisch annehmbaren Säuren.

2. Piperidine nach Anspruch 2, **dadurch gekennzeichnet,** daß X eine 4-Fluorbenzoylgruppe oder 6-Fluor-1,2-benzisoxazol-3-ylgruppe, Y ein Wasserstoffatom, m 0, 1 oder 2 und n 1 bedeuten, sowie deren Salze mit anorganischen oder organischen, pharmazeutisch annehmbaren Säuren.

3. Piperidin nach Anspruch 1, nämlich N-Cyano-N'-[2-[4-(4-fluorbenzoyl)-1-piperidinyl]-ethyll-N"-methylguanidin und dessen Salze mit anorganischen oder organischen, pharmazeutisch annehmbaren Säuren.

4. Piperidin nach Anspruch 1, nämlich N-Cyano-N'-[2-[4-(6-fluor-1,2-benzisoxazol-3-yl)-1-piperidinyl]-ethyl]-N"-methylguanidin und dessen Salze mit anorganischen oder organischen, pharmazeutisch annehmbaren Säuren.

5. Piperidin nach Anspruch 1, nämlich N-Cyano-N'-[3-[4-(6-fluor-1,2-benzisoxazol-3-yl)-1-piperidinyl]-propyl]-N"-methylguanidin und dessen Salze mit anorganischen oder organischen, pharmazeutisch annehmbaren Säuren.

6. Piperidin nach Anspruch 1, nämlich N-Cyano-N'-ethyl-N"-[3-[4-(6-fluor-1,2-benzisoxazol-3-yl)-1-piperidinyl]-propyl]-guanidin und dessen Salze mit anorganischen oder organischen, pharmazeutisch annehmbaren Säuren.

7. Piperidin nach Anspruch 1, nämlich N'-Cyano-N,N-dimethyl-N"-[3-[4-(6-fluor-1,2-benzisoxazol-3-yl)-1-piperidinyl]-propyl]-guanidin und dessen Salze mit anorganischen oder organischen, pharmazeutisch annehmbaren Säuren.

8. Piperidin nach Anspruch 1, nämlich N-Cyano-N'-[3-[4-(6-fluor-1,2-benzisoxazol-3-yl)-1-piperidinyl]-propyl]-guanidin und dessen Salze mit anorganischen oder organischen, pharmazeutisch annehmbaren Säuren.

9. Piperidin nach Anspruch 1, nämlich N-[2-[4-(6-Fluor-1,2-benzisoxazol-3-yl)-1-piperidinyl]-ethyl]-N'-methyl-2-nitro-1,1-ethendiamin.

10. Verfahren zur Herstellung der Verbindungen nach Anspruch 1, **dadurch gekennzeichnet**, daß man ein Amin der Formel

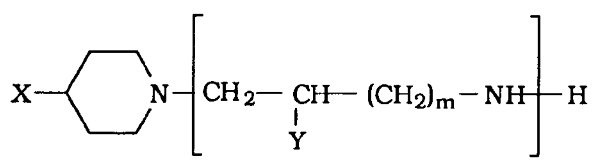

II

mit einem Derivat der Formel

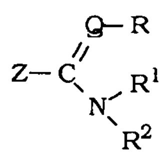

III

worin X, Y, Q, R, $R^1$, $R^2$, m und n die in Anspruch 1 angegebenen Bedeutungen besitzen und Z eine Methylthiogruppe oder eine Phenoxygruppe bedeutet, behandelt.

11. Verfahren nach Anspruch 10, **dadurch gekennzeichnet,** daß man die Umsetzung in einem inerten Lösungsmittel, vorzugsweise Ethanol oder 2-Methoxyethanol, durchführt.

12. Verfahren zur Herstellung der Verbindungen nach Anspruch 1, **dadurch gekennzeichnet,** daß man ein Derivat der Formel

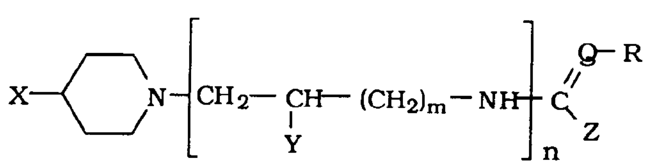

IV

mit einem Amin der Formel $HNR^1R^2$, worin X, Y, Q, R, $R^1$, $R^2$, m und n die in Anspruch 1 angegebenen Bedeutungen besitzen und Z eine Methylthiogruppe oder eine Phenoxygruppe darstellt, behandelt.

**13.** Verfahren nach Anspruch 12, **dadurch gekennzeichnet,** daß man die Umsetzung in einem inerten Lösungsmittel, vorzugsweise Ethanol, oder ohne Lösungsmittel in einem Überschuß des Amins der Formel $HNR^1R^2$ durchführt.

**14.** Zwischenprodukte für die Herstellung der Verbindungen gemäß Anspruch 12, **gekennzeichnet durch** die Formel

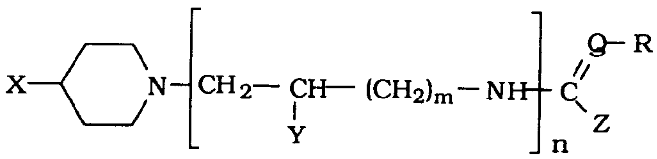

IV

in der X, Y, Q, R, m und n die in Anspruch 1 angegebenen Bedeutungen besitzen und Z eine Methylthiogruppe oder eine Phenoxygruppe bedeuten.

**15.** Verfahren zur Herstellung von pharmazeutischen Zubereitungen, **dadurch gekennzeichnet,** daß man mindestens eine Verbindung der Formel I

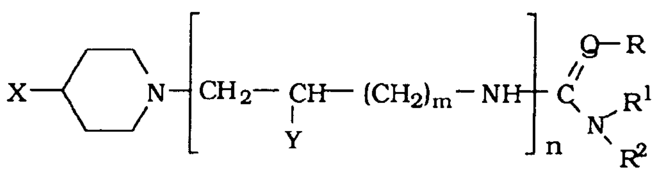

I

in der X eine 4-Fluorbenzoylgruppe, eine 2-(4-Fluorphenyl)-1,3-dioxolan-2-yl-gruppe oder eine 6-Fluor-1,2-benzisoxazol-3-yl-gruppe, Y ein Wasserstoffatom oder eine Hydroxygruppe, m eine ganze Zahl mit einem Wert zwischen 0 und 4 einschließlich, n 0 oder 1, Q ein Stickstoffatom oder eine Methingruppe; wenn Q ein Stickstoffatom darstellt, R eine Cyanogruppe oder eine Carbamoylgruppe; wenn Q eine Methingruppe darstellt, R eine Nitrogruppe; $R^1$ und $R^2$, die gleichartig oder verschieden sind, Wasserstoffatome, Niedrigalkylgruppen, Phenylgruppen, 2,2,2-Trifluorethylgruppen oder 2-[4-(4-Fluorbenzoyl)-1-piperidinyl]-ethylgruppen bedeuten; oder der Rest $NR^1R^2$ einen Piperidinorest oder einen 4-(4-Fluorbenzoyl)-1-piperidinylrest bedeutet; oder ein Salz dieser Verbindung mit anorganischen oder organischen, pharmazeutisch annehmbaren Säuren mit einem geeigneten pharmazeutischen Träger vermischt.

**16.** Verfahren nach Anspruch 15, **dadurch gekennzeichnet,** daß der Träger zur Herstellung der Zubereitung in Form von Tabletten, dragierten Tabletten, Gelkapseln oder Injizierbaren Lösungen geeignet ist.